# EUROPEAN PATENT SPECIFICATION

(11) **EP 0 385 679 B1**
(45) Date of publication and mention of the grant of the patent: **24.04.1996**
(21) Application number: 90301987.5
(22) Date of filing: 23.02.1990
(51) Int. Cl.: C07D 405/12, A61K 31/44, A61K 31/47

(54) **Heterocyclic cyclic ethers with inhibitory activity on 5-lipoxygenase**
Heterozyklische zyklische Ether mit hemmender Wirkung auf 5-Lipoxygenase
Ethers cycliques hétérocycliques avec activité comme inhibiteurs de la 5-lipoxygénase

(30) Priority: 28.02.1989 EP 89400558
(43) Date of publication of application: 05.09.1990
(73) Proprietor: ZENECA LIMITED, London W1Y 6LN (GB); ZENECA Pharma S.A., F-95022 Cergy Cédex (FR)
(72) Inventor: Crawley, Graham Charles, Macclesfield, Cheshire SK10 5AP (GB); Hamon, Annie, F-51100 Reims (FR)
(74) Representative: Tait, Brian Steele

(56) References cited:
- EP-A- 0 200 101
- EP-A- 0 226 342
- EP-A- 0 291 916
- JOURNAL OF MEDICINAL CHEMISTRY, vol. 30, no. 1, January 1987, pages 96-104, American Chemical Society, Columbus, Ohio, US; J.H. MUSSER et al.: "Substituted arylmethyl phenyl ethers. 1. A novel series of 5-lipoxygenase inhibitors and leukotriene antagonists"

## Description

This invention concerns novel heterocyclic cyclic ethers and more particularly novel heterocyclic cyclic ethers which are inhibitors of the enzyme 5-lipoxygenase (hereinafter referred to as 5-LO). The invention also concerns processes for the manufacture of said heterocyclic cyclic ethers and novel pharmaceutical compositions containing said heterocyclic cyclic ethers. Also included in the invention is the use of said heterocyclic cyclic ethers in the treatment of various inflammatory and/or allergic diseases in which the direct or indirect products of 5-LO catalysed oxidation of arachidonic acid are involved, and the production of new medicaments for such use.

As stated above the heterocyclic cyclic ethers described hereinafter are inhibitors of 5-LO, which enzyme is known to be involved in catalysing the oxidation of arachidonic acid to give rise via a cascade process to the physiologically active leukotrienes such as leukotriene B₄ (LTB₄) and the peptido-lipid leukotrienes such as leukotriene C₄ (LTC₄) and leukotriene D₄ (LTD₄) and various metabolites.

The biosynthetic relationship and physiological properties of the leukotrienes are summarised by G.W. Taylor and S.R. Clarke in Trends in Pharmacological Sciences, 1986, 7, 100-103. The leukotrienes and their metabolites have been implicated in the production and development of various inflammatory and allergic diseases such as arthritic diseases, asthma, allergic rhinitis, atopic dermatitis, psoriasis, cardiovascular and cerebrovascular disorders and inflammatory bowel disease. In addition the leukotrienes are mediators of inflammatory diseases by virtue of their ability to modulate lymphocyte and leukocyte function. Other physiologically active metabolites of arachidonic acid, such as the prostaglandins and thromboxanes, arise via the action of the enzyme cyclooxygenase on arachidonic acid.

European Patent Application No. 226342 discloses 5-phenyl-2-oxazole derivatives as anti-inflammatory/anti-allergic agents and European Patent Application No. 200101 discloses certain aryl and heteroaryl ethers as agents for the treatment of hypersensitive ailments.

We have now discovered that certain heterocyclic cyclic ethers are effective as inhibitors of the enzyme 5-LO and thus of leukotriene biosyntheses. Thus, such compounds are of value as therapeutic agents in the treatment of, for example, allergic conditions, psoriasis, asthma, cardiovascular and cerebrovascular disorders, and/or inflammatory and arthritic conditions, mediated alone or in part by one or more leukotrienes.

According to the invention there is provided a heterocyclic cyclic ether of the formula I (set out hereinafter) wherein
Q is a 6-membered monocyclic or 10-membered bicyclic heterocyclic moiety containing one or two nitrogen atoms which may optionally bear one, two or three substituents selected from halogeno, hydroxy, oxo, carboxy, cyano, amino, (1-4C)alkyl, (1-4C)alkoxy, fluoro-(1-4C)alkyl, (1-4C)alkylamino, di-[(1-4C)alkyl]amino, hydroxy-(1-4C)alkyl, amino-(1-4C)alkyl, (1-4C)alkylamino-(1-4C)alkyl, di-[(1-4C)alkyl]amino-(1-4C)alkyl, amino-(2-4C)alkoxy, (1-4C)alkylamino-(2-4C)alkoxy, di-[(1-4C)alkyl]amino-(2-4C)alkoxy and phenyl-(1-4C)alkyl;
wherein A is (1-6C)alkylene, (3-6C)alkenylene, (3-6C)alkynylene or cyclo(3-6C)alkylene;
wherein X is oxy, thio, sulphinyl, sulphonyl or imino;
wherein Ar is phenylene which may optionally bear one or two substituents selected from halogeno, hydroxy, amino, nitro, cyano, ureido, carbamoyl, (1-4C)alkyl, (3-4C)alkenyloxy, (1-4C)alkoxy, (1-4C)alkylthio, (1-4C)alkylsulphinyl, (1-4C)alkylsulphonyl, (1-4C)alkylamino, di-[(1-4C)alkyl]amino, fluoro-(1-4C)alkyl, (1-4C)alkoxycarbonyl, N-[(1-4C)alkyl]carbamoyl, N,N-di-[(1-4C)alkyl]carbamoyl, (2-4C)alkanoylamino, cyano-(1-4C)alkoxy, carbamoyl-(1-4C)alkoxy, amino-(2-4C)alkoxy, (1-4C)alkylamino-(2-4C)alkoxy, di-[(1-4C)alkyl]amino-(2-4C)alkoxy and (1-4C)alkoxycarbonyl-(1-4C)alkoxy; or
Ar is a 6-membered heterocyclene moiety containing up to three nitrogen atoms which may optionally bear one or two substituents selected from halogeno, hydroxy, amino, cyano, (1-4C)alkyl, (1-4C)alkoxy, (1-4C)alkylamino and di-[(1-4C)alkyl]amino; wherein R¹ and R together form a group of the formula -A-X-A³-which, together with the oxygen atom to which A is attached and with the carbon atom to which A³ is attached, defines a ring having 5 to 7 ring atoms, wherein A and A³, which may be the same or different, each is (1-3C)alkylene and X is oxy, thio, sulphinyl, sulphonyl or imino, and which ring may bear one, two or three substituents, which may be the same or different, selected from hydroxy, (1-4C)alkyl, (1-4C)alkoxy, (1-4C)alkylthio, (1-4C)alkylsulphinyl and (1-4C)alkylsulphonyl or which ring may bear a (1-4C)alkylenedioxy substituent, and wherein R³ is (1-6C)alkyl, (2-6C)alkenyl, (2-6C)alkynyl, fluoro-(1-4C)alkyl, cyano-(1-4C)alkyl, hydroxy-(1-4C)alkyl, (1-4C)alkoxy-(1-4C)alkyl or (2-4C)alkanoyloxy(1-4C)alkyl;
or a pharmaceutically-acceptable salt thereof.

The chemical formulae referred to herein by Roman numerals are set out for convenience on a separate sheet hereinafter.

In this specification the generic term "alkyl" includes both straight-chain and branched-chain alkyl groups. However references to individual alkyl groups such as "propyl" are specific for the straight-chain version only and references to individual branched-chain alkyl groups such as "isopropyl" are specific for the branched-chain version only. An analogous convention applies to other generic terms.

It is to be understood that, insofar as certain of the compounds of formula I defined above may exist in optically active or racemic forms by virtue of one or more substituents containing an asymmetric carbon atom, the invention includes in its definition of active ingredient any such optically active or racemic form which possesses the property of inhibiting 5-LO. The synthesis of optically active forms may be carried out by standard techniques of organic chemistry well known in the art, for example by synthesis from optically active starting materials or by resolution of a racemic form. Similarly, inhibitory properties against 5-LO may be evaluated using the standard laboratory techniques referred to hereinafter.

It is also to be understood that, insofar as certain of the compounds of the formula I as defined above may exhibit the phenomenon of tautomerism, for example a compound of the formula I wherein Q bears an oxo or hydroxy substituent, and as any formula drawing presented herein may represent only one of the possible tautomeric forms the invention includes in its definition any tautomeric form of a compound of the formula I which possesses the property of inhibiting 5-LO and is not to be limited merely to any one tautomeric form utilised within the formulae drawings.

Suitable values for the generic terms referred to above include those set out below.

A suitable value for Q when it is a 6-membered monocyclic or 10-membered bicyclic heterocyclic moiety containing one or two nitrogen atoms is, for example, pyridyl, pyridazinyl, pyrimidinyl, pyrazinyl, quinolyl, isoquinolyl, cinnolinyl, quinazolinyl, quinoxalinyl, phthalazinyl or naphthyridinyl, or a hydrogenated derivative thereof such as, for example, 1,2-dihydropyridyl or 1,2-dihydroquinolyl. The heterocyclic moiety may be attached through any available nitrogen atom and it may bear a substituent on any available position including on any available nitrogen atom.

When Q is a 10-membered bicyclic heterocyclic moiety containing one or two nitrogen atoms it will be appreciated that Q may be attached to A from either of the two rings of the bicyclic heterocyclic moiety.

Conveniently Q is, for example, 2-pyridyl, 3-pyridyl, 4-pyridyl, 3-pyridazinyl, 4-pyridazinyl, 2-pyrimidinyl, 4-pyrimidinyl, 5-pyrimidinyl, 2-pyrazinyl, 2-quinolyl, 3-quinolyl, 5-quinolyl, 6-quinolyl, 7-quinolyl, 1-isoquinolyl, 3-isoquinolyl, 6-isoquinolyl, 7-isoquinolyl, 3-cinnolyl, 6-cinnolyl, 7-cinnolyl, 2-quinazolinyl, 4-quinazolinyl, 6-quinazolinyl, 7-quinazolinyl, 2-quinoxalinyl, 5-quinoxalinyl, 6-quinoxalinyl, 1-phthalazinyl, 6-phthalazinyl, 1,5-naphthyridin-2-yl, 1,5-naphthyridin-3-yl, 1,6-naphthyridin-3-yl, 1,6-naphthyridin-7-yl, 1,7-naphthyridin-3-yl, 1,7-naphthyridin-6-yl, 1,8-naphthyridin-3-yl, 2,6-naphthyridin-3-yl or 2,7-naphthyridin-3-yl.

A suitable value for a halogeno substituent which may be present on Q or Ar is, for example, fluoro, chloro, bromo or iodo.

A suitable value for a (1-4C)alkyl substituent which may be present on Q or Ar is, for example, methyl, ethyl, propyl, isopropyl, butyl, isobutyl or sec-butyl.

A suitable value for a (1-4C)alkoxy substituent which may be present on Q or Ar is, for example, methoxy, ethoxy, propoxy, isopropoxy or butoxy.

A suitable value for a fluoro-(1-4C)alkyl substituent which may be present on Q or Ar, is, for example, fluoromethyl, difluoromethyl, trifluoromethyl, 2-fluoroethyl, 2,2,2-trifluoroethyl or pentafluoroethyl.

A suitable value for A when it is (1-6C)alkylene is, for example, methylene, ethylene, ethylidene, trimethylene, propylidene, tetramethylene or pentamethylene; when it is (3-6C)alkenylene is, for example, 1-propenylene, 2-methylprop-1-enylene, 3-methylprop-1-enylene, 1-butenylene or 2-butenylene; and when it is (3-6C)alkynylene is, for example, 1-propynylene, 3-methylprop-1-ynylene, 1-butynylene or 2-butynylene.

A suitable value for A when it is cyclo(3-6C)alkylene is, for example, cyclopropylidene, 1,2-cyclopropylene, cyclopentylidene, 1,2-cyclopentylene, cyclohexylidene or 1,4-cyclohexylene.

A suitable value for Ar when it is phenylene is, for example, 1,3-phenylene or 1,4-phenylene.

A suitable value for Ar when it is a 6-membered heterocyclene moiety containing up to three nitrogen atoms is, for example, pyridylene, pyrimidinylene, pyridazinylene, pyrazinylene or 1,3,5-triazinylene. Conveniently Ar when it is a 6-membered heterocyclene moiety containing up to three nitrogen atoms is, for example, 2,4-, 2,5-, 3,5- or 2,6-pyridylene, 2,4-, 2,5- or 4,6-pyrimidinylene, 3,5- or 3,6-pyridazinylene or 2,5- or 2,6-pyrazinylene.

Suitable values for substituents which may be present on Q or Ar include, for example:-
- for (1-4C)alkylamino:: methylamino, ethylamino propylamino and butylamino;
- for di-[(1-4C)alkyl]amino:: dimethylamino, diethylamino and dipropylamino;
- for amino-(2-4C)alkoxy:: 2-aminoethoxy, 3-aminopropoxy and 4-aminobutoxy;
- for (1-4C)alkylamino-(2-4C)alkoxy:: 2-methylaminoethoxy, 3-methylaminopropoxy and 2-ethylaminoethoxy;
- for di-[(1-4C)alkyl]amino-(2-4C)alkoxy:: 2-dimethylaminoethoxy, 3-dimethylaminopropoxy and 2-diethylaminoethoxy.

Suitable values for substituents which may be present on Q include, for example:-
- for hydroxy-(1-4C)alkyl:: hydroxymethyl, 1-hydroxyethyl, 2-hydroxyethyl, 2-hydroxypropyl and 3-hydroxypropyl;
- for amino-(1-4C)alkyl:: aminomethyl, 1-aminoethyl, 2-aminoethyl, 2-aminopropyl and 3-aminopropyl;
- for (1-4C)alkylamino-(1-4C)-alkyl:: methylaminomethyl, 2-methylaminoethyl, 3-methylaminopropyl, ethylaminomethyl and 2-ethylaminoethyl;
- for di-[(1-4C)alkyl]amino-(1-4C)alkyl:: dimethylaminomethyl, 2-dimethylaminoethyl, 3-dimethylaminopropyl, diethylaminomethyl and 2-diethylaminoethyl;
- for phenyl-(1-4C)alkyl:: benzyl, phenethyl and 3-phenylpropyl.

Suitable values for substituents which may be present on Ar include, for example:-
- for (3-4C)alkenyloxy:: allyloxy, methylallyloxy, but-2-enyloxy and but-3-enyloxy;
- for (1-4C)alkylthio:: methylthio, ethylthio, propylthio, isopropylthio and butylthio;
- for (1-4C)alkylsulphinyl:: methylsulphinyl, ethylsulphinyl, propylsulphinyl, isopropylsulphinyl and butylsulphinyl;
- for (1-4C)alkylsulphonyl:: methylsulphonyl, ethyl-sulphonyl, propylsulphonyl, isopropylsulphonyl and butylsulphonyl;
- for (1-4C)alkoxycarbonyl:: methoxycarbonyl, ethoxycarbonyl and tert-butoxycarbonyl;
- for N-[(1-4C)alkyl]carbamoyl:: N-methylcarbamoyl, N-ethylcarbamoyl and N-propylcarbamoyl;
- for N,N-di-[(1-4C)alkyl]carbamoyl:: N,N-dimethylcarbamoyl and N,N-diethylcarbamoyl;
- for (2-4C)alkanoylamino:: acetamido, propionamido and butyramido;
- for cyano-(1-4C)alkoxy:: cyanomethoxy, 2-cyanoethoxy and 3-cyanopropoxy;
- for carbamoyl-(1-4C)alkoxy:: carbamoylmethoxy, 2-carbamoylethoxy and 3-carbamoylpropoxy;
- for (1-4C)alkoxycarbonyl-(1-4C)-alkoxy:: methoxycarbonylmethoxy, 2-methoxycarbonylethoxy, ethoxycarbonylmethoxy and 2-ethoxycarbonylethoxy.

A suitable value for R³ when it is (1-6C)alkyl is, for example, methyl, ethyl, propyl, butyl, pentyl or hexyl.

A suitable value for R³ when it is (2-6C)alkenyl is, for example, vinyl, allyl, 2-butenyl or 3-butenyl; and when it is (2-6C)alkynyl is, for example, ethynyl, 1-propynyl, 2-propynyl, 1-butynyl or 2-butynyl.

A suitable value for R³ when it is cyano-(1-4C)alkyl is, for example, cyanomethyl, 2-cyanoethyl or 3-cyanopropyl.

A suitable value for R³ when it is fluoro-(1-4C)alkyl is, for example, fluoromethyl, difluoromethyl, trifluoromethyl, 2-fluoroethyl, 2,2,2-trifluoroethyl or pentafluoroethyl; when it is hydroxy-(1-4C)alkyl is, for example, hydroxymethyl, 1-hydroxyethyl, 2-hydroxyethyl, 1-hydroxypropyl, 2-hydroxypropyl or 3-hydroxypropyl; when it is (1-4C)alkoxy-(1-4C)alkyl is, for example, methoxymethyl, 1-methoxyethyl, 2-methoxyethyl, 1-methoxypropyl , 2-methoxypropyl, 3-methoxypropyl, ethyoxymethyl, 1-ethoxyethyl, 2-ethoxyethyl, 1-ethoxypropyl, 2-ethoxypropyl or 3-ethoxypropyl; and when it is (2-4C)alkanoyloxy-(1-4C)alkyl is, for example, acetoxymethyl, 2-acetoxyethyl, 3-acetoxypropyl, propionyloxymethyl, 2-propionyloxyethyl or 3-propionyloxypropyl.

When R¹ and R together form a group of the formula -A-X-A³- which together with the oxygen atom to which A is attached and with the carbon atom to which A³ is attached, defines a ring having 5 to 7 ring atoms then a suitable value for A or A³, which may be the same or different, when each is (1-3C)alkylene is, for example, methylene, ethylene or trimethylene.

Suitable values for the one, two or three substituents which may be present on said 5- to 7-membered ring include for example:-
- for (1-4C)alkyl:: methyl, ethyl, propyl, isopropyl, butyl, isobutyl and sec-butyl;
- for (1-4C)alkoxy:: methoxy, ethoxy, propoxy, isopropoxy and butoxy;
- for (1-4C)alkylthio:: methylthio, ethylthio, propylthio, isopropylthio and butylthio;
- for (1-4C)alkylsulphinyl:: methylsulphinyl, ethylsulphinyl, propylsulphinyl, isopropylsulphinyl and butylsulphinyl;
- for (1-4C)alkylsulphonyl:: methylsulphonyl, ethylsulphonyl, propylsulphonyl, isopropylsulphonyl and butylsulphonyl;
- for (1-4C)alkylenedioxy:: methylenedioxy and ethylenedioxy.

A suitable pharmaceutically-acceptable salt of a heterocyclic cyclic ether of the invention which is sufficiently basic is an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a heterocyclic cyclic ether of the invention which is sufficiently acidic (for example an heterocyclic cyclic ether of the invention which contains a carboxy group) is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Particular novel compounds of the invention are, for example, heterocyclic cyclic ethers of the formula I wherein:-
(a) Q is 2-pyridyl, 3-pyridyl, 3-pyridazinyl, 2-pyrimidinyl or 2-pyrazinyl which may optionally bear one substituent selected from chloro, hydroxy, cyano, methyl, methoxy and trifluoromethyl; and A, X, Ar, R¹, R and R³ have any of the meanings defined hereinbefore;
(b) Q is 2-pyridyl or 3-pyridyl; A is 1-propenylene or 1-propynylene; and X is oxy; and Ar, R¹, R and R³ have any of the meanings defined hereinbefore;
(c) Q is 2-quinolyl, 3-quinolyl, 6-quinolyl, 7-quinolyl, 3-isoquinolyl, 6-isoquinolyl, 7-isoquinolyl, 3-cinnolyl, 2-quinazolinyl, 6-quinazolinyl, 2-quinoxalinyl, 6-quinoxalinyl, 6-phthalazinyl, 1,7-naphthyridin-3-yl, 1,7-naphthyridin-6-yl, 1,8-naphthyridin-3-yl or 2,7-naphthyridin-3-yl which may optionally bear one or two substituents selected from fluoro, chloro, hydroxy, oxo, cyano, methyl, methoxy and trifluoromethyl; and A, X, Ar, R¹, R and R³ have any of the meanings defined hereinbefore;
(d) Q is 3-quinolyl, 5-quinolyl, 6-quinolyl, 7-quinolyl, 3-isoquinolyl, 2-quinazolinyl, 6-quinazolinyl or 6-quinoxalinyl which may optionally bear one, two or three substituents selected from fluoro, chloro, hydroxy, oxo, methyl, ethyl, propyl, trifluoromethyl, 2-fluoroethyl, 2-dimethylaminoethyl and benzyl; and A, X, Ar, R¹, R and R³ have any of the meanings defined hereinbefore;
(e) Q is 1,2-dihydro-2-oxoquinolin-3-yl, 1,2-dihydro-2-oxoquinolin-6-yl, 1,2-dihydro-2-oxoquinolin-7-yl, 3,4-dihydro-4-oxoquinazolin-6-yl, 1,2-dihydro-2-oxo-1,7-naphthyridin-3-yl or 1,2-dihydro-2-oxo-1,8-naphthyridin-3-yl which may optionally bear one or two substituents selected from fluoro, chloro, cyano, methyl, methoxy and trifluoromethyl; and A, X, Ar, R¹, R and R³ have any of the meanings defined hereinbefore;
(f) Q is 1,2-dihydro-2-oxoquinolin-3-yl, 1,2-dihydro-2-oxoquinolin-5-yl, 1,2-dihydro-2-oxoquinolin-6-yl or 1,2-dihydro-2-oxoquinolin-7-yl which may optionally bear one or two substituents selected from fluoro, chloro, methyl, ethyl, propyl, trifluoromethyl, 2-fluoroethyl, 2-dimethylaminoethyl and benzyl; and A, X, Ar, R¹, R and R³ have any of the meanings defined hereinbefore;
(g) Q is 1,2-dihydro-2-oxoquinolin-3-yl, 1,2-dihydro-2-oxoquinolin-5-yl, 1,2-dihydro-2-oxoquinolin-6-yl or 1,2-dihydro-2-oxoquinolin-7-yl which bears a 1-substituent selected from methyl, ethyl, propyl, 2-fluoroethyl, 2-dimethylaminoethyl and benzyl, and which may optionally bear a substituent selected from fluoro, chloro and trifluoromethyl; and A, X, Ar, R¹, R and R³ have any of the meanings defined hereinbefore;
(h) A is methylene, ethylene, trimethylene, 1-propenylene, 2-methylprop-1-enylene or 1-propynylene and Q, X, Ar, R¹, R and R³ have any of the meanings defined hereinbefore;
(i) A is methylene, 1-propenylene or 1-propynylene; and Q, X, Ar, R¹, R and R³ have any of the meanings defined hereinbefore;
(j) X is oxy and Q, A, Ar, R¹, R and R³ have any of the meanings defined hereinbefore;
(k) Ar is 1,3-phenylene or 1,4-phenylene which may optionally bear one substituent selected from fluoro, chloro, hydroxy, amino, nitro, methyl, methoxy, methylthio, methylsulphinyl, methylsulphonyl, methylamino, dimethylamino, trifluoromethyl, acetamido, cyanomethoxy and carbamoylmethoxy; and Q, A, X, R¹, R and R³ have any of the meanings defined hereinbefore;
(l) Ar is 1,3-phenylene or 1,4-phenylene which may optionally bear one or two substituents selected from fluoro, chloro, hydroxy, amino, methoxy and trifluoromethyl; and Q, A, X, R¹, R and R³ have any of the meanings defined hereinbefore;
(m) Ar is 2,4-, 2,5-, 3,5- or 2,6-pyridylene or 4,6-pyrimidinylene which may optionally bear one substituent selected from chloro, methyl and methoxy; and Q, A, X, R¹, R and R³ have any of the meanings defined hereinbefore; or
(n) Ar is 3,5-pyridylene; and Q, A, X, R¹, R and R³ have any of the meanings defined hereinbefore;
(o) R¹ and R together form a group of the formula -A-X-A³- which, together with the oxygen atom to which A is attached and with the carbon atom to which A³ is attached, defines a ring having 5 to 7 ring atoms, wherein A and A³, which may be the same or different, each is methylene or ethylene and X is oxy, and which ring may bear one or two substituents selected from hydroxy, methyl, methoxy, ethoxy, methylthio, methylsulphinyl, methylsulphonyl and methylenedioxy, and R³ is methyl or ethyl; and Q, A and Ar have any of the meanings defined hereinbefore;
(p) R¹ and R together form a group of the formula -A-X-A³- which, together with the oxygen atom to which A is attached and with the carbon atom to which A³ is attached, defines a ring have 5 or 6 ring atoms, wherein A is methylene, A³ is methylene or ethylene and X is oxy, and which ring may bear one, two or three substituents selected from methyl, ethyl, propyl, isopropyl, butyl, isobutyl and sec-butyl, and R³ is methyl or ethyl; and Q, A and Ar have any of the meanings defined hereinbefore;
or a pharmaceutically-acceptable salt thereof.

A particular compound of the invention comprises a heterocyclic cyclic ether of the formula I wherein Q is pyridyl, pyrimidinyl, pyrazinyl, quinolyl, isoquinolyl, quinazolinyl or quinoxalinyl which may optionally bear one, two or three substituents selected from fluoro, chloro, hydroxy, oxo, methyl, ethyl, propyl, trifluoromethyl, 2-fluoroethyl, 2-dimethylaminoethyl and benzyl;
wherein A is methylene, 1-propenylene or 1-propynylene;
wherein X is oxy;
wherein Ar is 1,3-phenylene or 1,4-phenylene which may optionally bear one or two substituents selected from fluoro, chloro, hydroxy, amino, methoxy and trifluoromethyl, or
Ar is 3,5-pyridylene;
wherein R¹ and R together form a group of the formula -A-X-A³- which, together with the oxygen atom to which A is attached and with the carbon atom to which A³ is attached, defines a ring having 5 or 6 ring atoms, wherein A is methylene, A³ is methylene or ethylene and X is oxy, and which ring may bear one, two or three substituents selected from methyl, ethyl, propyl, isopropyl, butyl, isobutyl and sec-butyl, and R³ is methyl or ethyl;
or a pharmaceutically-acceptable salt thereof.

A further particular compound of the invention comprises a heterocyclic cyclic ether of the formula I wherein Q is 2-pyridyl, 3-pyridyl, 3-quinolyl, 5-quinolyl, 6-quinolyl, 7-quinolyl or 6-quinoxalinyl which may optionally bear one or two substituents selected from hydroxy, oxo, methyl, ethyl, propyl, 2-fluoroethyl, 2-dimethylaminoethyl and benzyl;
wherein A is methylene, 1-propenylene or 1-propynylene;
wherein X is oxy;
wherein Ar is 1,3-phenylene or 1,4-phenylene which may optionally bear one or two substituents selected from fluoro, chloro, amino, methoxy and trifluoromethyl, or Ar is 3,5-pyridylene;
wherein R¹ and R together form a group of the formula -A-X-A³- which, together with the oxygen atom to which A is attached and with the carbon atom to which A³ is attached, defines a ring having 5 ring atoms, wherein A is methylene, A³ is methylene and X is oxy, and which ring may bear one, two or three substituents selected from methyl, ethyl, propyl, isopropyl, butyl, isobutyl and sec-butyl, and R³ is methyl or ethyl;
or a pharmaceutically-acceptable salt thereof.

A preferred compound of the invention comprises a heterocyclic cyclic ether of the formula I wherein Q is 2-pyridyl, 1,2-dihydro-1-methyl-2-oxoquinolin-3-yl, 2-quinolyl, 3-quinolyl, 1,2-dihydro-2-oxoquinolin-3-yl, 3-isoquinolyl or 6-quinoxalinyl;
A is methylene or 1-propynylene;
Ar is 1,3-phenylene or 5-fluoro-1,3-phenylene; and
wherein R¹ and R together form a group of the formula -A-X-A³ - which, together with the oxygen atom to which A is attached and with the carbon atom to which A³ is attached, defines a ring having 5 ring atoms, wherein each of A and A³ is methylene, X is oxy, and which ring may bear one or two methyl substituents, and R³ is ethyl;
or a pharmaceutically-acceptable salt thereof.

A further preferred compound of the invention comprises a heterocyclic cyclic ether of the formula I wherein Q is 1,2-dihydro-2-oxoquinolin-5-yl, 1,2-dihydro-2-oxoquinolin-6-yl or 1,2-dihydro-2-oxoquinolin-7-yl which bears a 1-substituent selected from methyl, ethyl and 2-fluoroethyl;
wherein A is methylene;
wherein X is oxy;
wherein Ar is 1,3-phenylene which may optionally bear one fluoro substituent;
wherein R¹ and R together form a group of the formula -A-X-A³- which, together with the oxygen atom to which A is attached and with the carbon atom to which A³ is attached defines a ring having 5 ring atoms, wherein A is methylene, A³ is methylene and X is oxy, and which ring may bear one or two substituents selected from methyl, ethyl, propyl and isopropyl, and R³ is methyl or ethyl;
or a pharmaceutically-acceptable salt thereof.

Specific especially preferred compounds of the invention include, for example, the following heterocyclic cyclic ethers of the formula I, or pharmaceutically-acceptable salts thereof:-
4-ethyl-2,2-dimethyl-4-[3-(3-(2-pyridyl)prop-2-yn-1-yloxy)phenyl]-1,3-dioxolane,
4-ethyl-4-[5-fluoro-3-(1,2-dihydro-1-methyl-2-oxoquinolin-6-ylmethoxy)phenyl]-2,2-dimethyl-1,3-dioxolane and
2,4-diethyl-4-[5-fluoro-3-(1,2-dihydro-1-methyl-2-oxoquinolin-6-ylmethoxy)phenyl]-1,3-dioxolane.

A compound of the invention comprising a heterocyclic cyclic ether of the formula I, or a pharmaceutically-acceptable salt thereof, may be prepared by any process known to be applicable to the preparation of structurally-related compounds. Such procedures are provided as a further feature of the invention and are illustrated by the following representative examples in which, unless otherwise stated, Q, A, X, Ar, R¹, R and R³ have any of the meanings defined hereinbefore.

(a) The alkylation, in the presence of a suitable reagent, of a compound of the formula II with a compound of the formula Q-A-Z wherein Z is a displaceable group; provided that, when there is an amino, imino, alkylamino, hydroxy or carboxy group in Q, Ar, X or R³, any amino, imino, alkylamino or carboxy group is protected by a conventional protecting group and any hydroxy group may be protected by a conventional protecting group or alternatively any hydroxy group need not be protected;
whereafter any undesired protecting group in Q, Ar, X or R³ is removed by conventional means.

A suitable displaceable group Z is, for example, a halogeno, sulphonyloxy or hydroxy group, for example a chloro, bromo, iodo, methanesulphonyloxy or toluene-p-sulphonyloxy group.

A suitable reagent for the alkylation reaction when Z is a halogeno or sulphonyloxy group is, for example, a suitable base, for example, an alkali or alkaline earth metal carbonate, hydroxide or hydride, for example sodium carbonate, potassium carbonate, sodium hydroxide, potassium hydroxide, sodium hydride or potassium hydride. The alkylation reaction is preferably performed in a suitable inert solvent or diluent, for example N,N-dimethylformamide, N,N-dimethylacetamide, dimethylsulphoxide, acetone, 1,2-dimethoxyethane or tetrahydrofuran, and at a temperature in the range, for example, -10 to 150°C, conveniently at or near ambient temperature.

A suitable reagent for the alkylation reaction when Z is a hydroxy group is, for example, the reagent obtained when a compound of the formula Q-A-OH is reacted with a di-(1-4C)alkyl azodicarboxylate in the presence of a triarylphosphine, for example with diethyl azodicarboxylate in the presence of triphenylphosphine. The alkylation reaction is preferably performed in a suitable inert solvent or diluent, for example acetone, 1,2-dimethoxyethane or tetrahydrofuran, and at a temperature in the range, for example, 10 to 80°C, conveniently at or near ambient temperature.

A suitable protecting group for an amino, imino or alkylamino group is, for example, an acyl group for example a (1-4C)alkanoyl group (especially acetyl), a (1-4C)alkoxycarbonyl group (especially methoxycarbonyl, ethoxycarbonyl or t-butoxycarbonyl), an arylmethoxycarbonyl group (especially benzyloxycarbonyl) or an aroyl group (especially benzoyl). The deprotection conditions for the above protecting groups necessarily vary with the choice of protecting group. Thus, for example, an acyl group such as an alkanoyl, alkoxycarbonyl or an aroyl group may be removed for example, by hydrolysis with a suitable base such as an alkali metal hydroxide, for example lithium or sodium hydroxide. Alternatively an acyl group such as a t-butoxycarbonyl group may be removed, for example, by treatment with a suitable acid such as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-charcoal.

A suitable protecting group for a carboxy group is, for example, an esterifying group, for example a (1-4C)alkyl group (especially methyl or ethyl) or an arylmethyl group (especially benzyl). The deprotection conditions for the above protecting groups necessarily vary with the choice of protecting group. Thus, for example, an esterifying group such as an alkyl or arylmethyl group may be removed, for example, by hydrolysis with a suitable base such as an alkali metal hydroxide, for example lithium or sodium hydroxide. Alternatively an esterifying group such as an arylmethyl group may be removed, for example, by hydrogenation over a catalyst such as palladium-on-charcoal.

A suitable protecting group for a hydroxy group is, for example, an acyl group, for example a (1-4C)alkanoyl group (especially acetyl), an aroyl group (especially benzoyl) or an arylmethyl group (especially benzyl). The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-charcoal.

The starting materials of the formula II may be obtained by standard procedures of organic chemistry. The preparation of examples of such starting materials is described within the accompanying non-limiting Examples which are provided for the purpose of illustration only. Other necessary starting materials are obtainable by analogous procedures to those described or by modification thereto which are within the ordinary skill of an organic chemist. Thus the starting material of the formula II may be obtained, for example, by deprotecting a protected heterocyclic cyclic ether of the formula III wherein R⁴ is a protecting group and X, Ar, A, X, A³ and R³ have the meanings defined hereinbefore.

A suitable protecting group R⁴ is, for example, an arylmethyl group (especially benzyl), a tri-(1-4C)alkylsilyl group (especially trimethylsilyl or t-butyldimethylsilyl), an aryldi-(1-4C)alkylsilyl group (especially dimethylphenylsilyl), a (1-4C)alkyl group (especially methyl), a (1-4C)alkoxymethyl group (especially methoxymethyl) or a tetrahydropyranyl group (especially tetrahydropyran-2-yl). The deprotection conditions for the above protecting groups will necessarily vary with the choice of protecting group. Thus, for example, an arylmethyl group such as a benzyl group may be removed, for example, by hydrogenation over a catalyst such as palladium-on-charcoal. Alternatively a trialkylsilyl or an aryldialkylsilyl group such as a t-butyldimethylsilyl or a dimethylphenylsilyl group may be removed, for example, by treatment with a suitable acid such as hydrochloric, sulphuric, phosphoric or trifluoroacetic acid or with an alkali metal or ammonium fluoride such as sodium fluoride or, preferably, tetrabutylammonium fluoride. Alternatively an alkyl group may be removed, for example, by treatment with an alkali metal (1-4C)alkylsulphide such as sodium thioethoxide or, for example, by treatment with an alkali metal diarylphosphide such as lithium diphenylphosphide or, for example, by treatment with a boron or aluminium trihalide such as boron tribromide. Alternatively a (1-4C)alkoxymethyl group or tetrahydropyranyl group may be removed, for example, by treatment with a suitable acid such as hydrochloric or trifluoroacetic acid.

The protecting group R⁴ may be, for example, a tri-(1-4C)alkylsilyl group which can be removed while the protecting group for any amino, imino, alkylamino, carboxy or hydroxy group in Ar, X or R³ is retained.

The protected starting material of the formula III may be obtained by standard procedures of organic chemistry as illustrated in the accompanying non-limiting Examples. Thus, for example, an alcohol of the formula R⁴-X-Ar-CH(OH)-R³, wherein R⁴ is a protecting group as defined hereinbefore, may be obtained by the reaction of an aldehyde of the formula R⁴-X-Ar-CHO with an organometallic compound of the formula R³-M or R³-M-Z, wherein R³ has the meaning defined hereinbefore, M is a metallic group, for example lithium, magnesium or zinc, and Z is a halogeno group, for example chloro, bromo or iodo, and provided that any amino, alkylamino or hydroxy group in Ar or R³ is protected by a conventional protecting group. The reaction may be carried out in, for example, a suitable solvent or diluent such as an ether (for example tetrahydrofuran, t-butylmethylether or diethyl ether) at a temperature in the range, for example, -100 to 50°C (especially -80 to 30°C).

The secondary alcohol of the formula R⁴-X-Ar-CH(OH)-R³ may be oxidised to give a ketone of the formula R⁴-X-Ar-CO-R³. A particular suitable oxidising agent is, for example, any agent known in the art for the oxidation of a secondary alcohol to a ketone, for example, manganese dioxide, chromium trioxide pyridine complex, 2,3-dichloro-5,6-dicyano-1,4-benzoquinone (hereinafter DDQ), a mixture of dimethylsulphoxide, oxalyl chloride and triethylamine, a mixture of acetic anhydride and dimethylsulphoxide or a mixture of dimethylsulphoxide and a dialkylcarbodiimide, for example N,N′-dicyclohexylcarbodiimide or N-ethyl-N′-(3-dimethylaminopropyl)-carbodiimide.

A tertiary alcohol of the formula IV, wherein R⁴ has the meaning defined hereinbefore, may be obtained by the reaction of the ketone R⁴-X-Ar-CO-R³ with an organometallic compound of the formula R⁷-X-A³-M-Z, wherein M is a metallic group, for example magnesium, Z is halogeno group, for example chloro, bromo or iodo, and R⁷ is a suitable protecting group as defined below, and provided that any amino, alkylamino or hydroxy group in Ar or R³ is protected by a conventional protecting group. The reaction may be carried out in a suitable solvent or diluent such as an ether (for example tetrahydrofuran, t-butyl methyl ether or diethyl ether) at a temperature in the range, for example, -30 to 100°C (especially ambient temperature to 80°C).

R⁷, when it is a suitable protecting group for an amino or hydroxy group, has one of the meanings defined hereinbefore. R⁷ when it is a suitable protecting group for a mercapto group is, for example, an acyl group, for example a (1-4C)alkanoyl group (especially acetyl) or an aroyl group (especially benzoyl). An acyl group such as an alkanoyl or an aroyl group may be removed, for example, by hydrolysis with a suitable base such as an alkali metal hydroxide, for example lithium or sodium hydroxide.

It will be appreciated that the tertiary alcohol of the formula IV may be obtained from the aldehyde of the formula R⁴-X-Ar-CHO by reversing the order of introduction of the groups R³ and R⁷-X-A³-. Thus the aldehyde of the formula R⁴-X-Ar-CHO may be treated initially with the organometallic compound of the formula R⁷-X-A³-M-Z, the product so obtained may be oxidised using a suitable oxidising agent as described above and the resultant ketone may be treated with the organometallic compound R³-M or R³-M-Z to give the compound of the formula IV, and provided that any amino, alkylamino or hydroxy group in Ar or R³ is protected by a conventional protecting group.

The cyclic ether derivative of the formula III, wherein R⁴ has the meaning defined hereinbefore, may be obtained from the tertiary alcohol of the formula IV by the removal of the protecting group R⁷, while the protecting group R⁴ and the protecting group for any amino, alkylamino, carboxy or hydroxy group in Ar or R³ is retained, and cyclisation in the presence of a suitable base of the compound of the formula V so formed by reaction with a compound of the formula Z-A-Z, wherein Z is a suitable displaceable group as defined hereinbefore, or the cyclisation in the presence of a suitable acid, for example hydrochloric, sulphuric, phosphoric, trifluoroacetic or p-toluenesulphonic acid, or a Lewis acid such as a boron trihalide, for example boron trifluoride, of the compound of the formula V so formed by reaction with an appropriate aldehyde, for example formaldehyde or acetaldehyde, or with an appropriate ketone, for example acetone, or with corresponding hemiacetal or acetal derivatives thereof.

Alternatively the tertiary alcohol starting material of the formula IV may be obtained by the reaction of a compound of the formula R⁴-X-Ar-Z, wherein R⁴ and Ar have the meanings defined hereinbefore and Z is a halogeno group as defined hereinbefore and provided that any amino, alkylamino or hydroxy group in Ar is protected with a conventional protecting group, with either an organometallic compound of the formula R⁵-M, wherein R⁵ is a (1-6C)alkyl group such as butyl and M is a metallic group, for example lithium, to give an organometallic compound of the formula R⁴-X-Ar-M, or with a metal such as magnesium to give an organometallic compound of the formula R⁴-X-Ar-M-Z; whereafter either of these organometallic compounds may be reacted with a ketone of the formula R⁷-X-A³-CO-R³, wherein R⁷, X, A³ and R³ have the meanings defined hereinbefore, and provided that any hydroxy group in R³ is protected by a conventional protecting group.

Alternatively the ketone of the formula R⁴-X-Ar-CO-R³ described hereinbefore may be obtained by the reaction of the nitrile of the formula R⁴-X-Ar-CN with an organometallic compound of the formula R³-M or R³-M-Z using the conditions defined hereinbefore for the corresponding reaction of the aldehyde of the formula R⁴-X-Ar-CHO.

(b) The cyclisation, in the presence of a suitable base as defined hereinbefore, of a compound of the formula VI by reaction with a compound of the formula Z-A-Z, wherein A and Z have the meanings defined hereinbefore, provided that, when there is an amino, imino, alkylamino, hydroxy or carboxy group in Q, X, Ar or R³, any amino, imino, alkylamino, hydroxy or carboxy group is protected by a conventional protecting group;
whereafter any undesired protecting group in Q, X, Ar or R³ is removed by conventional means.

The tertiary alcohol starting material of the formula VI may be obtained, for example, by the reaction of an aldehyde of the formula Q-A-X-Ar-CHO with an organometallic compound of the formula R³-M or R³-M-Z, having the meaning defined hereinbefore and using the conditions defined hereinbefore, to give a secondary alcohol of the formula Q-A-X-Ar-CH(OH)-R³ and provided that any amino, imino, alkylamino, carboxy or hydroxy group in Q, X, Ar or R³ is protected by a conventional protecting group. The product so obtained may be oxidised using a suitable oxidising agent, as defined hereinbefore, to give a ketone of the formula Q-A-X-Ar-CO-R³, which in turn may be treated with an organometallic compound of the formula R⁷-X-A³-M-Z, having the meaning defined hereinbefore and using the conditions defined hereinbefore, to give the tertiary alcohol of the formula VII, whereafter the protecting group R⁷ may be removed using the conditions defined hereinbefore to give the required tertiary alcohol starting material of the formula VI.

It will be appreciated that the tertiary alcohol of the formula VII may be obtained from the aldehyde of the formula Q-A-X-Ar-CHO by reversing the order of the introduction of the groups R⁷-X-A³-, i.e. by reaction of the aldehyde of the formula Q-A-X-Ar-CHO with the organometallic compound of the formula R⁷-X-A³-M-Z, oxidation of the secondary alcohol to a ketone of the formula Q-A-X-Ar-CO-A³-X-R⁷ and reaction of said ketone with the organometallic compound of the formula R³-M or R³-M-Z, and provided that any amino, imino, alkylamino, carboxy or hydroxy group in Q, X, Ar or R³ is protected by a conventional protecting group.

Alternatively the ketone intermediate of the formula Q-A-X-Ar-CO-A³-X-R⁷ may be obtained, for example, by the alkylation, in the presence of a suitable base as defined hereinbefore, of a ketone of the formula HX-Ar-CO-A³-X-R⁷ with a compound of the formula Q-A-Z, wherein Z is a displaceable group as defined hereinbefore, and provided that any amino, imino, alkylamino, carboxy or hydroxy group in Q or Ar is protected by a conventional protecting group.

The aldehyde starting material of the formula Q-A-X-Ar-CHO may be obtained, for example, by the alkylation, in the presence of a suitable base as defined hereinbefore, of an aldehyde of the formula H-X-Ar-CHO with a compound of the formula Q-A-Z, wherein Z is a displaceable group, as defined hereinbefore, and provided that any amino, alkylamino, carboxy or hydroxy group in Q or Ar is protected by a conventional protecting group.

Alternatively the tertiary alcohol of the formula VII may be obtained, for example, by the reaction of an ester of the formula Q-A-X-Ar-CO₂R⁶, wherein R⁶ is a (1-4C)alkyl group such as methyl or ethyl, with an organometallic compound of the formula R³-M or R³-M-Z, having the meaning defined hereinbefore and using the conditions defined hereinbefore for the corresponding reaction of the aldehyde of the formula R⁴-X-Ar-CHO, and provided that any amino, imino, alkylamino, carboxy or hydroxy group in Q, X, Ar or R³ is protected by a conventional protecting group, to give a ketone of the formula Q-A-X-Ar-CO-R³. The product so obtained may be treated with an organometallic compound of the formula R⁷-X-A³-M-Z, having the meaning defined hereinbefore and using the conditions defined hereinbefore, to give the tertiary alcohol of the formula VII.

It will be appreciated that the tertiary alcohol of the formula VII may be obtained from the ester of the formula Q-A-X-Ar-CO₂R⁶ reversing the order of the introduction of the groups R³ and R⁷-X-A³-, i.e. by reaction of the ester of the formula Q-A-X-Ar-CO₂R⁶ with the organometallic compound of the formula R⁷-X-A³-M-Z, to give a ketone of the formula Q-A-X-Ar-CO-A³-X-R⁷ and reaction of said ketone with the organometallic compound of the formula R³-M or R³-M-Z and provided that any amino, imino, alkylamino, carboxy or hydroxy group in Q, X, Ar or R³ is protected by a conventional protecting group.

The ester starting material of the formula Q-A-X-Ar-CO₂R⁶ may be obtained, for example, by the alkylation, in the presence of a suitable base as defined hereinbefore, of an ester of the formula H-X-Ar-CO₂R⁶, wherein R⁶ has the meaning defined hereinbefore, with a compound of the formula Q-A-Z, wherein Z is a displaceable group as defined hereinbefore, and provided that any amino, alkylamino, carboxy or hydroxy group in Q or Ar is protected by a conventional protecting group.

Alternatively the ketone of the formula Q-A-X-Ar-CO-R³ may be obtained by the reaction of a nitrile of the formula Q-A-X-Ar-CN with an organometallic compound of the formula R³-M or R³-M-Z using the conditions defined hereinbefore for the corresponding reaction of the aldehyde of the formula R⁴-X-Ar-CHO.

Alternatively the tertiary alcohol of the formula VII may be obtained, for example, by the alkylation, in the presence of a suitable base, of a compound of the formula HX-Ar-Z, wherein Ar has the meaning defined hereinbefore and Z is a halogeno group as defined hereinbefore, with a compound of the formula Q-A-Z, wherein Q, A and Z have the meanings defined hereinbefore, and provided that any amino, alkylamino, carboxy or hydroxy group in Q or Ar is protected by a conventional protecting group, to give a compound of the formula Q-A-X-Ar-Z. The product so obtained may be treated either with an organometallic compound of the formula R⁸-M, wherein R⁸ is a (1-6C)alkyl group such as butyl and M is a metallic group, for example lithium, to give an organometallic compound of the formula Q-A-X-Ar-M, or with a metal such as magnesium to give an organometallic compound of the formula Q-A-X-Ar-M-Z. Either of these organometallic compounds may be reacted with a ketone of the formula R³-CO-A³-X-R⁷, provided that any hydroxy group in R³ is protected by a conventional protecting group, to give the tertiary alcohol of the formula VII.

(c) The cyclisation, in the presence of a suitable acid as defined hereinbefore, of a compound of the formula VI by reaction with an appropriate aldehyde or with an appropriate ketone, or with corresponding hemiacetal or acetal derivatives thereof, provided that, when there is an amino, imino, alkylamino, hydroxy or carboxy group in Q, X, Ar or R³, any imino, amino, alkylamino, hydroxy or carboxy group is protected by a conventional protecting group; whereafter any undesired protecting group in Q, X, Ar or R³ is removed by conventional means.

The tertiary alcohol starting material of the formula VI may be obtained as defined hereinbefore.

(d) For the production of those compounds of the formula I wherein A is a (3-6C)alkynylene group, the coupling, in the presence of a suitable organometallic catalyst, of a heterocyclic compound of the formula Q-Z, wherein Q has the meaning defined hereinbefore and Z is a halogeno group such as iodo, with an ethynyl compound of the formula VIII, wherein A¹ is (1-4C)alkylene and X, Ar, R¹, R and R³ have the meanings defined hereinbefore.

A suitable organometallic catalyst is, for example, any agent known in the art for such a coupling reaction. Thus, for example, a suitable reagent is formed when, for example, bis(triphenylphosphine)palladium chloride, or tetrakis(triphenylphosphine)palladium, and a copper halide, for example cuprous iodide, are mixed. The coupling is generally carried out in a suitable inert solvent or diluent, for example acetonitrile, 1,2-dimethoxyethane, toluene or tetrahydrofuran, at a temperature in the range, for example, 10 to 80°C, conveniently at or near 70°C, and in the presence of a suitable base such as, for example, a tri-(1-4C)alkylamine such as triethylamine, or a cyclic amine such as piperidine.

The ethynyl compound of the formula VIII, used as a starting material, may be obtained, for example, by the alkylation, in the presence of a suitable base, of a compound of the formula II, wherein X, Ar, A, X, A³ and R³ have the meanings defined hereinbefore, with an alkylating agent of the formula H-C≡C-A¹-Z, wherein A¹ has the meaning defined hereinbefore and Z is a halogeno group, and provided that any amino, alkylamino, carboxy or hydroxy group in Ar, A, A³, X, or R³ is protected by a conventional protecting group.

(e) For the production of those compounds of the formula I wherein Ar bears an alkylsulphinyl or alkylsulphonyl substituent, wherein X is a sulphinyl or sulphonyl group, or wherein R¹ and R together form a group of the formula -A-X-A³-, X is a sulphinyl or sulphonyl group and which group may bear one or two alkylsulphinyl or alkylsulphonyl groups, the oxidation of a compound of the formula I wherein Ar bears an alkylthio substituent, wherein X is a thio group, or wherein R¹ and R together form a group of the formula -A-X-A³-, X is a thio group, and which group may bear one or two alkylthio groups.

A suitable oxidising agent is, for example, any agent known in the art for the oxidation of thio to sulphinyl and/or sulphonyl, for example, hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible and with the required stoichiometric amount of oxidising agent in order to reduce the risk of over oxidation and damage to other functional groups. In general the reaction is carried out in a suitable solvent or diluent such as methylene chloride, chloroform, acetone, tetrahydrofuran or t-butyl methyl ether and at a temperature, for example, at or near ambient temperature, that is in the range 15 to 35°C. When a compound carrying a sulphinyl group is required a milder oxidising agent may also be used, for example sodium or potassium metaperiodate, conveniently in a polar solvent such as acetic acid or ethanol. It will be appreciated that when a compound of the formula I containing a sulphonyl group is required, it may be obtained by oxidation of the corresponding sulphinyl compound as well as of the corresponding thio compound.

(f) For the production of those compounds of the formula I wherein Ar bears an alkanoylamino substituent, the acylation of a compound of the formula I wherein Ar bears an amino substituent.

A suitable acylating agent is, for example, any agent known in the art for the acylation of amino to acylamino, for example an acyl halide, for example a (2-6C)alkanoyl chloride or bromide, in the presence of a suitable base, an alkanoic acid anhydride, for example a (2-6C)alkanoic acid anhydride, or an alkanoic acid mixed anhydride, for example the mixed anhydride formed by the reaction of an alkanoic acid and a (1-4C)alkoxycarbonyl halide, for example a (1-4C)alkoxycarbonyl chloride, in the presence of a suitable base. In general the reaction is carried out in a suitable solvent or diluent such as methylene chloride, acetone, tetrahydrofuran or t- butyl methyl ether and at a temperature, for example, at or near ambient temperature, that is in the range 15 to 35°C. A suitable base when it is required is, for example, pyridine, 4-dimethylaminopyridine, triethylamine, ethyldiisopropylamine, N-methylmorpholine, an alkali metal carbonate, for example potassium carbonate, or an alkali metal carboxylate, for example sodium acetate.

(g) For the production of those compounds of the formula I wherein A is alkenylene, or wherein R¹ and R together from a group of the formula -A-X-A³- and R³ is alkenyl, the reduction of the corresponding compound wherein A is alkynylene or R³ is alkynyl. In general conditions which are standard in the art for the reduction of an alkynyl or alkynylene group are used. Thus, for example, the reduction may be carried out by the hydrogenation of a solution of the alkynyl or alkynylene compound in an inert solvent or diluent in the presence of a suitable metal catalyst. A suitable inert solvent is, for example, an alcohol, for example methanol or ethanol, or an ether, for example tetrahydrofuran or t-butyl methyl ether. A suitable metal catalyst is, for example, palladium or platinum on an inert support, for example charcoal or barium sulphate.

Preferably a palladium-on-barium sulphate catalyst is used to substantially prevent over-reduction of the alkynyl or alkynylene group to an alkyl or alkylene group respectively. The reaction is generally carried out at a temperature at or near ambient temperature, that is in the range 15 to 35°C.

Alternatively the reduction may be carried out by treating a solution of the alkynyl or alkynylene compound in an inert solvent or diluent with a suitable mixture such as a 1:1 mixture of an organometallic hydride, for example a di-(1-6C)alkylaluminium hydride such as diisobutylaluminium hydride, and an alkyl metal, for example a (1-6C)alkyl lithium such as methyl lithium. A suitable inert solvent or diluent is, for example, tetrahydrofuran, diethyl ether or t-butyl methyl ether and, in general, the reaction is carried out at a temperature, for example, in the range -25°C to ambient temperature (especially -10 to 10°C).

(h) For the production of those compounds of the formula I wherein Q bears an alkyl or substituted alkyl substituent on an available nitrogen atom, or wherein Ar bears an alkoxy or substituted alkoxy substituent, the alkylation of a compound of the formula I wherein Q bears a hydrogen atom on said available nitrogen atom, or wherein Ar bears a hydroxy substituent.

A suitable alkylating agent is, for example any agent known in the art for the alkylation of an available nitrogen atom, or of hydroxy to alkoxy or substituted alkoxy, for example an alkyl or substituted alkyl halide, for example a (1-6C)alkyl chloride, bromide or iodide or a substituted (1-4C)alkyl chloride, bromide or iodide, in the presence of a suitable base. A suitable base for the alkylation reaction is, for example, an alkali or alkaline earth metal carbonate, hydroxide or hydride, for example sodium carbonate, potassium carbonate, sodium hydroxide, potassium hydroxide, sodium hydride or potassium hydride. The alkylation reaction is preferably performed in a suitable inert solvent or diluent, for example N,N-dimethylformamide, dimethylsulphoxide, acetone, 1,2-dimethoxyethane or tetrahydrofuran, and at a temperature in the range, for example, 10 to 150°C, conveniently at or near ambient temperature.

(i) For the production of those compounds of the formula I wherein Q or Ar bears an amino substituent, the reduction of a compound of the formula I wherein Q or Ar bears a nitro substituent.

A suitable reducing agent is, for example, any agent know in the art for the reduction of a nitro group to an amino group. Thus, for example, the reduction may be carried out by the hydrogenation of a solution of the nitro compound in an inert solvent or diluent in the presence of a suitable metal catalyst, for example finely divided platinum metal (obtained by the reduction of platinum oxide in situ). A suitable inert solvent or diluent is, for example an alcohol, for example methanol, ethanol or isopropanol, or an ether, for example tetrahydrofuran.

A further suitable reducing agent is, for example, an activated metal such as activated iron (produced by washing iron powder with a dilute solution of an acid such as hydrochloric acid). Thus, for example, the reduction may be carried out by heating a mixture of the nitro compound and the activated metal in a suitable solvent or diluent such as a mixture of water and an alcohol, for example, methanol or ethanol, to a temperature in the range, for example, 50 to 150°C, conveniently at or near 70°C.

When a pharmaceutically-acceptable salt of a novel compound of the formula I is required, it may be obtained, for example, by reaction of said compound with a suitable acid or base using a conventional procedure. When an optically active form of a compound of the formula I is required, it may be obtained by carrying out one of the aforesaid procedures using an optically active starting material, as illustrated in the accompanying non-limiting Examples, or by resolution of a racemic form of said compound using a conventional procedure.

Many of the intermediates defined herein are novel, for example those of the formulae VI and VII and these are provided as a further feature of the invention.

As stated previously, the heterocyclic cyclic ethers of the formula I are inhibitors of the enzyme 5-LO. The effects of this inhibition may be demonstrated using one or more of the standard procedures set out below:-
a) An in vitro spectrophotometric enzyme assay system, which assesses the inhibitory properties of a test compound in a cell free system using 5-LO isolated from guinea pig neutrophils and as described by D. Aharony and R.L. Stein (J. Biol. Chem., 1986, 261(25), 11512-11519). This test provides a measure of the intrinsic inhibitory properties against soluble 5-LO in an extracellular environment.
b) An in vitro assay system involving incubating a test compound with heparinised human blood, prior to challenge with the calcium ionophore A23187 and then indirectly measuring the inhibitory effects on 5-LO by assaying the amount of LTB₄ using the specific radioimmunoassay described by Carey and Forder (F. Carey and R.A. Forder, Brit. J. Pharmacol. 1985, 84, 34P) which involves the use of a protein-LTB₄ conjugate produced using the procedure of Young et alia (Prostaglandins, 1983, 26(4), 605-613). The effects of a test compound on the enzyme cyclooxygenase (which is involved in the alternative metabolic pathway for arachidonic acid and gives rise to prostaglandins, thromboxanes and related metabolites) may be measured at the same time using the specific radioimmunoassay for thromboxane B₂(TxB₂) described by Carey and Forder (see above). This test provides an indication of the effects of a test compound against 5-LO and also cyclooxygenase in the presence of blood cells and proteins. It permits the selectivity of the inhibitory effect on 5-LO or cyclooxygenase to be assessed.
c) An ex vivo assay system, which is a variation of test b) above, involving administration of a test compound (usually orally as the suspension produced when a solution of the test compound in dimethylsulphoxide is added to carboxymethylcellulose), blood collection, heparinisation, challenge with A23187 and radioimmunoassay of LTB₄ and TxB₂. This test provides an indication of the bioavailability of a test compound as an inhibitor of 5-LO or cyclooxygenase.
d) An in vitro assay system involving the measurement of the inhibitory properties of a test compound against the liberation of LTC₄ and PGE₂ induced by zymosan on mouse resident peritoneal macrophages, using the procedure of Humes (J.L. Humes et alia, Biochem. Pharmacol., 1983, 32, 2319-2322) and conventional radioimmunoassay systems to measure LTC₄ and PGE₂. This test provides an indication of inhibitory effects against 5-LO and cyclooxygenase in a non-proteinaceous system.
e) An in vivo system involving the measurement of the effects of a test compound in inhibiting the inflammatory response to arachidonic acid in the rabbit skin model developed by D. Aked et alia (Brit. J. Pharmacol., 1986, 89, 431-438). This test provides an in vivo model for 5-LO inhibitors administered topically or orally.
f) An in vivo system involving measuring the effects of a test compound administered orally or intravenously on a leukotriene dependent bronchoconstriction induced by an antigen challenge in guinea pigs pre-dosed with an antihistamine (mepyramine), a beta-adrenergic blocking agent (propranolol) and a cyclooxygenase inhibitor (indomethacin), using the procedure of W.H. Anderson et alia (British J Pharmacology, 1983, 78(1), 67-574). This test provides a further in vivo test for detecting 5-LO inhibitors.

Although the pharmacological properties of the compounds of the formula I vary with structural changes as expected, in general compounds of the formula I possess 5-LO inhibitory effects at the following concentrations or doses in one or more of the above tests a)-f):-
- Test a):: IC₅₀ in the range, for example, 0.01-30 micromolar;
- Test b):: IC₅₀ (LTB₄) in the range, for example, 0.01-40 micromolar,
IC₅₀ (TxB₂) in the range, for example, 40-200 micromolar;
- Test c):: oral ED₅₀ (LTB₄) in the range, for example, 1-200 mg/kg;
- Test d):: IC₅₀ (LTC₄) in the range, for example, 0.001-1 micromolar,
IC₅₀ (PGE₂) in the range, for example, 20-1000 micromolar;
- Test e):: inhibition of inflammation in the range, for example, 0.3-100 micrograms intradermally;
- Test f):: ED₅₀ in the range, for example, 0.5-10mg/kg i.v.

No overt toxicity or other untoward effects are present in tests c), e) and/or f) when compounds of the formula I are administered at several multiples of their minimum inhibitory dose or concentration.

Thus, by way of example, the compound 4-ethyl-2,2-dimethyl-4-[3-(3-(2-pyridyl)prop-2-yn-1-yloxy)phenyl]-1,3-dioxolane has an IC₅₀ of 0.8 micromolar against LTB₄ and of >40 micromolar against TxB₂ in test b), and the compound 4-ethyl-4-[5-fluoro-3-(1,2-dihyro-1-methyl-2-oxoquinolin-6-ylmethoxy)phenyl]-2,2-dimethyl-1,3-dioxolane has an IC₅₀ of 0.08 micromolar against LTB₄ in test b). In general those compounds of the formula I which are particularly preferred have an IC₅₀ of <1 micromolar against LTB₄ and of >40 micromolar against TxB₂ in test b), and an oral ED₅₀ of <100 mg/kg against LTB₄ in test c).

These compounds are examples of heterocyclic cyclic ethers of the invention which show selective inhibitory properties for 5-LO as opposed to cyclooxygenase, which selective properties are expected to impart improved therapeutic properties, for example, a reduction in or freedom from the gastrointestinal side-effects frequently associated with cyclooxygenase inhibitors such as indomethacin.

According to a further feature of the invention there is provided a pharmaceutical composition which comprises a heterocyclic cyclic ether of the formula I, or a pharmaceutically-acceptable salt thereof, in association with a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder or a liquid aerosol; for sub-lingual or buccal use, for example a tablet or capsule; or for parenteral use (including intravenous, subcutaneous, intramuscular, intravascular or infusion), for example a sterile aqueous or oily solution or suspension. In general the above compositions may be prepared in a conventional manner using conventional excipients.

The amount of active ingredient (that is a heterocyclic cyclic ether of the formula I or a pharmaceutically-acceptable salt thereof) that is combined with one or more excipients to produce a single dosage form will necessarily vary depending upon the host treated and the particular route of administration. For example, a formulation intended for oral administration to humans will generally contain, for example, from 0.5 mg to 2g of active agent compounded with an appropriate and convenient amount of excipients which may vary from about 5 to about 98 percent by weight of the total composition. Dosage unit forms will generally contain about 1 mg to about 500 mg of an active ingredient.

According to a further feature of the invention there is provided a heterocyclic cyclic ether of the formula I, or a pharmaceutically-acceptable salt thereof, for use in a method of treatment of the human or animal body by therapy.

The invention also includes a method of treating a disease or medical condition mediated alone or in part by one or more leukotrienes which comprises administering to a warm-blooded animal requiring such treatment an effective amount of an active ingredient as defined above. The invention also provides the use of such an active ingredient in the production of a new medicament for use in a leukotriene mediated disease or medical condition.

The size of the dose for therapeutic or prophylactic purposes of an ether of the formula I will naturally vary according to the nature and severity of the conditions, the age and sex of the animal or patient and the route of administration, according to well known principles of medicine. As mentioned above, heterocyclic cyclic ethers of the formula I are useful in treating those allergic and inflammatory conditions which are due alone or in part to the effects of the metabolites of arachidonic acid arising by the linear (5-LO catalysed) pathway and in particular the leukotrienes, the production of which is mediated by 5-LO. As previously mentioned, such conditions include, for example, asthmatic conditions, allergic reactions, allergic rhinitis, allergic shock, psoriasis, atopic dermatitis, cardiovascular and cerebrovascular disorders of an inflammatory nature, arthritic and inflammatory joint disease, and inflammatory bowel diseases.

In using a compound of the formula I for therapeutic or prophylactic purposes it will generally be administered so that a daily dose in the range, for example, 0.5mg to 75mg per kg body weight is received, given if required in divided doses. In general lower doses will be administered when a parenteral route is employed. Thus, for example, for intravenous administration, a dose in the range, for example, 0.5mg to 30 mg per kg body weight will generally be used. Similarly, for administration by inhalation, a dose in the range, for example, 0.5 mg to 25 mg per kg body weight will be used.

Although the compounds of the formula I are primarily of value as therapeutic agents for use in warm-blooded animals (including man), they are also useful whenever it is required to inhibit the enzyme 5-LO. Thus, they are useful as pharmacological standards for use in the development of new biological tests and in the search for new pharmacological agents.

By virtue of their effects on leukotriene production, the compounds of the formula I have certain cytoprotective effects, for example they are useful in reducing or suppressing certain of the adverse gastrointestinal effects of the cyclooxygenase inhibitory non-steroidal anti-inflammatory agents (NSAIA), such as indomethacin, acetylsalicylic acid, ibuprofen, sulindac, tolmetin and piroxicam. Furthermore, co-administration of a 5-LO inhibitor of the formula I with a NSAIA can result in a reduction in the quantity of the latter agent needed to produce a therapeutic effect, thereby reducing the likelihood of adverse side-effects. According to a further feature of the invention there is provided a pharmaceutical composition which comprises a heterocyclic cyclic ether of the formula I, or a pharmaceutically-acceptable salt thereof as defined hereinbefore, in conjunction or admixture with a cyclooxygenase inhibitory non-steroidal anti-inflammatory agent (such as mentioned above), and a pharmaceutically-acceptable diluent or carrier.

The cytoprotective effects of the compounds of the formula I may be demonstrated, for example in a standard laboratory model which assesses protection against indomethacin-induced or ethanol-induced ulceration in the gastrointestinal tract of rats.

The compositions of the invention may in addition contain one or more therapeutic or prophylactic agents known to be of value for the disease under treatment. Thus, for example a known platelet aggregation inhibitor, hypolipidemic agent, anti-hypertensive agent, beta-adrenergic blocker or a vasodilator may usefully also be present in a pharmaceutical composition of the invention for use in treating a heart or vascular disease or condition. Similarly, by way of example, an anti-histamine, steroid (such as beclomethasone dipropionate), sodium cromoglycate, phosphodiesterase inhibitor or a beta-adrenergic stimulant may usefully also be present in a pharmaceutical composition of the invention for use in treating a pulmonary disease or condition.

The compounds of the formula I may also be used in combination with leukotriene antagonists such as those disclosed in European Patent Specifications Nos. 179619, 199543, 220066, 227241, 242167, 290145, 337765, 337766 and 337767, which are incorporated herein by way of reference.

The invention will now be illustrated in the following non-limiting Examples in which, unless otherwise stated:-
(i) evaporations were carried out by rotary evaporations in vacuo and work-up procedures were carried out after removal of residual solids by filtration;
(ii) operations were carried out at room temperature, that is in the range 18-20^{o} and under an atmosphere of an inert gas such as argon;
(iii) column chromatography (by the flash procedure) and medium pressure liquid chromatography (MPLC) were performed on Merck Kieselgel silica (Art. 9385) obtained from E. Meck, Darmstadt, W. Germany;
(iv) yields are given for illustration only and are not necessarily the maximum attainable;
(v) the end-products of the formula I have satisfactory microanalyses and their structures were confirmed by NMR and mass spectral techniques;
(vi) intermediates were not generally fully characterised and purity was assessed by thin layer chromatographic, infra-red (IR) or NMR analysis; and
(vii) melting points are uncorrected and were determined using a Mettler SP62 automatic melting point apparatus or an oil-bath apparatus; melting points for the end-products of the formula I were determined after recrystallisation from a conventional organic solvent such as ethanol, methanol, acetone, ether or hexane, alone or in admixture; and
(viii) the specific rotation, [alpha]^{t}, of plane polarised light was determined using the sodium D line (5890 Angstroms), at 20^{o}C, and generally using sample concentrations of approximately 1g/100ml of solvent.

### Example 1

A mixture of 3-(2-pyridyl)prop-2-yn-1-yl bromide hydrobromide (0.8 g), 2,2-dimethyl-4-ethyl-4-(3-hydroxyphenyl)-1,3-dioxolane (0.58 g), potassium carbonate (0.77 g) and dimethylformamide (5 ml) was stirred at ambient temperature for 15 hours. The mixture was partitioned between methylene chloride and water. The organic layer was washed with a saturated aqueous sodium chloride solution, dried (MgSO₄) and evaporated. The residue was purified by column chromatography using a 1:1 v/v mixture of toluene and ethyl acetate as eluent. There was thus obtained 2,2-dimethyl-4-ethyl-4-[3-(3-(2-pyridyl)prop-2-yn-1-yloxy)phenyl]-1,3-dioxolane (0.55 g, 62%), m.p. 28-29°C.

3-(2-Pyridyl)prop-2-yn-1-yl bromide hydrobromide used as a starting material was obtained as follows:-

2-Propynyl alcohol (35 ml) was added dropwise to a stirred mixture of 2-bromopyridine (23.7 g), bis(triphenylphosphine)palladium chloride (1.54 g), triethylamine (21 ml), cuprous iodide (1.5 g) and acetonitrile (150 ml) and the mixture was stirred at ambient temperature for 30 minutes and then heated to 60°C for 2 hours. The mixture was cooled to ambient temperature, poured into water (200 ml) and neutralised by adding dilute aqueous hydrochloric acid. The mixture was extracted with methylene chloride (2 x 500 ml) and the combined extracts were washed with water (500 ml), dried (MgSO₄) and evaporated. The residue was purified by column chromatography eluting with a 1:1 v/v mixture of methylene chloride and ethyl acetate to give 3-(2-pyridyl)prop-2-yn-1-yl alcohol (14 g, 70%), m.p. 78-80^{o}C (recrystallised from a mixture of hexane and ethyl acetate).

A solution of bromine (3.1 ml) in methylene chloride (3 ml) was added to a mixture of triphenylphosphine (10.1 g) and methylene chloride (72 ml) which had been cooled to -8°C in a salted ice-bath. A solution of the alcohol (4.8 g) obtained immediately above in methylene chloride (36 ml) was added and the mixture was stirred for 10 minutes and cooled to approximately -10°C. The mixture was filtered to give 3-(2-pyridyl)prop-2-yn-1-yl bromide hydrobromide (5.8 g, 58%), m.p. 112-114°C, which was used without further purification.

The 2,2-dimethyl-4-ethyl-4-(3-hydroxyphenyl)-1,3-dioxolane used as a starting material was obtained as follows:-

A solution of 3-benzyloxypropiophenone (7.2 g; J. Med. Chem., 1973, 16, 797) in tetrahydrofuran (30 ml) was added dropwise to a solution of isopropoxydimethylsilylmethylmagnesium chloride [prepared as described in J. Org. Chem., 1983, 48, 2120 from chloromethylisopropoxydimethylsilane (10 g) and magnesium powder (1.46 g) in tetrahydrofuran (15 ml)]. The mixture was stirred at ambient temperature for 2 hours, washed with a saturated aqueous solution of ammonium chloride and then with a saturated aqueous solution of sodium chloride. The organic layer was separated, dried (Na₂SO₄) and evaporated to give 1-isopropoxydimethylsilyl-2-[3-benzyloxyphenyl]butan-2-ol as a yellow oil.

A mixture of the product so obtained, sodium bicarbonate (2.52 g), hydrogen peroxide (27 ml, 30% w/v in water), methanol (75 ml) and tetrahydrofuran (45 ml) was heated to reflux for 15 hours. The mixture was evaporated to remove the organic solvents and the residue was extracted with diethyl ether. The organic layer was separated, washed with a saturated aqueous solution of sodium chloride, dried (MgSO₄) and evaporated. The residue was purified by column chromatography using initially methylene chloride and then increasingly polar mixtures of methylene chloride and acetone, up to a 9:1 v/v mixture, as eluent. There was thus obtained 2-(3-benzyloxyphenyl)butane-1,2-diol (3.4 g, 42%), m.p. 74-75°C.

A mixture of the product so obtained (3.4 g), concentrated sulphuric acid (2 drops) and acetone (90 ml) was stirred at ambient temperature for 2 hours. The mixture was neutralised by adding 2N aqueous sodium hydroxide solution and evaporated. The residue was purified by column chromatography using a 9:1 v/v mixture of methylene chloride and diethyl ether as eluent. There was thus obtained 4-(3-benzyloxyphenyl)-2,2-dimethyl-4-ethyl-1,3-dioxolane (3.3 g, 89%), m.p. 51-53°C.

A solution of the product so obtained in ethanol (50 ml) was hydrogenated in the presence of 10% palladium-on-charcoal catalyst and under a gas pressure of 30 pounds per square inch. The calculated volume of hydrogen was consumed over 5 hours. The mixture was filtered and the filtrate was evaporated. The residue was purified by column chromatography using methylene chloride as eluent. There was thus obtained 2,2-dimethyl-4-ethyl-4-(3-hydroxyphenyl)-1,3-dioxolane (1.7 g, 70%) as a colourless oil.

### Example 2

Using the procedure described in Example 1, the appropriate alkyl bromide was reacted with the appropriate phenol. There were thus obtained the compounds described in the following table:-

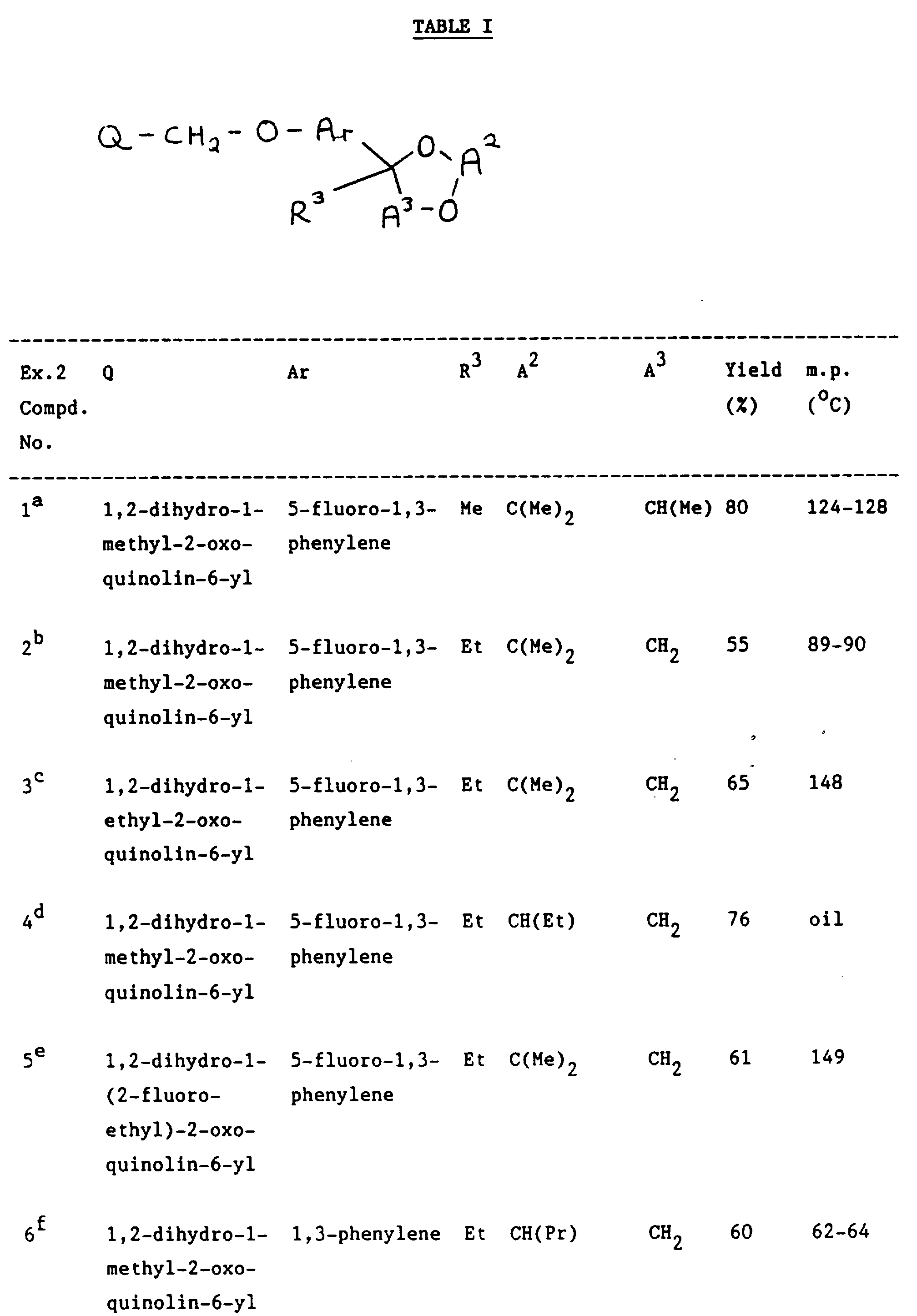

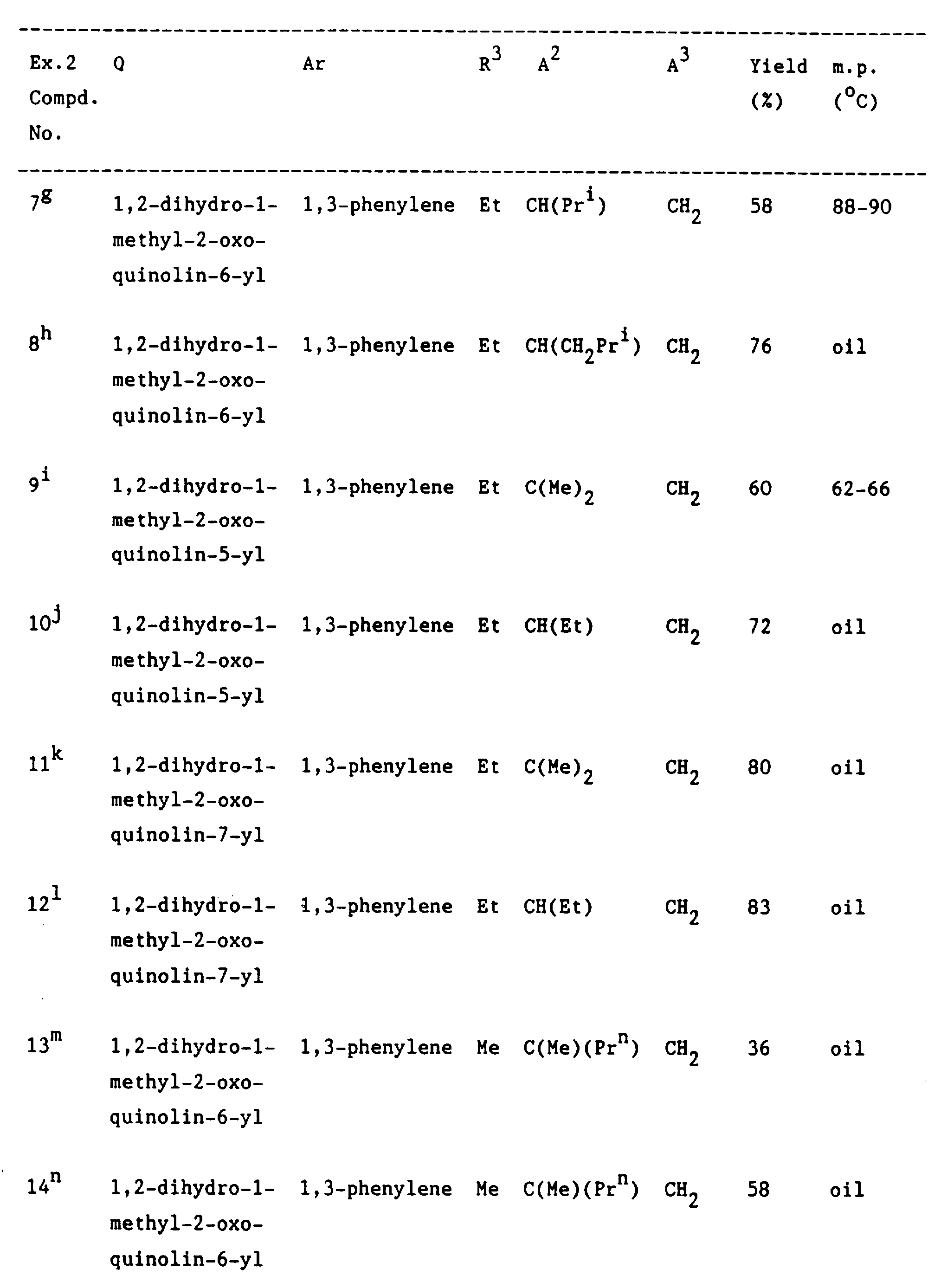

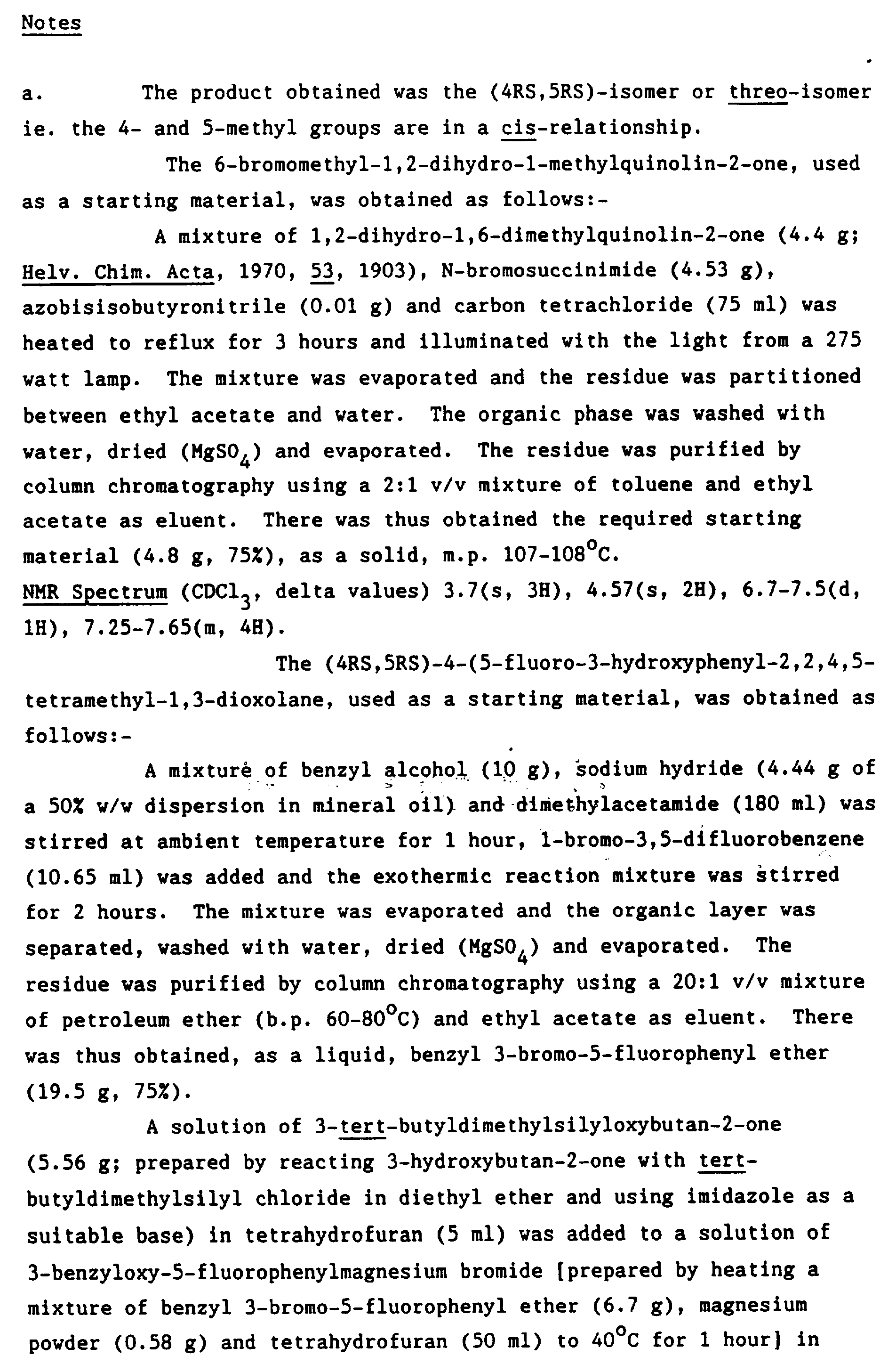

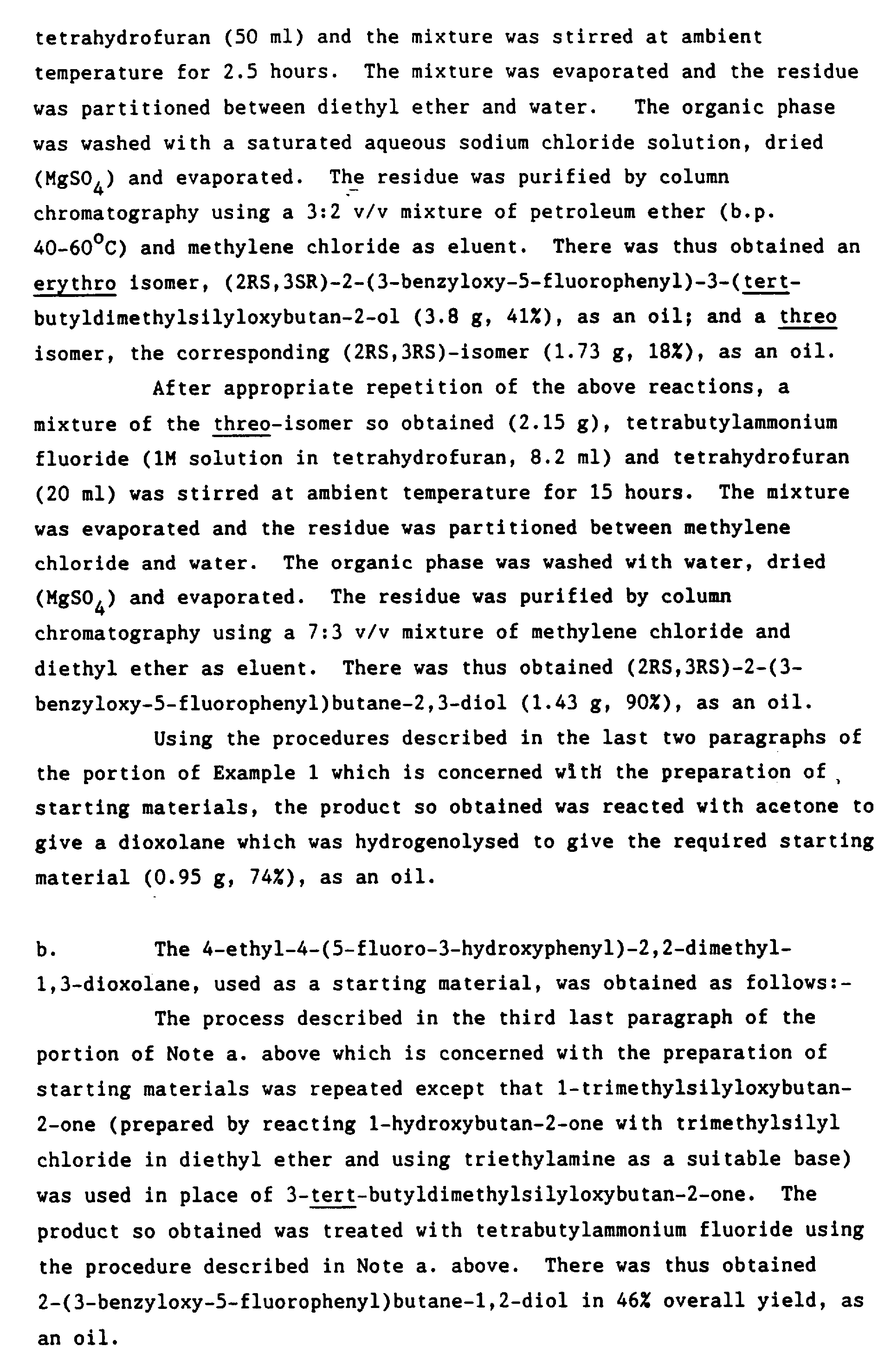

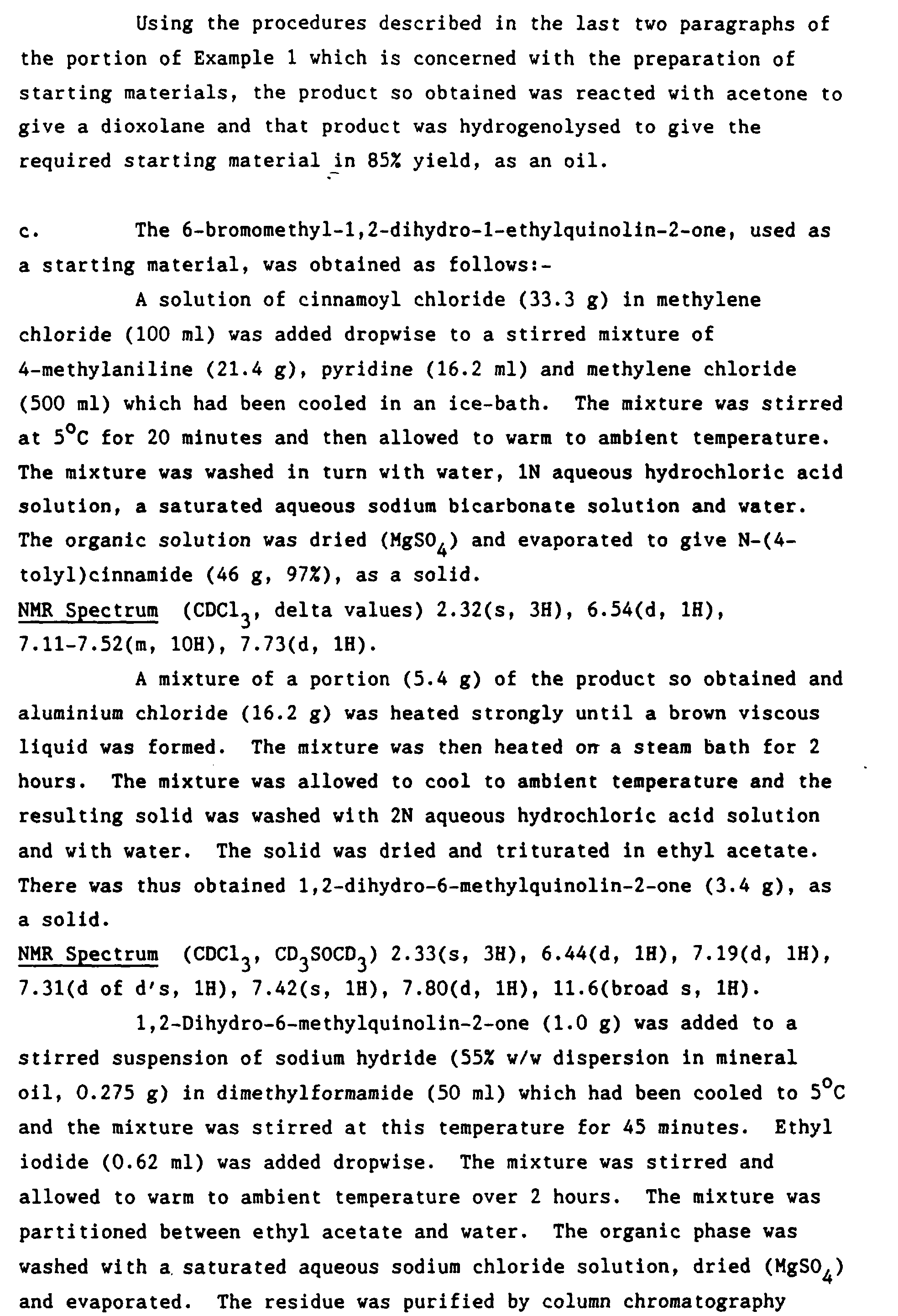

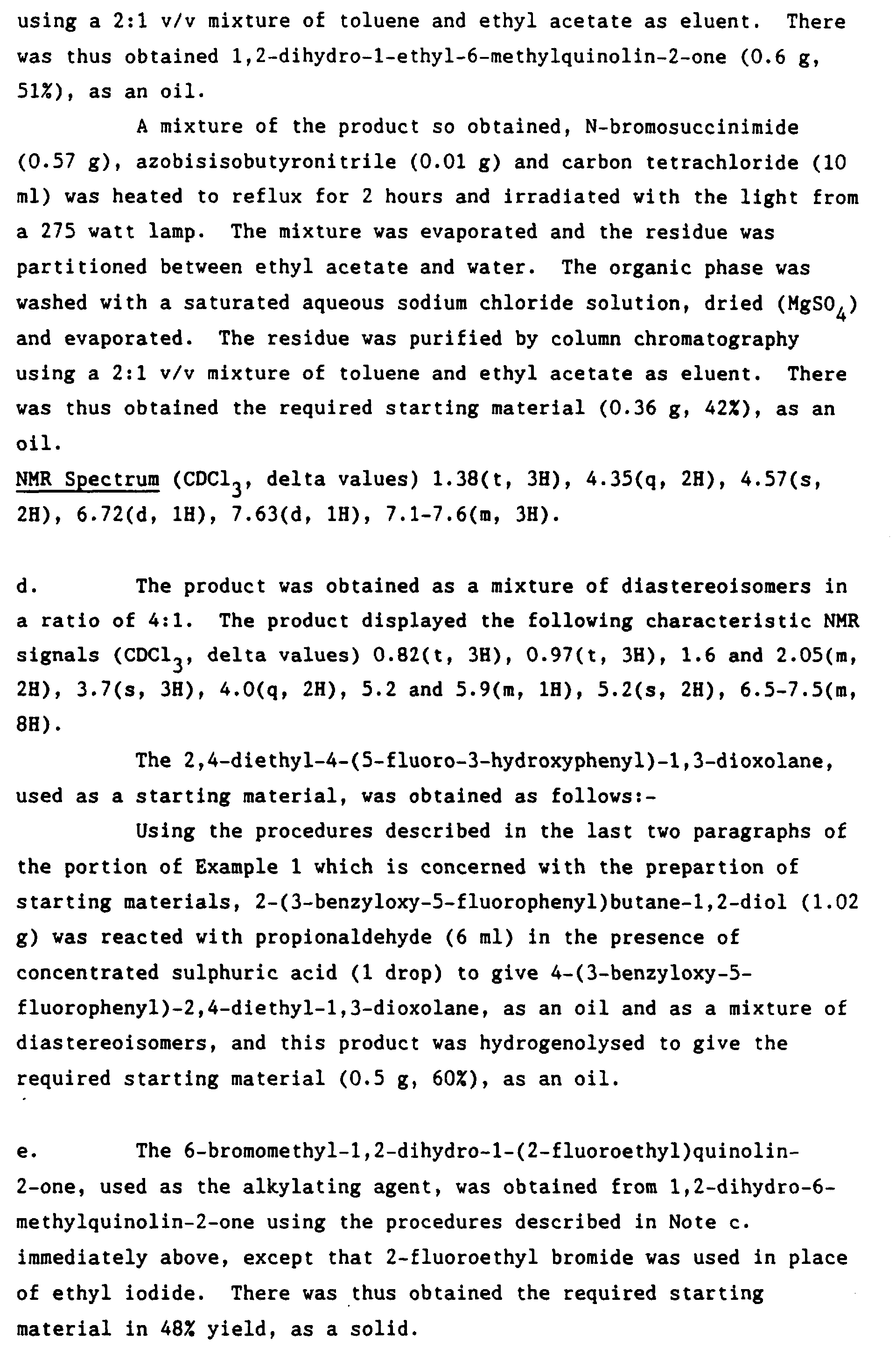

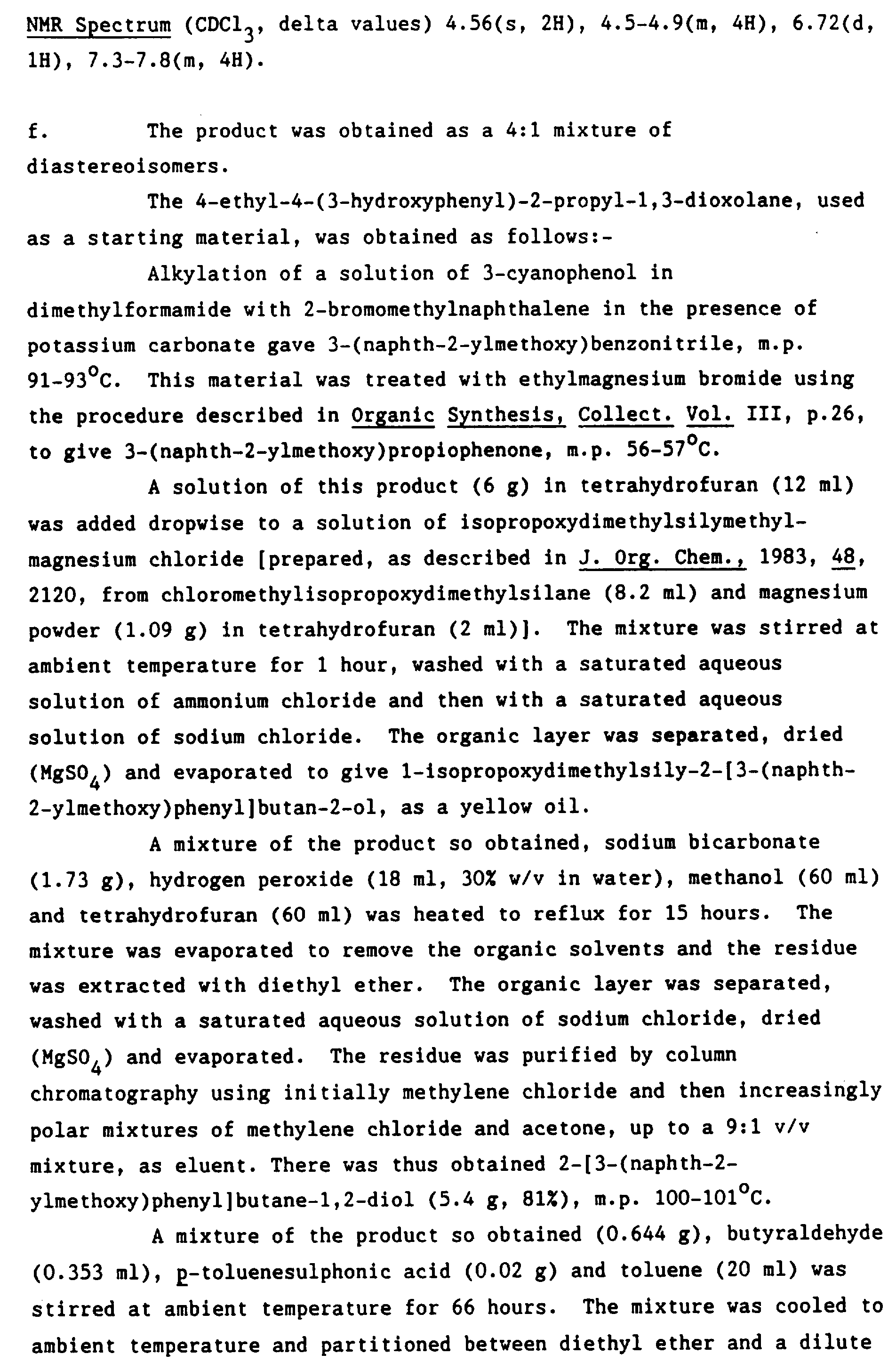

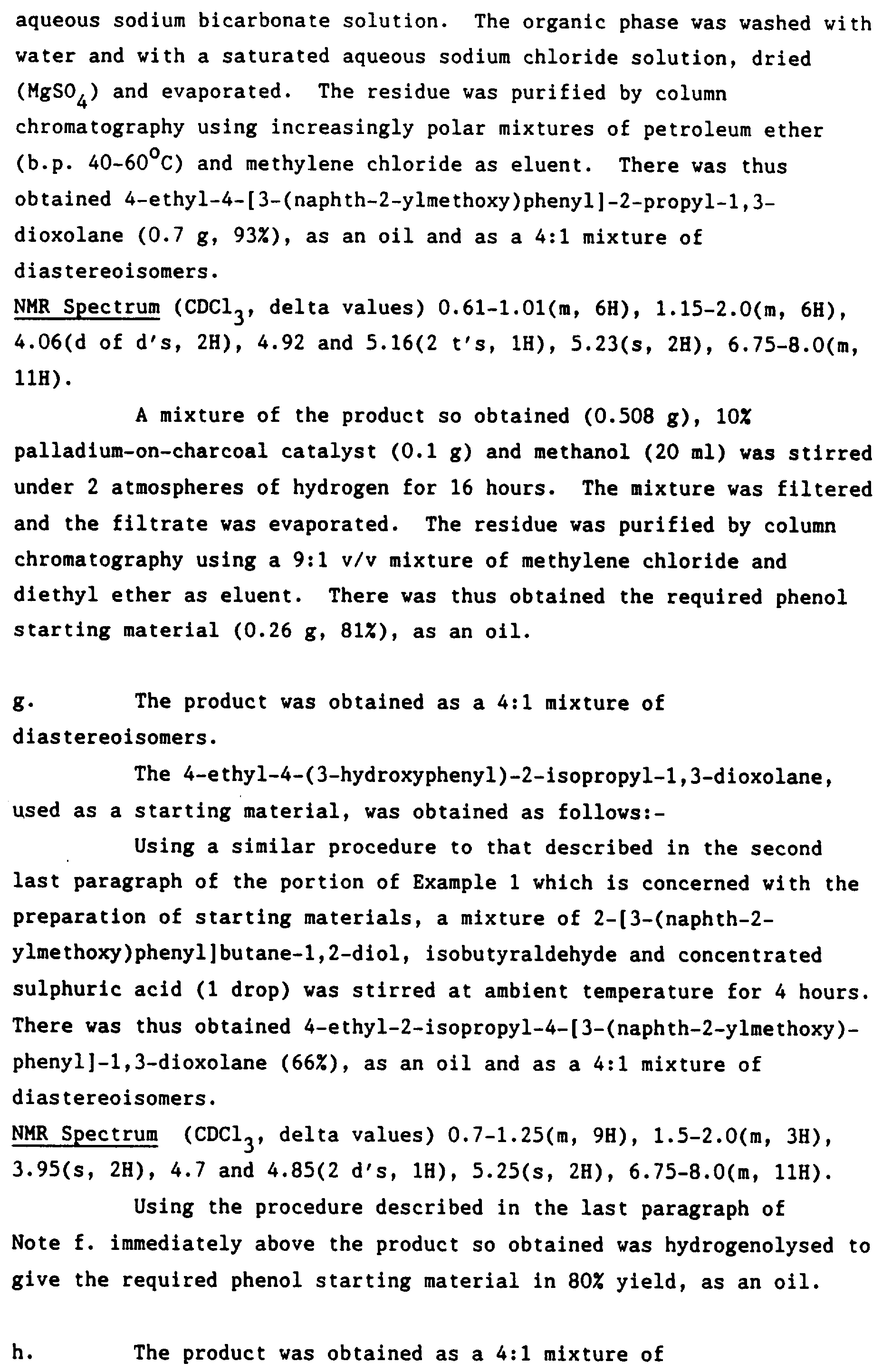

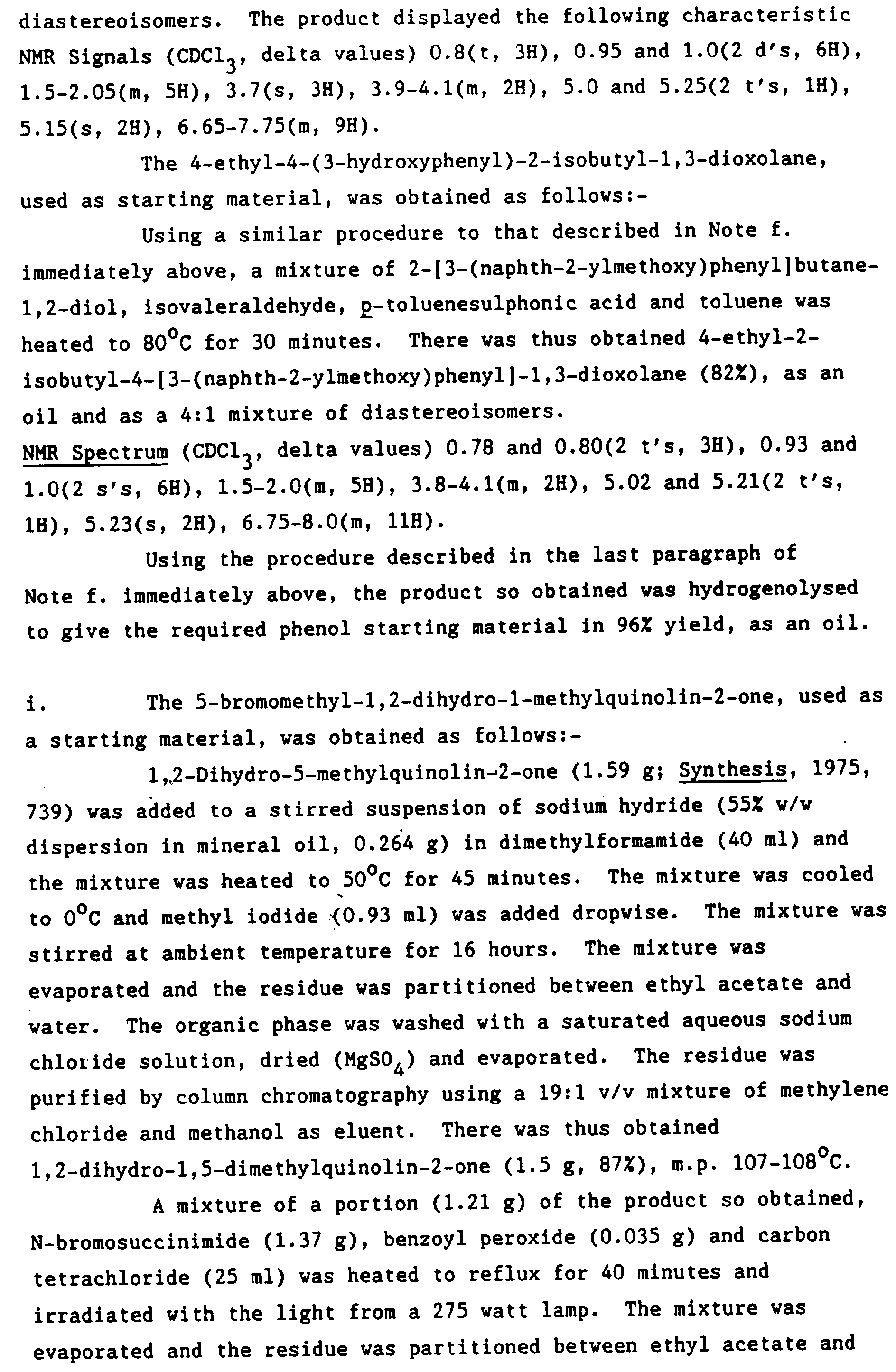

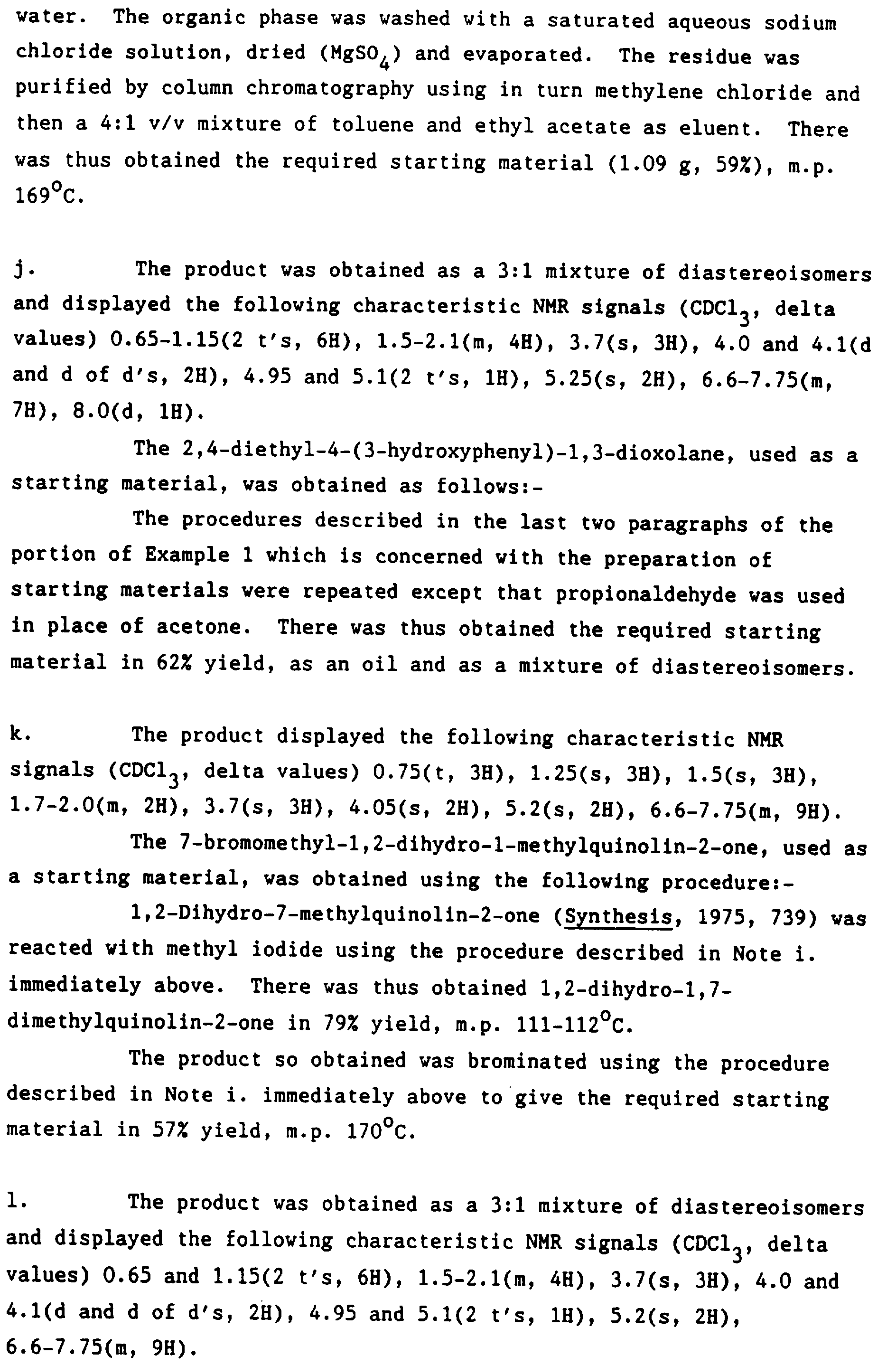

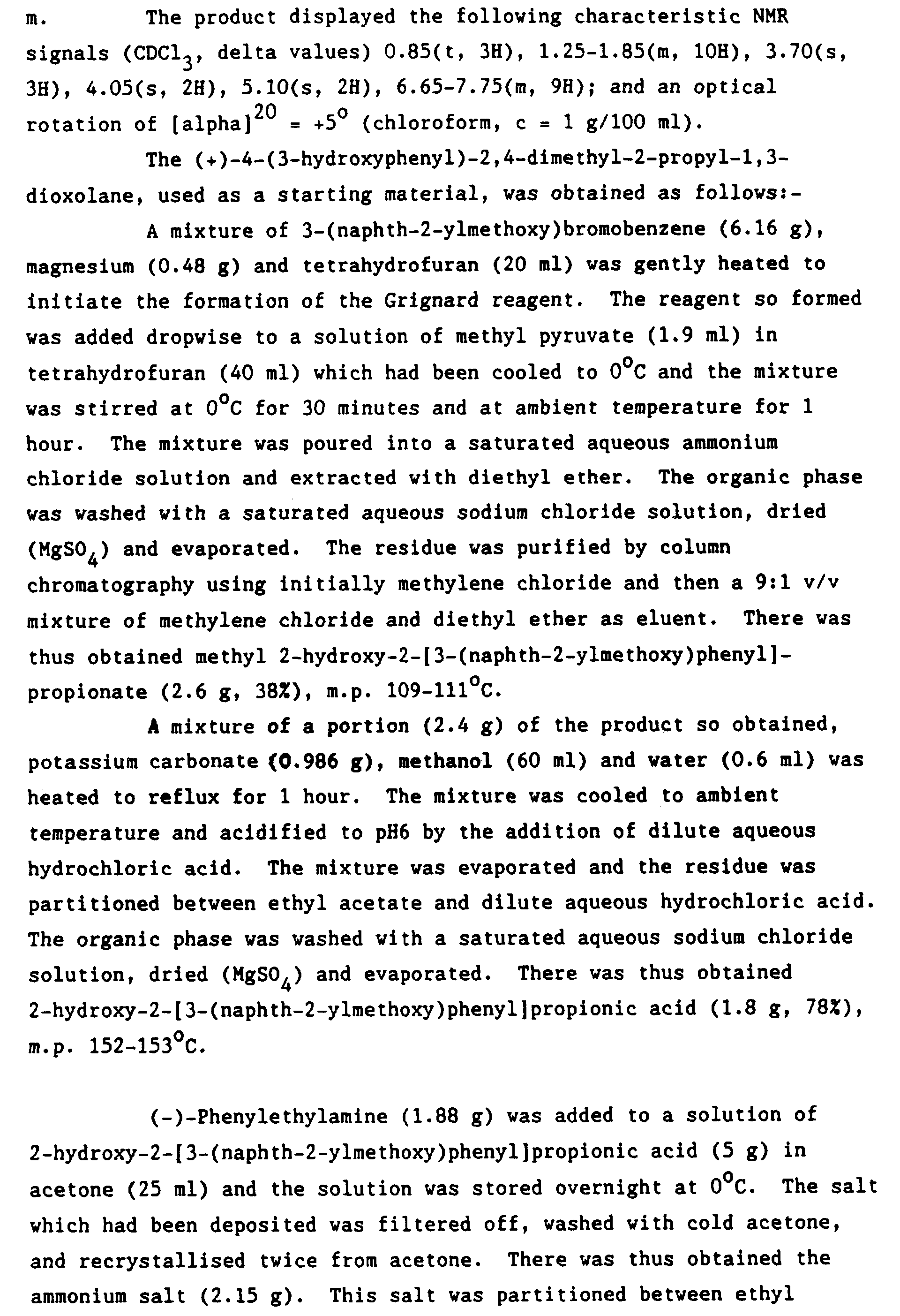

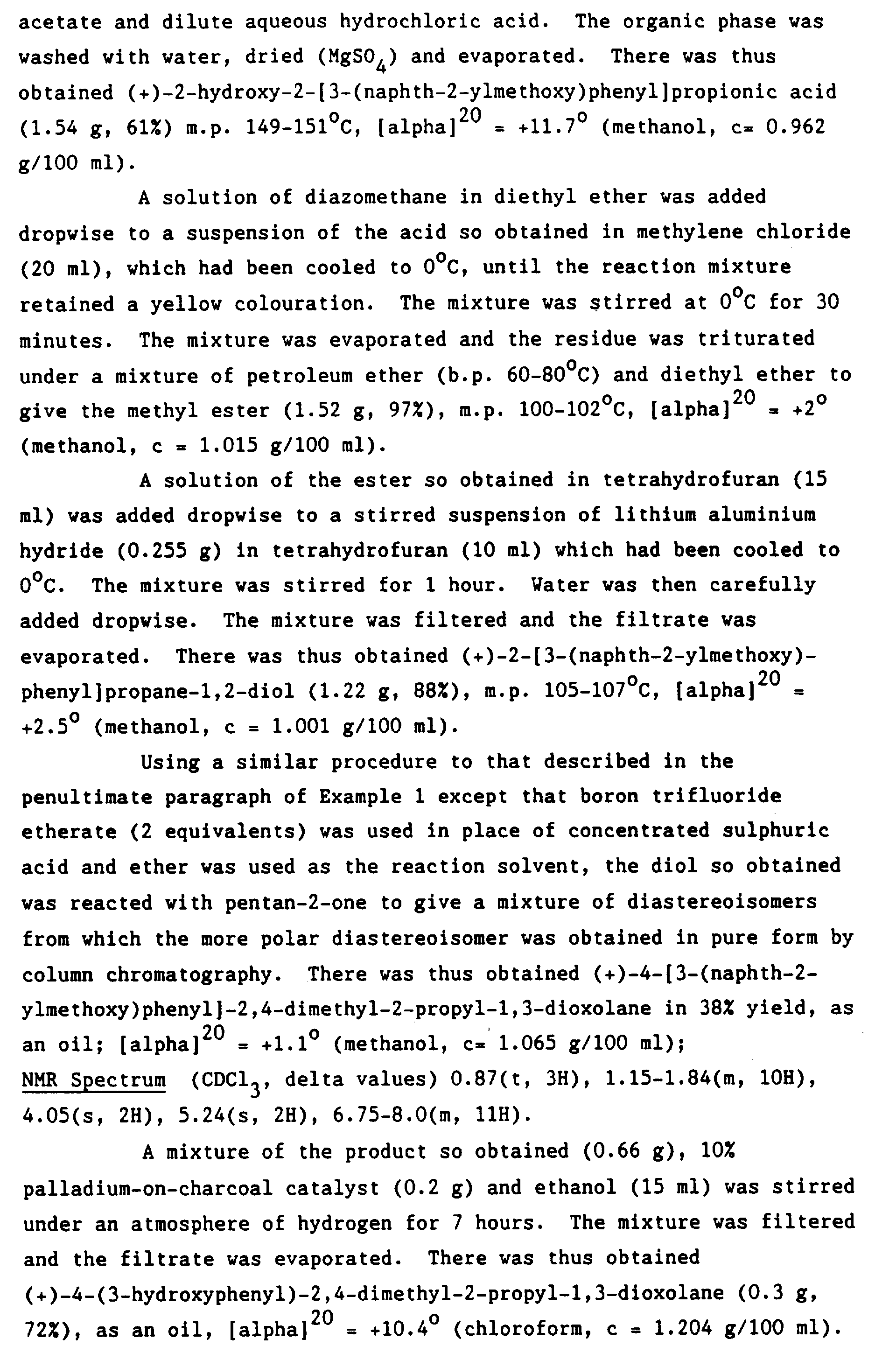

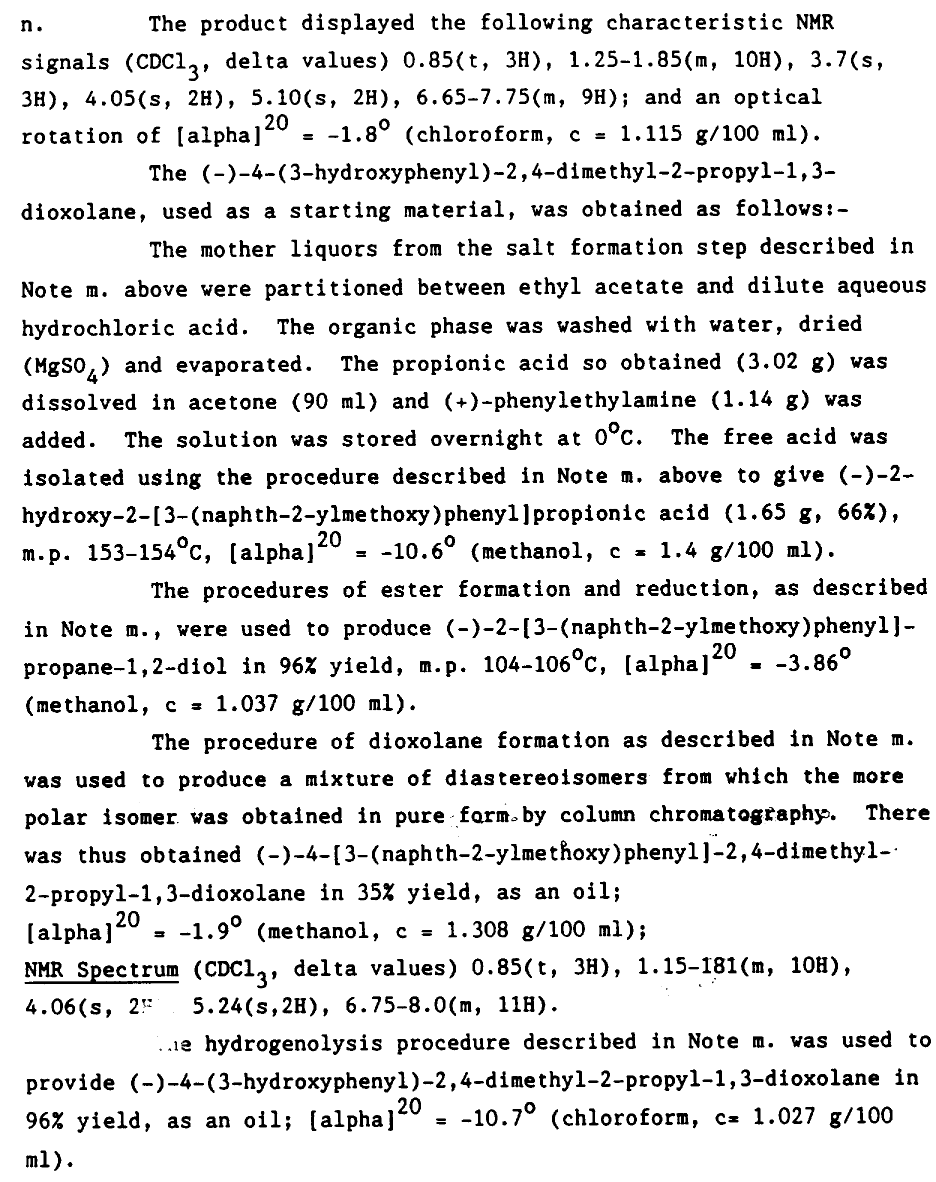

### Example 3

The following illustrate representative pharmaceutical dosage forms containing the compound of formula I, or a pharmaceutically-acceptable salt salt thereof (hereafter compound X), for therapeutic or prophylactic use in humans:
(a)

| Tablet I | mg/tablet |
|---|---|
| Compound X | 100 |
| Lactose Ph.Eur | 182.75 |
| Croscarmellose sodium | 12.0 |
| Maize starch paste (5% w/v paste) | 2.25 |
| Magnesium stearate | 3.0 |

(b)

| Tablet II | mg/tablet |
|---|---|
| Compound X | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |

(c)

| Tablet III | mg/tablet |
|---|---|
| Compound X | 1.0 |
| Lactose Ph.Eur | 93.25 |
| Croscarmellose sodium | 4.0 |
| Maize starch paste (5% w/v paste) | 0.75 |
| Magnesium stearate | 1.0 |

(d)

| Capsule | mg/capsule |
|---|---|
| Compound X | 10 mg |
| Lactose Ph.Eur | 488.5 |
| Magnesium stearate | 1.5 |

(e)

| Injection I | (50 mg/ml) |
|---|---|
| Compound X | 5.0% w/v |
| 1M Sodium hydroxide solution | 15.0% v/v |
| 0.1M Hydrochloric acid (to adjust pH to 7.6) | |
| Polyethylene glycol 400 | 4.5% w/v |
| Water for injection to 100% | |

(f) 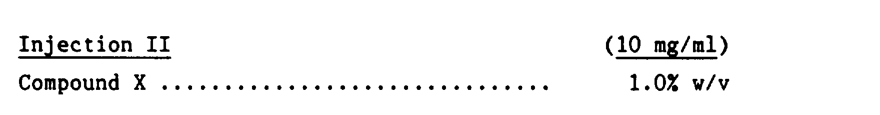 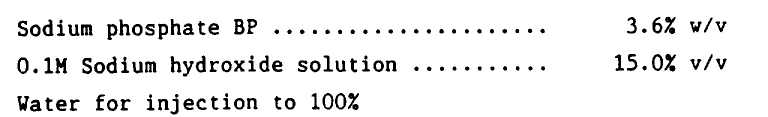
(g)

| Injection III | (1mg/ml, buffered to pH6) |
|---|---|
| Compound X | 0.1% w/v |
| Sodium phosphate BP | 2.26% w/v |
| Citric acid | 0.38% w/v |
| Polyethylene glycol 400 | 3.5% w/v |
| Water for injection to 100% | |

(h)

| Aerosol I | mg/ml |
|---|---|
| Compound X | 10.0 |
| Sorbitan trioleate | 13.5 |
| Trichlorofluoromethane | 910.0 |
| Dichlorodifluoromethane | 490.0 |

(i)

| Aerosol II | mg/ml |
|---|---|
| Compound X | 0.2 |
| Sorbitan trioleate | 0.27 |
| Trichlorofluoromethane | 70.0 |
| Dichlorodifluoromethane | 280.0 |
| Dichlorotetrafluoroethane | 1094.0 |

(j)

| Aerosol III | mg/ml |
|---|---|
| Compound X | 2.5 |
| Sorbitan trioleate | 3.38 |
| Trichlorofluoromethane | 67.5 |
| Dichlorodifluoromethane | 1086.0 |
| Dichlorotetrafluoroethane | 191.6 |

(k)

| Aerosol IV | mg/ml |
|---|---|
| Compound X | 2.5 |
| Soya lecithin | 2.7 |
| Trichlorofluoromethane | 67.5 |
| Dichlorodifluoromethane | 1086.0 |
| Dichlorotetrafluoroethane | 191.6 |

### Note

The above formulations may be obtained by conventional procedures well known in the pharmaceutical art. The tablets (a)-(c) may be enteric coated by conventional means, for example to provide a coating of cellulose acetate phthalate. The aerosol formulations (h)-(k) may be used in conjunction with standard, metered dose aerosol dispensers, and the suspending agents sorbitan trioleate and soya lecithin may be replaced by an alternative suspending agent such as sorbitan monooleate, sorbitan sesquioleate, polysorbate 80, polyglycerol oleate or oleic acid.

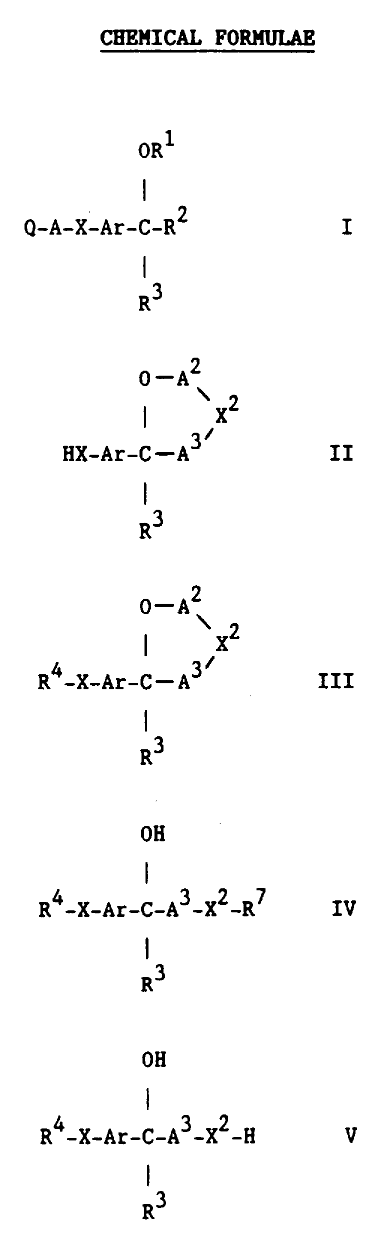

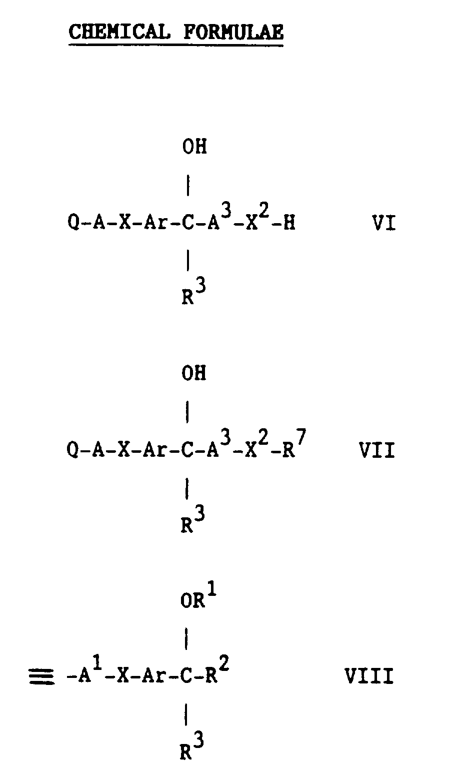

## Claims (Claims for the following Contracting State(s): AT, BE, CH, DE, DK, FR, GB, IT, LI, LU, NL, SE)

1. A heterocyclic cyclic ether of the formula I 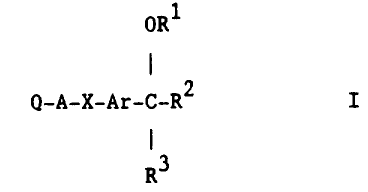 wherein Q is a 6-membered monocyclic or 10-membered bicyclic heterocyclic moiety containing one or two nitrogen atoms which may optionally bear one, two or three substituents selected from halogeno, hydroxy, oxo, carboxy, cyano, amino, (1-4C)alkyl, (1-4C)alkoxy, fluoro-(1-4C)alkyl, (1-4C)alkylamino, di-[(1-4C)alkyl]amino, hydroxy-(1-4C)alkyl, amino-(1- 4C)alkyl, (1-4C)alkylamino-(1-4C)alkyl, di-[(1-4C)alkyl]amino-(1-4C)alkyl, amino-(2-4C)alkoxy, (1-4C)alkylamino-(2-4C)alkoxy, di-[(1-4C)alkyl]amino-(2-4C)alkoxy and phenyl-(1-4C)alkyl;
wherein A is (1-6C)alkylene, (3-6C)alkenylene, (3-6C)alkynylene or cyclo(3-6C)alkylene;
wherein X is oxy, thio, sulphinyl, sulphonyl or imino;
wherein Ar is phenylene which may optionally bear one or two substituents selected from halogeno, hydroxy, amino, nitro, cyano, ureido, carbamoyl, (1-4C)alkyl, (3-4C)alkenyloxy, (1-4C)alkoxy, (1-4C)alkylthio, (1-4C)alkylsulphinyl, (1-4C)alkylsulphonyl, (1-4C)alkylamino, di-[(1-4C)alkyl]amino, fluoro-(1-4C)alkyl, (1-4C)alkoxycarbonyl, N-[(1-4C)alkyl]carbamoyl, N,N-di-[(1-4C)alkyl]carbamoyl, (2-4C)alkanoylamino, cyano-(1-4C)alkoxy; carbamoyl-(1-4C)alkoxy, amino-(2-4C)alkoxy, (1-4C)alkylamino-(2-4C)alkoxy, di-[(1-4C)alkyl]amino-(2-4C)alkoxy and (1-4C)alkoxycarbonyl-(1-4C)alkoxy; or
Ar is a 6-membered heterocyclene moiety containing up to three nitrogen atoms which may optionally bear one or two substituents selected from halogeno, hydroxy, amino, cyano, (1-4C)alkyl, (1-4C)alkoxy, (1-4C)alkylamino and di-[(1-4C)alkyl]amino;
wherein R¹ and R together form a group of the formula -A-X-A³- which, together with the oxygen atom to which A is attached and with the carbon atom to which A³ is attached, defines a ring having 5 to 7 ring atoms, wherein A and A³, which may be the same or different, each is (1-3C)alkylene and X is oxy, thio, sulphinyl, sulphonyl or imino, and which ring may bear one, two or three substituents, which may be the same or different, selected from hydroxy, (1-4C)alkyl, (1-4C)alkoxy, (1-4C)alkylthio, (1-4C)alkylsulphinyl and (1-4C)alkylsulphonyl or which ring may bear a (1-4C)alkylenedioxy substituent, and wherein R³ is (1-6C)alkyl, (2-6C)alkenyl, (2-6C)alkynyl, fluoro-(1-4C)alkyl, cyano-(1-4C)alkyl, hydroxy-(1-4C)alkyl, (1-4C)alkoxy-(1-4C)alkyl or (2-4C)alkanoyloxy(1-4C)alkyl; or a pharmaceutically-acceptable salt thereof.

2. A heterocyclic cyclic ether of the formula I as claimed in claim1 wherein Q is pyridyl, pyrimidinyl, pyrazinyl, quinolyl, isoquinolyl, quinazolinyl or quinoxalinyl which may optionally bear one, two or three substituents selected from fluoro, chloro, hydroxy, oxo, methyl, ethyl, propyl, trifluoromethyl, 2-fluoroethyl, 2-dimethylaminoethyl and benzyl;
wherein A is methylene, 1-propenylene or 1-propynylene;
wherein X is oxy;
wherein Ar is 1,3-phenylene or 1,4-phenylene which may optionally bear one or two substituents selected from fluoro, chloro, hydroxy, amino, methoxy and trifluoromethyl, or
Ar is 3,5-pyridylene;
wherein R¹ and R together form a group of the formula -A-X-A³- which, together with the oxygen atom to which A is attached and with the carbon atom to which A³ is attached, defines a ring having 5 or 6 ring atoms, wherein A is methylene, A³ is methylene or ethylene and X is oxy, and which ring may bear one, two or three substituents selected from methyl, ethyl, propyl, isopropyl, butyl, isobutyl and sec-butyl, and R³ is methyl or ethyl;
or a pharmaceutically-acceptable salt thereof.

3. A heterocyclic cyclic ether of the formula I as claimed in claim 1 wherein Q is 2-pyridyl, 3-pyridyl, 3-quinolyl, 5-quinolyl, 6-quinolyl, 7-quinolyl or 6-quinoxalinyl which may optionally bear one or two substituents selected from hydroxy, oxo, methyl, ethyl, propyl, 2-fluoroethyl, 2-dimethylaminoethyl and benzyl;
wherein A is methylene, 1-propenylene or 1-propynylene;
wherein X is oxy;
wherein Ar is 1,3-phenylene or 1,4-phenylene which may optionally bear one or two substituents selected from fluoro, chloro, amino, methoxy and trifluoromethyl, or Ar is 3,5-pyridylene;
wherein R¹ and R together form a group of the formula -A-X-A³- which, together with the oxygen atom to which A is attached and with the carbon atom to which A³ is attached, defines a ring having 5 ring atoms, wherein A is methylene, A³ is methylene and X is oxy, and which ring may bear one, two or three substituents selected from methyl, ethyl, propyl, isopropyl, butyl, isobutyl and sec-butyl, and R³ is methyl or ethyl;
or a pharmaceutically-acceptable salt thereof.

4. A heterocyclic cyclic ether of the formula I as claimed in claim 1 wherein Q is 2-pyridyl, 1,2-dihydro-1-methyl-2-oxoquinolin-3-yl, 2-quinolyl, 3-quinolyl, 1,2-dihydro-2-oxoquinolin-3-yl, 3-isoquinolyl or 6-quinoxalinyl;
A is methylene or 1-propynylene;
Ar is 1,3-phenylene or 5-fluoro-1,3-phenylene; and
wherein R¹ and R together form a group of the formula -A-X-A³- with which, together with the oxygen atom to which A is attached and with the carbon atom to which A³ is attached, defines a ring having 5 ring atoms, wherein each of A and A³ is methylene, X is oxy, and which ring may bear one or two methyl substituents, and R³ is ethyl; or a pharmaceutically-acceptable salt thereof.

5. A heterocyclic cyclic ether of the formula I as claimed in claim 1 wherein Q is 1,2-dihydro-2-oxoquinolin-5-yl, 1,2-dihydro-2-oxoquinolin-6-yl or 1,2-dihydro-2-oxoquinolin-7-yl which bears a 1-substituent selected from methyl, ethyl and 2-fluoroethyl;
wherein A is methylene;
wherein X is oxy;
wherein Ar is 1,3-phenylene which may optionally bear one fluoro substituent;
wherein R¹ and R together form a group of the formula -A-X-A³- which, together with the oxygen atom to which A is attached and with the carbon atom to which A³ is attached defines a ring having 5 ring atoms, wherein A is methylene, A³ is methylene and X is oxy, and which ring may bear one or two substituents selected from methyl, ethyl, propyl and isopropyl, and R³ is methyl or ethyl;
or a pharmaceutically-acceptable salt thereof.

6. A heterocyclic cyclic ether of the formula I as claimed in claim 1 selected from the group consisting of :-
4-ethyl-2,2-dimethyl-4-[3-(3-(2-pyridyl)prop-2-yn-1-yloxy)phenyl]-1,3-dioxolane,
4-ethyl-4-[5-fluoro-3-(1,2-dihydro-1-methyl-2-oxoquinolin-6-ylmethoxy)phenyl]-2,2-dimethyl-1,3-dioxolane and
2,4-diethyl-4-[5-fluoro-3-(1,2-dihydro-1-methyl-2-oxoquinolin-6-ylmethoxy)phenyl]-1,3-dioxolane;
or a pharmaceutically-acceptable salt thereof.

7. A process for the manufacture of a heterocyclic cyclic ether of the formula I, or a pharmaceutically-acceptable salt thereof, as claimed in claim 1 which comprises :-
(a) the alkylation, in the presence of a suitable reagent, of a compound of the formula II 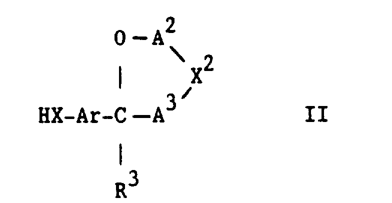 with a compound of the formula Q-A-Z wherein Z is a displaceable group; provided that, when there is an amino, imino, alkylamino, hydroxy or carboxy group in Q, Ar, X or R³, any amino, imino, alkylamino or carboxy group is protected by a conventional protecting group and any hydroxy group may be protected by a conventional protecting group or alternatively any hydroxy group need not be protected;
whereafter any undesired protecting group in Q, Ar, X or R³ is removed by conventional means;
(b) the cyclisation, in the presence of a suitable base, of a compound of the formula VI 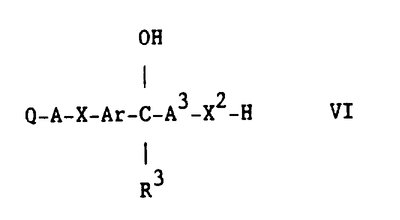 by reaction with a compound of the formula Z-A-Z, wherein A and Z have the meanings defined hereinbefore, provided that, when there is an amino, imino, alkylamino, hydroxy or carboxy group in Q, X, Ar or R³, any amino, imino, alkylamino, hydroxy or carboxy group is protected by a conventional protecting group;
whereafter any undesired protecting group in Q, X, Ar or R³ is removed by conventional means;
(c) the cyclisation, in the presence of a suitable acid, of a compound of the formula VI 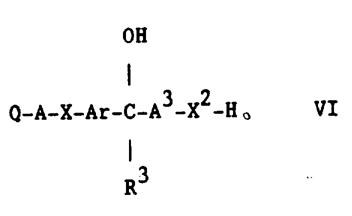 by reaction with an appropriate aldehyde or with an appropriate ketone, or with corresponding hemiacetal or acetal derivatives thereof, provided that, when there is an amino, imino, alkylamino, hydroxy or carboxy group in Q, X, Ar or R³, any imino, amino, alkylamino, hydroxy or carboxy group is protected by a conventional protecting group;
whereafter any undesired protecting group in Q, X, Ar or R³ is removed by conventional means;
(d) for the production of those compounds of the formula I wherein A is a (3-6C)alkynylene group, the coupling, in the presence of a suitable organometallic catalyst, of a heterocyclic compound of the formula Q-Z, wherein Q has the meaning defined hereinbefore and Z is a halogeno group, with an ethynyl compound of the formula VIII 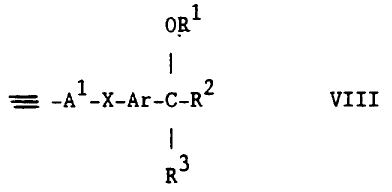 wherein A¹ is (1-4C)alkylene and X, Ar, R¹, R and R³ have the meanings defined hereinbefore;
(e) for the production of those compounds of the formula I wherein Ar bears an alkylsulphinyl or alkylsulphonyl substituent, wherein X is a sulphinyl or sulphonyl group, or wherein R¹ and R together form a group of the formula -A-X-A³-, X is a sulphinyl or sulphonyl group and which group may bear one or two alkylsulphinyl or alkylsulphonyl groups, the oxidation of a compound of the formula I wherein Ar bears an alkylthio substituent, wherein X is a thio group, or wherein R¹ and R together form a group of the formula -A-X-A³-, X is a thio group, and which group may bear one or two alkylthio groups;
(f) for the production of those compounds of the formula I wherein Ar bears an alkanoylamino substituent, the acylation of a compound of the formula I wherein Ar bears an amino substituent;
(g) for the production of those compounds of the formula I wherein A is alkenylene, or wherein R¹ and R together from a group of the formula -A-X-A³- and R³ is alkenyl, the reduction of the corresponding compound wherein A is alkynylene or R³ is alkynyl;
(h) for the production of those compounds of the formula I wherein Q bears an alkyl or substituted alkyl substituent on an available nitrogen atom, or wherein Ar bears an alkoxy or substituted alkoxy substituent, the alkylation of a compound of the formula I wherein Q bears a hydrogen atom on said available nitrogen atom, or wherein Ar bears a hydroxy substituent; or
(i) for the production of those compounds of the formula I wherein Q or Ar bears an amino substituent, the reduction of a compound of the formula I wherein Q or Ar bears a nitro substituent;
and when a pharmaceutically-acceptable salt of a novel compound of the formula I is required, it may be obtained by reaction of said compound with a suitable acid or base using a conventional procedure.

8. A pharmaceutical composition which comprises a heterocyclic cyclic ether of the formula I, or a pharmaceutically-acceptable salt thereof, as claimed in any one of claims 1 to 6 in association with a pharmaceutically-acceptable diluent or carrier.

9. A heterocyclic cyclic ether of the formula I, or a pharmaceutically-acceptable salt thereof, as claimed in any one of claims 1 to 6 for use in a method of treatment of the human or animal body by therapy.

10. The use of a heterocyclic cyclic ether of the formula I, or a pharmaceutically-acceptable salt thereof, as claimed in any one of claims 1 to 6 in the production of a new medicament for use in a leukotriene mediated disease or medical condition.

## Claims (Claims for the following Contracting State(s): ES, GR)

1. A process for the manufacture of a heterocyclic cyclic ether of the formula I 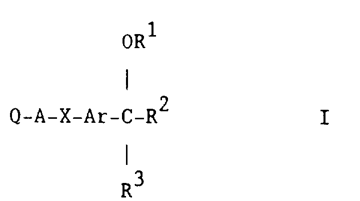 wherein Q is a 6-membered monocyclic or 10-membered bicyclic heterocyclic moiety containing one or two nitrogen atoms which may optionally bear one, two or three substituents selected from halogeno, hydroxy, oxo, carboxy, cyano, amino, (1-4C)alkyl, (1-4C)alkoxy, fluoro-(1-4C)alkyl, (1-4C)alkylamino, di-[(1-4C)alkyl]amino, hydroxy-(1-4C)alkyl, amino-(1- 4C)alkyl, (1-4C)alkylamino-(1-4C)alkyl, di-[(1-4C)alkyl]amino-(1-4C)alkyl, amino-(2-4C)alkoxy, (1-4C)alkylamino-(2-4C)alkoxy, di-[(1-4C)alkyl]amino-(2-4C)alkoxy and phenyl-(1-4C)alkyl;
wherein A is (1-6C)alkylene, (3-6C)alkenylene, (3-6C)alkynylene or cyclo(3-6C)alkylene;
wherein X is oxy, thio, sulphinyl, sulphonyl or imino;
wherein Ar is phenylene which may optionally bear one or two substituents selected from halogeno, hydroxy, amino, nitro, cyano, ureido, carbamoyl, (1-4C)alkyl, (3-4C)alkenyloxy, (1-4C)alkoxy, (1-4C)alkylthio, (1-4C)alkylsulphinyl, (1-4C)alkylsulphonyl, (1-4C)alkylamino, di-[(1-4C)alkyl]amino, fluoro-(1-4C)alkyl, (1-4C)alkoxycarbonyl, N-[(1-4C)alkyl]carbamoyl, N,N-di-[(1-4C)alkyl]carbamoyl, (2-4C)alkanoylamino, cyano-(1-4C)alkoxy, carbamoyl-(1-4C)alkoxy, amino-(2-4C)alkoxy, (1-4C)alkylamino-(2-4C)alkoxy, di-[(1-4C)alkyl]amino-(2-4C)alkoxy and (1-4C)alkoxycarbonyl-(1-4C)alkoxy; or
Ar is a 6-membered heterocyclene moiety containing up to three nitrogen atoms which may optionally bear one or two substituents selected from halogeno, hydroxy, amino, cyano, (1-4C)alkyl, (1-4C)alkoxy, (1-4C)alkylamino and di-[(1-4C)alkyl]amino;
wherein R¹ and R together form a group of the formula -A-X-A³- which, together with the oxygen atom to which A is attached and with the carbon atom to which A³ is attached, defines a ring having 5 to 7 ring atoms, wherein A and A³, which may be the same or different, each is (1-3C)alkylene and X is oxy, thio, sulphinyl, sulphonyl or imino, and which ring may bear one, two or three substituents, which may be the same or different, selected from hydroxy, (1-4C)alkyl, (1-4C)alkoxy, (1-4C)alkylthio, (1-4C)alkylsulphinyl and (1-4C)alkylsulphonyl or which ring may bear a (1-4C)alkylenedioxy substituent, and wherein R³ is (1-6C)alkyl, (2-6C)alkenyl, (2-6C)alkynyl, fluoro-(1-4C)alkyl, cyano-(1-4C)alkyl, hydroxy-(1-4C)alkyl, (1-4C)alkoxy-(1-4C)alkyl or (2-4C)alkanoyloxy(1-4C)alkyl;
or a pharmaceutically-acceptable salt thereof; characterised by
(a) the alkylation, in the presence of a suitable reagent, of a compound of the formula II 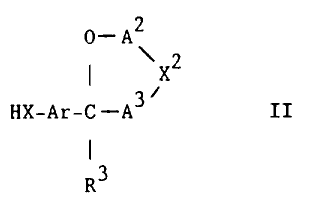 with a compound of the formula Q-A-Z wherein Z is a displaceable group; provided that, when there is an amino, imino, alkylamino, hydroxy or carboxy group in Q, Ar, X or R³, any amino, imino, alkylamino or carboxy group is protected by a conventional protecting group and any hydroxy group may be protected by a conventional protecting group or alternatively any hydroxy group need not be protected;
whereafter any undesired protecting group in Q, Ar, X or R³ is removed by conventional means;
(b) the cyclisation, in the presence of a suitable base, of a compound of the formula VI 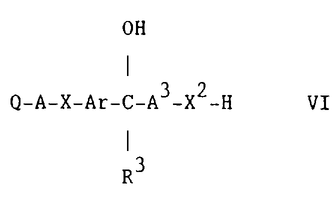 by reaction with a compound of the formula Z-A-Z, wherein A and Z have the meanings defined hereinbefore, provided that, when there is an amino, imino, alkylamino, hydroxy or carboxy group in Q, X, Ar or R³, any amino, imino, alkylamino, hydroxy or carboxy group is protected by a conventional protecting group;
whereafter any undesired protecting group in Q, X, Ar or R³ is removed by conventional means;
(c) the cyclisation, in the presence of a suitable acid, of a compound of the formula VI 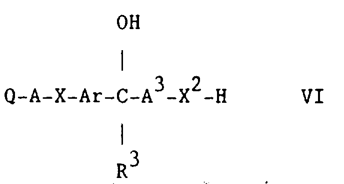 by reaction with an appropriate aldehyde or with an appropriate ketone, or with corresponding hemiacetal or acetal derivatives thereof, provided that, when there is an amino, imino, alkylamino, hydroxy or carboxy group in Q, X, Ar or R³, any imino, amino, alkylamino, hydroxy or carboxy group is protected by a conventional protecting group;
whereafter any undesired protecting group in Q, X, Ar or R³ is removed by conventional means;
(d) for the production of those compounds of the formula I wherein A is a (3-6C)alkynylene group, the coupling, in the presence of a suitable organometallic catalyst, of a heterocyclic compound of the formula Q-Z, wherein Q has the meaning defined hereinbefore and Z is a halogeno group, with an ethynyl compound of the formula VIII 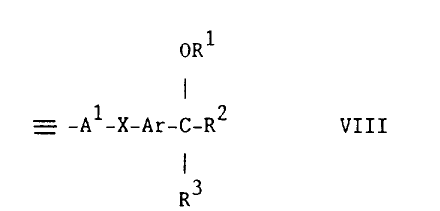 wherein A¹ is (1-4C)alkylene and X, Ar, R¹, R and R³ have the meanings defined hereinbefore;
(e) for the production of those compounds of the formula I wherein Ar bears an alkylsulphinyl or alkylsulphonyl substituent, wherein X is a sulphinyl or sulphonyl group, or wherein R¹ and R together form a group of the formula -A-x-A³-, X is a sulphinyl or sulphonyl group and which group may bear one or two alkylsulphinyl or alkylsulphonyl groups, the oxidation of a compound of the formula I wherein Ar bears an alkylthio substituent, or wherein X is a thio group or wherein R¹ and R together form a group of the formula -A-x-A³-, X is a thio group, and which group may bear one or two alkylthio groups;
(f) for the production of those compounds of the formula I wherein Ar bears an alkanoylamino substituent, the acylation of a compound of the formula I wherein Ar bears an amino substituent;
(g) for the production of those compounds of the formula I wherein A is alkenylene, or wherein R¹ and R together from a group of the formula -A-X-A³- and R³ is alkenyl, the reduction of the corresponding compound wherein A is alkynylene or R³ is alkynyl;
(h) for the production of those compounds of the formula I wherein Q bears an alkyl or substituted alkyl substituent on an available nitrogen atom, or wherein Ar bears an alkoxy or substituted alkoxy substituent, the alkylation of a compound of the formula I wherein Q bears a hydrogen atom on said available nitrogen atom, or wherein Ar bears a hydroxy substituent; or
(i) for the production of those compounds of the formula I wherein Q or Ar bears an amino substituent, the reduction of a compound of the formula I wherein Q or Ar bears a nitro substituent;
and when a pharmaceutically-acceptable salt of a novel compound of the formula I is required, it may be obtained by reaction of said compound with a suitable acid or base using a conventional procedure.

2. A process as claimed in claim 1 for the manufacture of a heterocyclic cyclic ether of the formula I wherein Q is pyridyl, pyrimidinyl, pyrazinyl, quinolyl, isoquinolyl, quinazolinyl or quinoxalinyl which may optionally bear one, two or three substituents selected from fluoro, chloro, hydroxy, oxo, methyl, ethyl, propyl, trifluoromethyl, 2-fluoroethyl, 2-dimethylaminoethyl and benzyl;
where in A is methylene, 1-propenylene or 1-propynylene;
wherein X is oxy;
wherein Ar is 1,3-phenylene or 1,4-phenylene which may optionally bear one or two substituents selected from fluoro, chloro, hydroxy, amino, methoxy and trifluoromethyl, or
Ar is 3,5-pyridylene;
wherein R¹ and R together form a group of the formula -A-X-A³- which, together with the oxygen atom to which A is attached and with the carbon atom to which A³ is attached, defines a ring having 5 or 6 ring atoms, wherein A is methylene, A³ is methylene or ethylene and X is oxy, and which ring may bear one, two or three substituents selected from methyl, ethyl, propyl, isopropyl, butyl, isobutyl and sec-butyl, and R³ is methyl or ethyl;
or a pharmaceutically-acceptable salt thereof.

3. A process as claimed in claim 1 for the manufacture of a heterocyclic cyclic ether of the formula I wherein Q is 2-pyridyl, 3-pyridyl, 3-quinolyl, 5-quinolyl, 6-quinolyl, 7-quinolyl or 6-quinoxalinyl which may optionally bear one or two substituents selected from hydroxy, oxo, methyl, ethyl, propyl, 2-fluoroethyl, 2-dimethylaminoethyl and benzyl;
wherein A is methylene, 1-propenylene or 1-propynylene;
wherein X is oxy;
wherein Ar is 1,3-phenylene or 1,4-phenylene which may optionally bear one or two substituents selected from fluoro, chloro, amino, methoxy and trifluoromethyl, or Ar is 3,5-pyridylene;
wherein R¹ and R together form a group of the formula -A-x-A³- which, together with the oxygen atom to which A is attached and with the carbon atom to which A³ is attached, defines a ring having 5 ring atoms, wherein A is methylene, A³ is methylene and X is oxy, and which ring may bear one, two or three substituents selected from methyl, ethyl, propyl, isopropyl, butyl, isobutyl and sec-butyl, and R³ is methyl or ethyl;
or a pharmaceutically-acceptable salt thereof.

4. A process as claimed in claim 1 for the manufacture of a heterocyclic cyclic ether of the formula I wherein Q is 2-pyridyl, 1,2-dihydro-1-methyl-2-oxoquinolin-3-yl, 2-quinolyl, 3-quinolyl, 1,2-dihydro-2-oxoquinolin-3-yl, 3-isoquinolyl or 6-quinoxalinyl;
A is methylene or 1-propynylene;
Ar is 1,3-phenylene or 5-fluoro-1,3-phenylene; and
wherein R¹ and R together form a group of the formula -A-X-A³- which, together with the oxygen atom to which A is attached and with the carbon atom to which A³ is attached, defines a ring having 5 ring atoms, wherein each of A and A³ is methylene, X is oxy, and which ring may bear one or two methyl substituents, and R³ is ethyl;
or a pharmaceutically-acceptable salt thereof.

5. A process as claimed in claim 1 for the manufacture of a heterocyclic cyclic ether of the formula I wherein Q is 1,2-dihydro-2-oxoquinolin-5-yl, 1,2-dihydro-2-oxoquinolin-6-yl or 1,2-dihydro-2-oxoquinolin-7-yl which bears a 1-substituent selected from methyl, ethyl and 2-fluoroethyl;
wherein A is methylene;
wherein X is oxy;
wherein Ar is 1,3-phenylene which may optionally bear one fluoro substituent;
wherein R¹ and R together form a group of the formula -A-X-A³- which, together with the oxygen atom to which A is attached and with the carbon atom to which A³ is attached defines a ring having 5 ring atoms, wherein A is methylene, A³ is methylene and X is oxy, and which ring may bear one or two substituents selected from methyl, ethyl, propyl and isopropyl, and R³ is methyl or ethyl;
or a pharmaceutically-acceptable salt thereof.

6. A process for the manufacture of a pharmaceutical composition characterised by bringing into association a heterocyclic cyclic ether of the formula I, or a pharmaceutically-acceptable salt thereof, as defined in any one of claims 1 to 5 and a pharmaceutically-acceptable diluent or carrier.

## Patentansprüche (Patentansprüche für folgende(n) Vertragsstaat(en): AT, BE, CH, DE, DK, FR, GB, IT, LI, LU, NL, SE)

1. Hetercyclischer cyclischer Ether mit der folgenden Formel I: 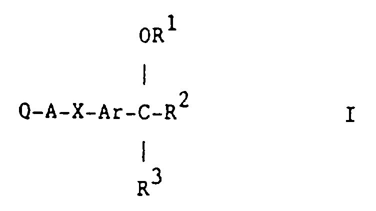 in der
Q für einen 6-gliedrigen monocyclischen oder 10-gliedrigen bicyclischen heterocyclischen Rest steht, der einen oder zwei Stickstoffatome enthält, welcher gegebenenfalls einen, zwei oder drei Substituenten tragen kann, die unter Halogen, Hydroxy, Oxo, Carboxy, Cyano, Amino, (1-4C)Alkyl, (1-4C)Alkoxy, Fluor-(1-4C)alkyl, (1-4C)Alkylamino, Di-[(1-4C)alkyl]amino, Hydroxy-(1-4C)alkyl, Amino-(1-4C)alkyl, (1-4C)Alkylamino-(1-4C)alkyl, Di-[(1-4C)alkyl]amino-(1-4C)alkyl, Amino-(2-4C)alkoxy, (1-4C)Alkylamino-(2-4C)alkoxy, Di-[(1-4C)alkyl]amino-(2-4C)alkoxy und Phenyl-(1-4C)alkyl ausgewählt sind;
A für (1-6C)Alkylen, (3-6C)Alkenylen, (3-6C)Alkinylen oder Cyclo(3-6C)alkylen steht;
X für Oxy, Thio, Sulfinyl, Sulfonyl oder Imino steht;
Ar für Phenylen steht, das gegebenenfalls einen oder zwei Substituenten tragen kann, die unter Halogen, Hydroxy, Amino, Nitro, Cyano, Ureido, Carbamoyl, (1-4C)Alkyl, (3-4C)Alkenyloxy, (1-4C)Alkoxy, (1-4C)Alkylthio, (1-4C)Alkylsulfinyl, (1-4C)Alkylsulfonyl, (1-4C)Alkylamino, Di-[(1-4C)alkyl]amino, Fluor-(1-4C)alkyl, (1-4C)Alkoxycarbonyl, N-[(1-4C)Alkyl]carbamoyl, N,N-Di-[(1-4C)alkyl]carbamoyl, (2-4C)Alkanoylamino, Cyano-(1-4C)alkoxy, Carbamoyl-(1-4C)alkoxy, Amino-(2-4C)alkoxy, (1-4C)Alkylamino-(2-4C)alkoxy, Di-[(1-4C)alkyl]amino-(2-4C)alkoxy und (1-4C)Alkoxycarbonyl-(1-4C)alkoxy ausgewählt sind;
oder in der
Ar für einen 6-gliedrigen Heterocyclen-Rest steht, der bis zu drei Stickstoffatome enthält, der gegebenenfalls einen oder zwei Substituenten tragen kann, die unter Halogen, Hydroxy, Amino, Cyano, (1-4C)Alkyl, (1-4C)Alkoxy, (1-4C)Alkylamino und Di-[(1-4C)alkyl]-amino ausgewählt sind;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 bis 7 Ringatomen definiert, wobei A und A³, die gleich oder unterschiedlich sein können, jeweils für (1-3C)Alkylen stehen und X für Oxy, Thio, Sulfinyl, Sulfonyl oder Imino steht, und wobei der Ring einen, zwei oder drei Substituenten tragen kann, die gleich oder unterschiedlich sein können, welche unter Hydroxy, (1-4C)Alkyl, (1-4C)Alkoxy, (1-4C)Alkylthio, (1-4C)Alkylsulfinyl und (1-4C)Alkylsulfonyl ausgewählt sind, oder wobei der Ring einen (1-4C)Alkylendioxy-Substituenten tragen kann, und
R³ für (1-6C)Alkyl, (2-6C)Alkenyl, (2-6C)Alkinyl, Fluor-(1-4C)alkyl, Cyano-(1-4C)alkyl, Hydroxy-(1-4C)alkyl, (1-4C)Alkoxy-(1-4C)alkyl oder (2-4C)Alkanoyloxy-(1-4C)alkyl steht;
oder ein pharmazeutisch geeignetes Salz davon.

2. Heterocyclischer cyclischer Ether mit der Formel I nach Anspruch 1, in der
Q für Pyridyl, Pyrimidinyl, Pyrazinyl, Chinolyl, Isochinolyl, Chinazolinyl oder Chinoxalinyl steht, das gegebenenfalls einen, zwei oder drei Substituenten tragen kann, die unter Fluor, Chlor, Hydroxy, Oxo, Methyl, Ethyl, Propyl, Trifluormethyl, 2-Fluorethyl, 2-Dimethylaminoethyl und Benzyl ausgewählt sind;
A für Methylen, 1-Propenylen oder 1-Propinylen steht;
X für Oxy steht;
Ar für 1,3-Phenylen oder 1,4-Phenylen steht, das gegebenenfalls einen oder zwei Substituenten tragen kann, die unter Fluor, Chlor, Hydroxy, Amino, Methoxy und Trifluormethyl ausgewählt sind, oder in der Ar für 3,5-Pyridylen steht;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 oder 6 Ringatomen definiert, wobei A für Methylen steht, A³ für Methylen oder Ethylen steht und X für Oxy steht, und wobei der Ring einen, zwei oder drei Substituenten tragen kann, die unter Methyl, Ethyl, Propyl, Isopropyl, Butyl, Isobutyl und sek-Butyl ausgewählt sind, und
R³ für Methyl oder Ethyl steht;
oder ein pharmazeutisch geeignetes Salz davon.

3. Heterocyclischer cyclischer Ether mit der Formel I nach Anspruch 1, in der
Q für 2-Pyridyl, 3-Pyridiyl, 3-Chinolyl, 5-Chinolyl, 6-Chinolyl, 7-Chinolyl oder 6-Chinoxalinyl steht, das gegebenenfalls einen oder zwei Substituenten tragen kann, die unter Hydroxy, Oxo, Methyl, Ethyl, Propyl, 2-Fluorethyl, 2-Dimethylaminoethyl und Benzyl ausgewählt sind;
A für Methylen, 1-Propenylen oder 1-Propinylen steht; X für Oxy steht;
Ar für 1,3-Phenylen oder 1,4-Phenylen steht, das gegebenenfalls einen oder zwei Substituenten tragen kann, die unter Fluor, Chlor, Amino, Methoxy und Trifluormethyl ausgewählt sind, oder in der Ar für 3,5-Pyridylen steht;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 Ringatomen definiert, wobei A für Methylen steht, A³ für Methylen steht und X für Oxy steht, und wobei der Ring einen, zwei oder drei Substituenten tragen kann, die unter Methyl, Ethyl, Propyl, Isopropyl, Butyl, Isobutyl und sek-Butyl ausgewählt sind; und
R³ für Methyl oder Ethyl steht;
oder ein pharmazeutisch geeignet Salz davon.

4. Heterocyclischer cyclischer Ether mit der Formel I nach Anspruch 1, in der
Q für 2-Pyridyl, 1,2-Dihydro-1-methyl-2-oxochinolin-3-yl, 2-Chinolyl, 3-Chinolyl, 1,2-Dihydro-2-oxochinolin-3-yl, 3-Isochinolyl oder 6-Chinoxalinyl steht;
A für Methylen oder 1-Propinylen steht;
Ar für 1,3-Phenylen oder 5-Fluor-1,3-phenylen steht; und
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 Ringatomen definiert, wobei A und A³ jeweils für Methylen stehen und X für Oxy steht, und wobei der Ring einen oder zwei Methyl-Substituenten tragen kann,
und R³ für Ethyl steht;
oder ein pharmazeutisch geeignetes Salz davon.

5. Heterocyclischer cyclischer Ether mit der Formel I nach Anspruch 1, in der
Q für 1,2-Dihydro-2-oxochinolin-5-yl, 1,2-Dihydro-2-oxochinolin-6-yl oder 1,2-Dihydro-2-oxochinolin-7-yl steht, das einen 1-Substituenten trägt, der unter Methyl, Ethyl und 2-Fluorethyl ausgewählt ist;
A für Methylen steht;
X für Oxy steht;
Ar für 1,3-Phenylen steht, das gegebenenfalls einen Fluor-Substituenten tragen kann;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 Ringatomen definiert, wobei A für Methylen steht, A³ für Methylen steht und X für Oxy steht, und wobei der Ring einen oder zwei Substituenten tragen kann, die unter Methyl, Ethyl, Propyl und Isopropyl ausgewählt sind; und
R³ für Methyl oder Ethyl steht;
oder ein pharmazeutisch geeignetes Salz davon.

6. Heterocyclischer cyclischer Ether mit der Formel I nach Anspruch 1, der aus der folgenden Gruppe ausgewählt ist:-
4-Ethyl-2,2-dimethyl-4-[3-(3-(2-pyridyl)prop-2-in-1-yloxy)phenyl]-1,3-dioxolan,
4-Ethyl-4-[5-fluor-3-(1,2-dihydro-1-methyl-2-oxochinolin-6-ylmethoxy)phenyl]-2,2-dimethyl-1,3-dioxolan und
2,4-Diethyl-4-[5-fluor-3-(1,2-dihydro-1-methyl-2-oxochinolin-6-ylmethoxy)phenyl]-1,3-dioxolan
oder ein pharmazeutisch geeignetes Salz davon.

7. Verfahren zur Herstellung eines heterocyclischen cyclischen Ethers mit der Formel I, oder eines pharmazeutisch geeigneten Salzes davon, nach Anspruch 1, bei dem:-
(a) eine Verbindung mit der Formel II 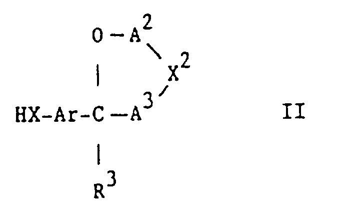 in Gegenwart eines geeigneten Reagenzes mit einer Verbindung mit der Formel Q-A-Z, in der Z für eine austauschbare Gruppe steht, alkyliert wird, mit der Maßgabe, daß jede Amino-, Imino-, Alkylamino- oder Carboxy-Gruppe mit einer üblichen Schutzgruppe geschützt ist, wenn eine Amino-, Imino-, Alkylamino-, Hydroxy- oder Carboxy-Gruppe in Q, Ar, X oder R³ vorhanden ist, und jede Hydroxy-Gruppe mit einer üblichen Schutzgruppe geschützt sein kann, oder alternativ keine Hydroxy-Gruppe geschützt werden muß, woraufhin jede unerwünschte Schutzgruppe in Q, Ar, X oder R³ mit üblichen Mitteln entfernt wird;
(b) eine Verbindung mit der Formel VI 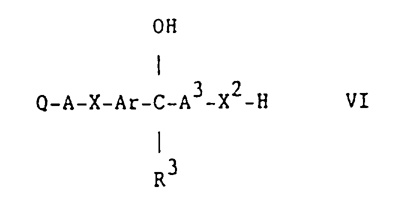 in Gegenwart einer geeigneten Base durch Umsetzung mit einer Verbindung mit der Formel Z-A-Z, in der A und Z die oben definierten Bedeutungen haben, cyclisiert wird, mit der Maßgabe, daß jede Amino-, Imino-, Alkylamino-, Hydroxy- oder Carboxy-Gruppe mit einer üblichen Schutzgruppe geschützt ist, wenn eine Amino-, Imino-, Alkylamino-, Hydroxy- oder Carboxy-Gruppe in Q, X, Ar oder R³ vorhanden ist, woraufhin jede unerwünschte Schutzgruppe in Q, X, Ar oder R³ mit üblichen Mitteln entfernt wird;
(c) eine Verbindung mit der Formel VI 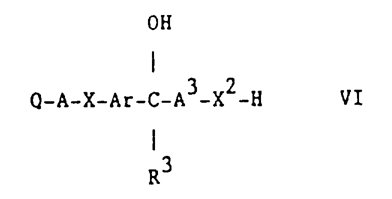 in Gegenwart einer geeigneten Säure durch Umsetzung mit einem geeigneten Aldehyd oder mit einem geeigneten Keton, oder mit entsprechenden Hemiacetal- oder Acetal-Derivaten davon, cyclisiert wird, mit der Maßgabe, daß jede Imino-, Amino-, Alkylamino-, Hydroxy- oder Carboxy-Gruppe mit einer üblichen Schutzgruppe geschützt ist, wenn eine Amino-, Imino-, Alkylamino-, Hydroxy- oder Carboxy-Gruppe in Q, X, Ar oder R³ vorhanden ist, woraufhin jede unerwünschte Schutzgruppe in Q, X, Ar oder R³ mit üblichen Mitteln entfernt wird;
(d) zur Herstellung derjenigen Verbindungen mit der Formel I, bei denen A für eine (3-6C)Alkinylen-Gruppe steht, eine heterocyclische Verbindung mit der Formel Q-Z, in der Q die oben definierte Bedeutung hat und Z für eine Halogen-Gruppe steht, in Gegenwart eines geeigneten organometallischen Katalysators mit einer Ethinyl-Verbindung mit der Formel VIII, 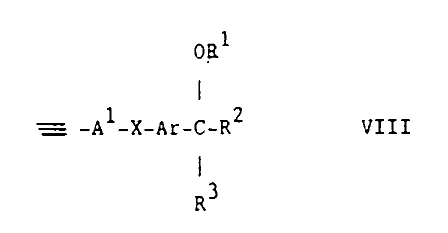 in der A¹ für (1-4C)Alkylen steht und X, Ar, R¹, R und R³ die oben definierten Bedeutungen haben, gekuppelt wird;
(e) zur Herstellung derjenigen Verbindungen mit der Formel I, bei denen Ar einen Alkylsulfinyl- oder Alkylsulfonyl-Substituenten trägt, bei denen X für eine Sulfinyl- oder Sulfonyl-Gruppe steht, oder bei denen R¹ und R zusammen eine Gruppe mit der Formel -A-X-A³- bilden, in der X für eine Sulfinyl- oder Sulfonyl-Gruppe steht, wobei die Gruppe eine oder zwei Alkylsulfinyl- oder Alkylsulfonyl-Gruppen tragen kann, eine Verbindung mit der Formel I, bei der Ar einen Alkylthio-Substituenten trägt, oder bei der X für eine Thio-Gruppe steht, oder bei der R¹ und R zusammen eine Gruppe mit der Formel -A-X-A³- bilden, in der X für eine Thio-Gruppe steht, wobei die Gruppe einen oder zwei Alkylthio-Gruppen tragen kann, oxidiert wird;
(f) zur Herstellung derjenigen Verbindungen mit der Formel I, bei denen Ar einen Alkanoylamino-Substituenten trägt, eine Verbindung mit der Formel I, in der Ar einen Amino-Substituenten trägt, acyliert wird;
(g) zur Herstellung derjenigen Verbindungen der Formel I, bei denen A für Alkenylen steht, oder bei denen R¹ und R zusammen eine Gruppe mit der Formel -A-X-A³- bilden und R³ für Alkenyl steht, die entsprechende Verbindung, bei der A für Alkinylen steht oder R³ für Alkinyl steht, reduziert wird;
(h) zur Herstellung derjenigen Verbindungen mit der Formel I, bei denen Q einen Alkyl- oder substituierten Alkyl-Substituenten an einem verfügbaren Stickstoffatom trägt, oder bei denen Ar einen Alkoxy- oder substituierten Alkoxy-Substituenten trägt, eine Verbindung mit Formel I, bei der Q ein Wasserstoffatom an dem verfügbaren Stickstoffatom trägt, oder bei den Ar einen Hydroxy-Substituenten trägt, alkyliert wird;
(i) zur Herstellung derjenigen Verbindungen mit der Formel I, bei denen Q oder Ar einen Amino-Substituenten trägt, eine Verbindung mit der Formel I, bei der Q oder Ar einen Nitro-Substituenten trägt, reduziert wird;
wenn ein pharmazeutisch geeignetes Salz einer neuen Verbindung mit der Formel I benötigt wird, dieses durch Umsetzung der Verbindung mit einer geeigneten Säure oder Base unter Anwendung eines üblichen Verfahrens erhältlich ist.

8. Pharmazeutische Zusammensetzung, die einen heterocyclischen cyclischen Ether mit der Formel I, oder ein pharmazeutisch geeignetes Salz davon, nach einem der Ansprüche 1 bis 6 in Verbindung mit einem pharmazeutisch geeigneten Streckmittel oder Trägermittel enthält.

9. Heterocyclischer cyclischer Ether mit der Formel I, oder ein pharmazeutisch geeignetes Salz davon, nach einem der Ansprüche 1 bis 6 zu Verwendung in einem Verfahren zur therapeutischen Behandlung des menschlichen oder tierischen Körpers.

10. Verwendung eines heterocyclischen cyclischen Ethers mit der Formel I, oder eines pharmazeutisch geeigneten Salzes davon, nach einem der Ansprüche 1 bis 6 zur Herstellung eines neuen Arzneimittels zur Verwendung gegen durch Leukotriene hervorgerufene Erkrankungen oder medizinische Beschwerden.

## Patentansprüche (Patentansprüche für folgende(n) Vertragsstaat(en): ES, GR)

1. Verfahren zur Herstellung eines hetercyclischen cyclischen Ethers mit der folgenden Formel I: 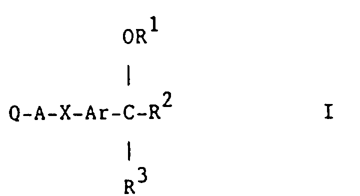 in der
Q für einen 6-gliedrigen monocyclischen oder 10-gliedrigen bicyclischen heterocyclischen Rest steht, der einen oder zwei Stickstoffatome enthält, welcher gegebenenfalls einen, zwei oder drei Substituenten tragen kann, die unter Halogen, Hydroxy, Oxo, Carboxy, Cyano, Amino, (1-4C)Alkyl, (1-4C)Alkoxy, Fluor-(1-4C)alkyl, (1-4C)Alkylamino, Di-[(1-4C)alkyl]amino, Hydroxy-(1-4C)alkyl, Amino-(1-4C)alkyl, (1-4C)Alkylamino-(1-4C)alkyl, Di-[(1-4C)alkyl]amino-(1-4C)alkyl, Amino-(2-4C)alkoxy, (1-4C)Alkylamino-(2-4C)alkoxy, Di-[(1-4C)alkyl]amino-(2-4C)alkoxy und Phenyl-(1-4C)alkyl ausgewählt sind;
A für (1-6C)Alkylen, (3-6C)Alkenylen, (3-6C)Alkinylen oder Cyclo(3-6C)alkylen steht;
X für Oxy, Thio, Sulfinyl, Sulfonyl oder Imino steht;
Ar für Phenylen steht, das gegebenenfalls einen oder zwei Substituenten tragen kann, die unter Halogen, Hydroxy, Amino, Nitro, Cyano, Ureido, Carbamoyl, (1-4C)Alkyl, (3-4C)Alkenyloxy, (1-4C)Alkoxy, (1-4C)Alkylthio, (1-4C)Alkylsulfinyl, (1-4C)Alkylsulfonyl, (1-4C)Alkylamino, Di-[(1-4C)alkyl]amino, Fluor-(1-4C)alkyl, (1-4C)Alkoxycarbonyl, N-[(1-4C)Alkyl]carbamoyl, N,N-Di-[(1-4C)alkyl]carbamoyl, (2-4C)Alkanoylamino, Cyano-(1-4C)alkoxy, Carbamoyl-(1-4C)alkoxy, Amino-(2-4C)alkoxy, (1-4C)Alkylamino-(2-4C)alkoxy, Di-[(1-4C)alkyl]amino-(2-4C)alkoxy und (1-4C)Alkoxycarbonyl-(1-4C)alkoxy ausgewählt sind;
oder in der
Ar für einen 6-gliedrigen Heterocyclen-Rest steht, der bis zu drei Stickstoffatome enthält, der gegebenenfalls einen oder zwei Substituenten tragen kann, die unter Halogen, Hydroxy, Amino, Cyano, (1-4C)Alkyl, (1-4C)Alkoxy, (1-4C)Alkylamino und Di-[(1-4C)alkyl]amino ausgewählt sind;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 bis 7 Ringatomen definiert, wobei A und A³, die gleich oder unterschiedlich sein können, jeweils für (1-3C)Alkylen stehen und X für Oxy, Thio, Sulfinyl, Sulfonyl oder Imino steht, und wobei der Ring einen, zwei oder drei Substituenten tragen kann, die gleich oder unterschiedlich sein können, welche unter Hydroxy, (1-4C)Alkyl, (1-4C)Alkoxy, (1-4C)Alkylthio, (1-4C)Alkylsulfinyl und (1-4C)Alkylsulfonyl ausgewählt sind, oder wobei der Ring einen (1-4C)Alkylendioxy-Substituenten tragen kann, und
R³ für (1-6C)Alkyl, (2-6C)Alkenyl, (2-6C)Alkinyl, Fluor-(1-4C)alkyl, Cyano-(1-4C)alkyl, Hydroxy-(1-4C)alkyl, (1-4C)Alkoxy-(1-4C)alkyl oder (2-4C)Alkanoyloxy-(1-4C)alkyl steht;
oder eines pharmazeutisch geeigneten Salzes davon,
dadurch **gekennzeichnet,** daß
(a) eine Verbindung mit der Formel II 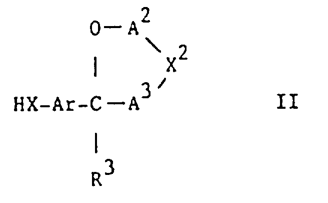 in Gegenwart eines geeigneten Reagenzes mit einer Verbindung mit der Formel Q-A-Z, in der Z für eine austauschbare Gruppe steht, alkyliert wird, mit der Maßgabe, daß jede Amino-, Imino-, Alkylamino- oder Carboxy-Gruppe mit einer üblichen Schutzgruppe geschützt ist, wenn eine Amino-, Imino-, Alkylamino-, Hydroxy- oder Carboxy-Gruppe in Q, Ar, X oder R³ vorhanden ist, und jede Hydroxy-Gruppe mit einer üblichen Schutzgruppe geschützt sein kann, oder alternativ keine Hydroxy-Gruppe geschützt werden muß, woraufhin jede unerwünschte Schutzgruppe in Q, Ar, X oder R³ mit üblichen Mitteln entfernt wird;
(b) eine Verbindung mit der Formel VI 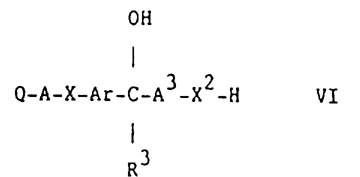 in Gegenwart einer geeigneten Base durch Umsetzung mit einer Verbindung mit der Formel Z-A-Z, in der A und Z die oben definierten Bedeutungen haben, cyclisiert wird, mit der Maßgabe, daß jede Amino-, Imino-, Alkylamino-, Hydroxy- oder Carboxy-Gruppe mit einer üblichen Schutzgruppe geschützt ist, wenn eine Amino-, Imino-, Alkylamino-, Hydroxy- oder Carboxy-Gruppe in Q, X, Ar oder R³ vorhanden ist, woraufhin jede unerwünschte Schutzgruppe in Q, X, Ar oder R³ mit üblichen Mitteln entfernt wird;
(c) eine Verbindung mit der Formel VI 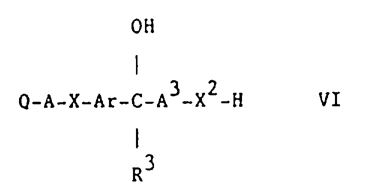 in Gegenwart einer geeigneten Säure durch Umsetzung mit einem geeigneten Aldehyd oder mit einem geeigneten Keton, oder mit entsprechenden Hemiacetal- oder Acetal-Derivaten davon, cyclisiert wird, mit der Maßgabe, daß jede Imino-, Amino-, Alkylamino-, Hydroxy- oder Carboxy-Gruppe mit einer üblichen Schutzgruppe geschützt ist, wenn eine Amino-, Imino-, Alkylamino-, Hydroxy- oder Carboxy-Gruppe in Q, X, Ar oder R³ vorhanden ist, woraufhin jede unerwünschte Schutzgruppe in Q, X, Ar oder R³ mit üblichen Mitteln entfernt wird;
(d) zur Herstellung derjenigen Verbindungen mit der Formel I, bei denen A für eine (3-6C)Alkinylen-Gruppe steht, eine heterocyclische Verbindung mit der Formel Q-Z, in der Q die oben definierte Bedeutung hat und Z für eine Halogen-Gruppe steht, in Gegenwart eines geeigneten organometallischen Katalysators mit einer Ethinyl-Verbindung mit der Formel VIII, 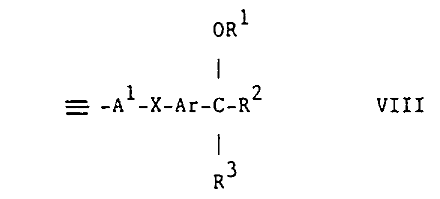 in der A¹ für (1-4C)Alkylen steht und X, Ar, R¹, R und R³ die oben definierten Bedeutungen haben, gekuppelt wird;
(e) zur Herstellung derjenigen Verbindungen mit der Formel I, bei denen Ar einen Alkylsulfinyl- oder Alkylsulfonyl-Substituenten trägt, bei denen X für eine Sulfinyl- oder Sulfonyl-Gruppe steht, oder bei denen R¹ und R zusammen eine Gruppe mit der Formel -A-X-A³- bilden, in der X für eine Sulfinyl- oder Sulfonyl-Gruppe steht, wobei die Gruppe eine oder zwei Alkylsulfinyl- oder Alkylsulfonyl-Gruppen tragen kann, eine Verbindung mit der Formel I, bei der Ar einen Alkylthio-Substituenten trägt, oder bei der X für eine Thio-Gruppe steht, oder bei der R¹ und R zusammen eine Gruppe mit der Formel -A-X-A³- bilden, in der X für eine Thio-Gruppe steht, wobei die Gruppe einen oder zwei Alkylthio-Gruppen tragen kann, oxidiert wird;
(f) zur Herstellung derjenigen Verbindungen mit der Formel I, bei denen Ar einen Alkanoylamino-Substituenten trägt, eine Verbindung mit der Formel I, in der Ar einen Amino-Substituenten trägt, acyliert wird;
(g) zur Herstellung derjenigen Verbindungen der Formel I, bei denen A für Alkenylen steht, oder bei denen R¹ und R zusammen eine Gruppe mit der Formel -A-X-A³- bilden und R³ für Alkenyl steht, die entsprechende Verbindung, bei der A für Alkinylen steht oder R³ für Alkinyl steht, reduziert wird;
(h) zur Herstellung derjenigen Verbindungen mit der Formel I, bei denen Q einen Alkyl- oder substituierten Alkyl-Substituenten an einem verfügbaren Stickstoffatom trägt, oder bei denen Ar einen Alkoxy- oder substituierten Alkoxy-Substituenten trägt, eine Verbindung mit Formel I, bei der Q ein Wasserstoffatom an dem verfügbaren Stickstoffatom trägt, oder bei den Ar einen Hydroxy-Substituenten trägt, alkyliert wird;
(i) zur Herstellung derjenigen Verbindungen mit der Formel I, bei denen Q oder Ar einen Amino-Substituenten trägt, eine Verbindung mit der Formel I, bei der Q oder Ar einen Nitro-Substituenten trägt, reduziert wird; wenn ein pharmazeutisch geeignetes Salz einer neuen Verbindung mit der Formel I benötigt wird, dieses durch Umsetzung der Verbindung mit einer geeigneten Säure oder Base unter Anwendung eines üblichen Verfahrens erhältlich ist.

2. Verfahren nach Anspruch 1 zur Herstellung eines heterocyclischen cyclischen Ethers mit der Formel I, in der
Q für Pyridyl, Pyrimidinyl, Pyrazinyl, Chinolyl, Isochinolyl, Chinazolinyl oder Chinoxalinyl steht, das gegebenenfalls einen, zwei oder drei Substituenten tragen kann, die unter Fluor, Chlor, Hydroxy, Oxo, Methyl, Ethyl, Propyl, Trifluormethyl, 2-Fluorethyl, 2-Dimethylaminoethyl und Benzyl ausgewählt sind;
A für Methylen, 1-Propenylen oder 1-Propinylen steht; X für Oxy steht;
Ar für 1,3-Phenylen oder 1,4-Phenylen steht, das gegebenenfalls einen oder zwei Substituenten tragen kann, die unter Fluor, Chlor, Hydroxy, Amino, Methoxy und Trifluormethyl ausgewählt sind, oder in der Ar für 3,5-Pyridylen steht;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 oder 6 Ringatomen definiert, wobei A für Methylen steht, A³ für Methylen oder Ethylen steht und X für Oxy steht, und wobei der Ring einen, zwei oder drei Substituenten tragen kann, die unter Methyl, Ethyl, Propyl, Isopropyl, Butyl, Isobutyl und sek-Butyl ausgewählt sind, und
R³ für Methyl oder Ethyl steht;
oder eines pharmazeutisch geeigneten Salzes davon.

3. Verfahren nach Anspruch 1 zur Herstellung eines heterocyclischen cyclischen Ethers mit der Formel I, in der
Q für 2-Pyridyl, 3-Pyridiyl, 3-Chinolyl, 5-Chinolyl, 6-Chinolyl, 7-Chinolyl oder 6-Chinoxalinyl steht, das gegebenenfalls einen oder zwei Substituenten tragen kann, die unter Hydroxy, Oxo, Methyl, Ethyl, Propyl, 2-Fluorethyl, 2-Dimethylaminoethyl und Benzyl ausgewählt sind;
A für Methylen, 1-Propenylen oder 1-Propinylen steht; X für Oxy steht;
Ar für 1,3-Phenylen oder 1,4-Phenylen steht, das gegebenenfalls einen oder zwei Substituenten tragen kann, die unter Fluor, Chlor, Amino, Methoxy und Trifluormethyl ausgewählt sind, oder in der Ar für 3,5-Pyridylen steht;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 Ringatomen definiert, wobei A für Methylen steht, A³ für Methylen steht und X für Oxy steht, und wobei der Ring einen, zwei oder drei Substituenten tragen kann, die unter Methyl, Ethyl, Propyl, Isopropyl, Butyl, Isobutyl und sek-Butyl ausgewählt sind; und
R³ für Methyl oder Ethyl steht;
oder eines pharmazeutisch geeigneten Salzes davon.

4. Verfahren nach Anspruch 1 zur Herstellung eines heterocyclischen cyclischen Ethers mit der Formel I, in der
Q für 2-Pyridyl, 1,2-Dihydro-1-methyl-2-oxochinolin-3-yl, 2-Chinolyl, 3-Chinolyl, 1,2-Dihydro-2-oxochinolin-3-yl, 3-Isochinolyl oder 6-Chinoxalinyl steht;
A für Methylen oder 1-Propinylen steht;
Ar für 1,3-Phenylen oder 5-Fluor-1,3-phenylen steht; und
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 Ringatomen definiert, wobei A und A³ jeweils für Methylen stehen und X für Oxy steht, und wobei der Ring einen oder zwei Methyl-Substituenten tragen kann,
und R³ für Ethyl steht;
oder eines pharmazeutisch geeigneten Salzes davon.

5. Verfahren nach Anspruch 1 zur Herstellung eines heterocyclischen cyclischen Ethers mit der Formel I, in der
Q für 1,2-Dihydro-2-oxochinolin-5-yl, 1,2-Dihydro-2-oxochinolin-6-yl oder 1,2-Dihydro-2-oxochinolin-7-yl steht, das einen 1-Substituenten trägt, der unter Methyl, Ethyl und 2-Fluorethyl ausgewählt ist;
A für Methylen steht;
X für Oxy steht;
Ar für 1,3-Phenylen steht, das gegebenenfalls einen Fluor-Substituenten tragen kann;
R¹ und R zusammen eine Gruppe mit der Formel -A-X-A³- bilden, die zusammen mit dem Sauerstoffatom, an das A gebunden ist, und mit dem Kohlenstoffatom, an das A³ gebunden ist, einen Ring mit 5 Ringatomen definiert, wobei A für Methylen steht, A³ für Methylen steht und X für Oxy steht, und wobei der Ring einen oder zwei Substituenten tragen kann, die unter Methyl, Ethyl, Propyl und Isopropyl ausgewählt sind; und
R³ für Methyl oder Ethyl steht;
oder eines pharmazeutisch geeigneten Salzes davon.

6. Verfahren zur Herstellung einer pharmazeutischen Zusammensetzung, dadurch **gekennzeichnet,** daß ein heterocyclischer cyclischer Ether mit der Formel I, oder ein pharmazeutisch geeignetes Salz davon, wie in einem der Ansprüche 1 bis 5 definiert, und ein pharmazeutisch geeignetes Streckmittel oder Trägermittel miteinander in Verbindung gebracht werden.

## Revendications (Revendications pour l'(les) Etat(s) contractant(s) suivant(s): AT, BE, CH, DE, DK, FR, GB, IT, LI, LU, NL, SE)

1. Ether cyclique hétérocyclique de formule I 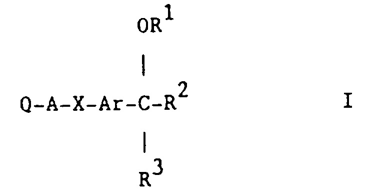 dans laquelle Q représente un groupement hétérocyclique monocyclique hexagonal ou bicyclique décagonal contenant un ou deux atomes d'azote qui peuvent porter facultativement un, deux ou trois substituants choisis entre des substituants halogéno, hydroxy, oxo, carboxy, cyano, amino, alkyle en C₁ à C₄, alkoxy en C₁ à C₄, fluoralkyle en C₁ à C₄, alkylamino en C₁ à C₄, di(alkyle en C₁ à C₄)-amino, hydroxyalkyle en C₁ à C₄, aminoalkyle en C₁ à C₄, (alkyle en C₁ à C₄)-amino-(alkyle en C₁ à C₄), di(alkyle en C₁ à C₄)-amino-(alkyle en C₁ à C₄), aminoalkoxy en C₂ à C₄, (alkyle en C₁ à C₄)-amino-(alkoxy en C₂ à C₄), di(alkyle en C₁ à C₄)-amino-(alkoxy en C₂ à C₄) et phényl-(alkyle en C₁ à C₄) ;
A représente un groupe alkylène en C₁ à C₆, alcénylène en C₃ à C₆, alcynylène en C₃ à C₆ ou cycloalkylène en C₃ à C₆ ;
X représente un groupe oxy, thio, sulfinyle, sulfonyle ou imino ;
Ar représente un groupe phénylène qui peut porter facultativement un ou deux substituants choisis entre des substituants halogéno, hydroxy, amino, nitro, cyano, uréido, carbamoyle, alkyle en C₁ à C₄, alcényloxy en C₃ ou C₄, alkoxy en C₁ à C₄, alkylthio en C₁ à C₄, alkylsulfinyle en C₁ à C₄, alkylsulfonyle en C₁ à C₄, alkylamino en C₁ à C₄, di(alkyle en C₁ à C₄)-amino, fluoralkyle en C₁ à C₄, (alkoxy en C₁ à C₄)-carbonyle, N-(alkyle en C₁ à C₄)-carbamoyle, N,N-di(alkyle en C₁ à C₄)-carbamoyle, alcanoylamino en C₂ à C₄, cyano-(alkoxy en C₁ à C₄), carbamoyl-(alkoxy en C₁ à C₄), amino-(alkoxy en C₂ à C₄), (alkyle en C₁ à C₄)-amino-(alkoxy en C₂ à C₄), di(alkyle en C₁ à C₄)-amino-(alkoxy en C₂ à C₄) et (alkoxy en C₁ à C₄)-carbonyl-(alkoxy en C₁ à C₄); ou
Ar représente un groupement hétérocyclène hexagonal contenant jusqu'à trois atomes d'azote pouvant porter facultativement un ou deux substituants choisis entre des substituants halogéno, hydroxy, amino, cyano, alkyle en C₁ à C₄, alkoxy en C₁ à C₄, alkylamino en C₁ à C₄ et di(alkyle en C₁ à C₄)amino ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 à 7 atomes dans le cycle, dans lequel A et A³, qui peuvent être identiques ou différents, représentent chacun un groupe alkylène en C₁ à C₃ et X représente un groupe oxy, thio, sulfinyle, sulfonyle ou imino, noyau qui peut porter un, deux ou trois substituants, qui peuvent être identiques ou différents, choisis entre des substituants hydroxy, alkyle en C₁ à C₄, alkoxy en C₁ à C₄, alkylthio en C₁ à C₄, alkylsulfinyle en C₁ à C₄ et alkylsulfonyle en C₁ à C₄, ou noyau qui peut porter un substituant alkylènedioxy en C₁ à C₄, et dans laquelle R³ représente un groupe alkyle en C₁ à C₆, alcényle en C₂ à C₆, alcynyle en C₂ à C₆, fluoralkyle en C₁ à C₄, cyano-(alkyle en C₁ à C₄), hydroxyalkyle en C₁ à C₄, (alkoxy en C₁ à C₄)-(alkyle en C₁ à C₄) ou (alcanoyle en C₂ à C₄)-oxy-(alkyle en C₁ à C₄) ;
ou un de ses sels pharmaceutiquement acceptables.

2. Ether cyclique hétérocyclique de formule I suivant la revendication 1, dans lequel Q représente un groupe pyridyle, pyrimidinyle, pyrazinyle, quinolyle, isoquinolyle, quinazolinyle ou quinoxalinyle qui peut porter facultativement un, deux ou trois substituants choisis entre les substituants fluoro, chloro, hydroxy, oxo, méthyle, éthyle, propyle, trifluorométhyle, 2-fluoréthyle, 2-diméthylaminoéthyle et benzyle ;
A représente un groupe méthylène, 1-propénylène ou 1-propynylène ;
X représente un groupe oxy ;
Ar représente un groupe 1,3-phénylène ou 1,4-phénylène qui peut porter facultativement un ou deux substituants choisis entre les substituants fluoro, chloro, hydroxy, amino, méthoxy et trifluorométhyle, ou
Ar représente un groupe 3,5-pyridylène ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 ou 6 atomes dans le cycle, dans lequel A représente un groupe méthylène, A³ représente un groupe méthylène ou éthylène et X représente un groupe oxy, noyau qui peut porter un, deux ou trois substituants choisis entre les substituants méthyle, éthyle, propyle, isopropyle, butyle, isobutyle et sec.-butyle, et R³ représente un groupe méthyle ou éthyle ;
ou un de ses sels pharmaceutiquement acceptables.

3. Ether cyclique hétérocyclique de formule I suivant la revendication 1, dans lequel Q représente un groupe 2-pyridyle, 3-pyridyle, 3-quinolyle, 5-quinolyle, 6-quinolyle, 7-quinolyle ou 6-quinoxalinyle qui peut porter facultativement un ou deux substituants choisis entre les substituants hydroxy, oxo, méthyle, éthyle, propyle, 2-fluoréthyle, 2-diméthylaminoéthyle et benzyle ;
A représente un groupe méthylène, 1-propénylène ou 1-propynylène ;
X représente un groupe oxy ;
Ar représente un groupe 1,3-phénylène ou 1,4-phénylène qui peut porter facultativement un ou deux substituants choisis entre les substituants fluoro, chloro, amino, méthoxy et trifluorométhyle, ou Ar représente un groupe 3,5-pyridylène ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 atomes dans le cycle, dans lequel A représente un groupe méthylène, A³ représente un groupe méthylène et X représente un groupe oxy, noyau qui peut porter un, deux ou trois substituants choisis entre les substituants méthyle, éthyle, propyle, isopropyle, butyle, isobutyle et sec.-butyle, et R³ représente un groupe méthyle ou éthyle ;
ou un de ses sels pharmaceutiquement acceptables.

4. Ether cyclique hétérocyclique de formule I suivant la revendication 1, dans lequel Q représente un groupe 2-pyridyle, 1,2-dihydro-1-méthyl-2-oxoquinoline-3-yle, 2-quinolyle, 3-quinolyle, 1,2-dihydro-2-oxoquinoline-3-yle, 3-isoquinolyle ou 6-quinoxalinyle ;
A représente un groupe méthylène ou 1-propynylène ;
Ar représente un groupe 1,3-phénylène ou 5-fluoro-1,3-phénylène ; et
R¹ et R forment conjointement un groupe de formule -A-X-A³- qui, conjointement avec l'atome d'oxygène auquel A est fixé et avec l'atome de carbone auquel A³ est fixé, définit un noyau ayant 5 atomes dans le cycle, dans lequel chacun des groupes A et A³ représente un groupe méthylène, X représente un groupe oxy, noyau qui peut porter un ou deux substituants méthyle, et R³ représente un groupe éthyle ;
ou un de ses sels pharmaceutiquement acceptables.

5. Ether cyclique hétérocyclique de formule I suivant la revendication 1, dans lequel Q représente un groupe 1,2-dihydro-2-oxoquinoline-5-yle, 1,2-dihydro-2-oxoquinoline-6-yle ou 1,2-dihydro-2-oxoquinoline-7-yle qui porte un substituant en position 1 choisi entre les substituants méthyle, éthyle et 2-fluoréthyle ;
A représente un groupe méthylène ;
X représente un groupe oxy ;
Ar représente un groupe 1,3-phénylène qui peut porter facultativement un substituant fluoro ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 atomes dans le cycle, dans lequel A représente un groupe méthylène, A³ représente un groupe méthylène et X représente un groupe oxy, noyau qui peut porter un ou deux substituants choisis entre les substituants méthyle, éthyle, propyle et isopropyle, et R³ représente un groupe méthyle ou éthyle ;
ou un de ses sels pharmaceutiquement acceptables.

6. Ether cyclique hétérocyclique de formule I suivant la revendication 1, choisi dans le groupe consistant en :
le 4-éthyl-2,2-diméthyl-4-[3-(3-(2-pyridyl)prop-2-yne-1-yloxy)phényl]-1,3-dioxolanne,
le 4-éthyl-4-[5-fluoro-3-(1,2-dihydro-1-méthyl-2-oxoquinoline-6-ylméthoxy)phényl]-2,2-diméthyl-1,3-dioxolanne et
le 2,4-diéthyl-4-[5-fluoro-3-(1,2-dihydro-1-méthyl-2-oxoquinoline-6-ylméthoxy)phényl]-1,3-dioxolanne;
ou un de ses sels pharmaceutiquement acceptables.

7. Procédé pour la production d'un éther cyclique hétérocyclique de formule I, ou d'un de ses sels pharmaceutiquement acceptables, suivant la revendication 1, qui comprend :
(a) l'alkylation, en présence d'un réactif convenable, d'un composé de formule II 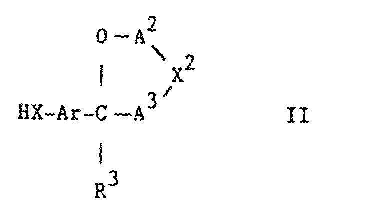 avec un composé de formule Q-A-Z dans laquelle Z représente un groupe déplaçable ; sous réserve que, lorsqu'il existe un groupe amino, imino, alkylamino, hydroxy ou carboxy dans Q, Ar, X ou R³, n'importe quel groupe amino, imino, alkylamino ou carboxy soit protégé par un groupe protecteur classique et n'importe quel groupe hydroxy puisse être protégé par un groupe protecteur classique ou, en variante, un quelconque tel groupe hydroxy ne nécessite pas une protection ;
tout groupe protecteur non désiré dans Q, Ar, X ou R³ étant ensuite éliminé par un moyen classique ;
(b) la cyclisation, en présence d'une base convenable, d'un composé de formule VI 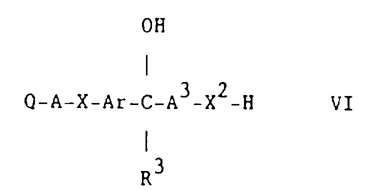 par réaction avec un composé de formule Z-A-Z, dans laquelle A et Z répondent aux définitions précitées, sous réserve que, lorsqu'il existe un groupe amino, imino, alkylamino, hydroxy ou carboxy dans Q, X, Ar ou R³, tout groupe amino, imino, alkylamino, hydroxy ou carboxy soit protégé par un groupe protecteur classique ;
tout groupe protecteur non désiré dans Q, X, Ar R³ étant ensuite éliminé par un moyen classique ;
(c) la cyclisation, en présence d'un acide convenable, d'un composé de formule VI 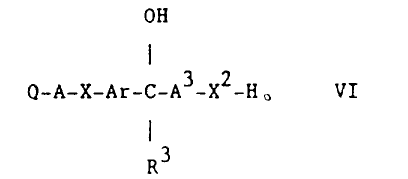 par réaction avec un aldéhyde approprié ou avec une cétone appropriée, ou avec un de leurs dérivés hémiacétaliques ou acétaliques correspondants, sous réserve que, lorsqu'il existe un groupe amino, imino, alkylamino, hydroxy ou carboxy dans Q, X, Ar ou R³, n'importe quel groupe imino, amino, alkylamino, hydroxy ou carboxy soit protégé par un groupe protecteur classique ; tout groupe protecteur non désiré dans Q, X, Ar ou R³ étant ensuite éliminé par un moyen classique ;
(d) pour la production des composés de formule I dans laquelle A représente un groupe alcynylène en C₃ à C₆, le couplage, en présence d'un catalyseur organométallique convenable, d'un composé hétérocyclique de formule Q-Z, dans laquelle Q répond à la définition précitée et Z représente un groupe halogéno, avec un composé à fonction éthynyle de formule VIII 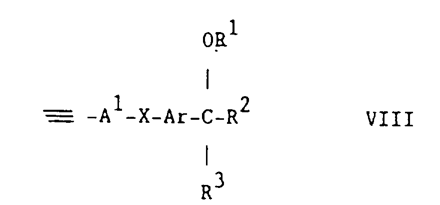 dans laquelle A¹ représente un groupe alkylène en C₁ à C₄ et X, Ar, R¹, R et R³ répondent aux définitions précitées ;
(e) pour la production des composés de formule I dans laquelle Ar porte un substituant alkylsulfinyle ou alkylsulfonyle, dans laquelle X représente un groupe sulfinyle ou sulfonyle, ou dans laquelle R¹ et R forment conjointement un groupe de formule -A-X-A³-, X représente un groupe sulfinyle ou sulfonyle, groupe qui peut porter un ou deux groupes alkylsulfinyle ou alkylsulfonyle, l'oxydation d'un composé de formule I dans laquelle Ar porte un substituant alkylthio, dans laquelle X représente un groupe thio ou dans laquelle R¹ et R forment conjointement un groupe de formule -A-X-A³-, X représente un groupe thio, groupe qui peut porter un ou deux groupes alkylthio ;
(f) pour la production des composés de formule I dans laquelle Ar porte un substituant alcanoylamino, l'acylation d'un composé de formule I dans laquelle Ar porte un substituant amino.
(g) pour la production des composés de formule I dans laquelle A représente un groupe alcénylène, ou dans laquelle R¹ et R forment conjointement un groupe de formule -A-X-A³- et R³ représente un groupe alcényle, la réduction du composé correspondant dans lequel A représente un groupe alcynylène ou R³ représente un groupe alcynyle ;
(h) pour la production des composés de formule I dans laquelle Q porte un substituant alkyle ou alkyle substitué sur un atome d'azote disponible, ou dans laquelle Ar porte un substituant alkoxy ou alkoxy substitué, l'alkylation d'un composé de formule I dans laquelle Q porte un atome d'hydrogène sur ledit atome d'azote disponible, ou dans laquelle Ar porte un substituant hydroxy ; ou
(i) pour la production des composés de formule I dans laquelle Q ou Ar porte un substituant amino, la réduction d'un composé de formule I dans laquelle Q ou Ar porte un substituant nitro ;
et, lorsqu'un sel pharmaceutiquement acceptable d'un composé nouveau de formule I est requis, l'obtention de ce sel par réaction dudit composé avec un acide ou une base convenable au moyen d'un mode opératoire classique.

8. Composition pharmaceutique qui comprend un éther cyclique hétérocyclique de formule I, ou un de ses sels pharmaceutiquement acceptables, suivant l'une quelconque des revendications 1 à 6 en association avec un diluant ou support pharmaceutiquement acceptable.

9. Ether cyclique hétérocyclique de formule I, ou un de ses sels pharmaceutiquement acceptables suivant l'une quelconque des revendications 1 à 6, destiné à être utilisé dans une méthode de traitement de l'organisme de l'homme ou d'un animal par thérapeutique.

10. Utilisation d'un éther cyclique hétérocyclique de formule I, ou d'un de ses sels pharmaceutiquement acceptables, suivant l'une quelconque des revendications 1 à 6 dans la production d'un médicament nouveau destiné à être utilisé dans une maladie ou un état pathologique à médiation par des leucotriènes.

## Revendications (Revendications pour l'(les) Etat(s) contractant(s) suivant(s): ES, GR)

1. Procédé de production d'un éther cyclique hétérocyclique de formule I 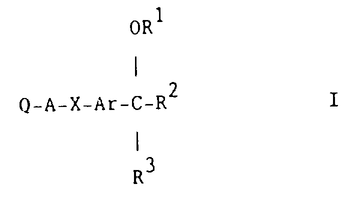 dans laquelle Q représente un groupement hétérocyclique monocyclique hexagonal ou bicyclique décagonal contenant un ou deux atomes d'azote qui peuvent porter facultativement un, deux ou trois substituants choisis entre des substituants halogéno, hydroxy, oxo, carboxy, cyano, amino, alkyle en C₁ à C₄, alkoxy en C₁ à C₄, fluoralkyle en C₁ à C₄, alkylamino en C₁ à C₄, di(alkyle en C₁ à C₄)-amino, hydroxyalkyle en C₁ à C₄, aminoalkyle en C₁ à C₄, (alkyle en C₁ à C₄)-amino-(alkyle en C₁ à C₄), di(alkyle en C₁ à C₄)-amino-(alkyle en C₁ à C₄), aminoalkoxy en C₂ à C₄, (alkyle en C₁ à C₄)-amino-(alkoxy en C₂ à C₄), di(alkyle en C₁ à C₄)-amino-(alkoxy en C₂ à C₄) et phényl-(alkyle en C₁ à C₄) ;
A représente un groupe alkylène en C₁ à C₆, alcénylène en C₃ à C₆, alcynylène en C₃ à C₆ ou cycloalkylène en C₃ à C₆ ;
X représente un groupe oxy, thio, sulfinyle, sulfonyle ou imino ;
Ar représente un groupe phénylène qui peut porter facultativement un ou deux substituants choisis entre des substituants halogéno, hydroxy, amino, nitro, cyano, uréido, carbamoyle, alkyle en C₁ à C₄, alcényloxy en C₃ ou C₄, alkoxy en C₁ à C₄, alkylthio en C₁ à C₄, alkylsulfinyle en C₁ à C₄, alkylsulfonyle en C₁ à C₄, alkylamino en C₁ à C₄, di(alkyle en C₁ à C₄)-amino, fluoralkyle en C₁ à C₄, (alkoxy en C₁ à C₄)-carbonyle, N-(alkyle en C₁ à C₄)-carbamoyle, N,N-di(alkyle en C₁ à C₄)-carbamoyle, alcanoylamino en C₂ à C₄, cyano-(alkoxy en C₁ à C₄), carbamoyl-(alkoxy en C₁ à C₄), amino-(alkoxy en C₂ à C₄), (alkyle en C₁ à C₄)-amino-(alkoxy en C₂ à C₄), di(alkyle en C₁ à C₄)-amino-(alkoxy en C₂ à C₄) et (alkoxy en C₁ à C₄)-carbonyl-(alkoxy en C₁ à C₄) ; ou
Ar représente un groupement hétérocyclène hexagonal contenant jusqu'à trois atomes d'azote pouvant porter facultativement un ou deux substituants choisis entre des substituants halogéno, hydroxy, amino, cyano, alkyle en C₁ à C₄, alkoxy en C₁ à C₄, alkylamino en C₁ à C₄ et di(alkyle en C₁ à C₄)amino ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 à 7 atomes dans le cycle, dans lequel A et A³, qui peuvent être identiques ou différents, représentent chacun un groupe alkylène en C₁ à C₃ et X représente un groupe oxy, thio, sulfinyle, sulfonyle ou imino, noyau qui peut porter un, deux ou trois substituants, qui peuvent être identiques ou différents, choisis entre des substituants hydroxy, alkyle en C₁ à C₄, alkoxy en C₁ à C₄, alkylthio en C₁ à C₄, alkylsulfinyle en C₁ à C₄ et alkylsulfonyle en C₁ à C₄, ou noyau qui peut porter un substituant alkylènedioxy en C₁ à C₄, et dans laquelle R³ représente un groupe alkyle en C₁ à C₆, alcényle en C₂ à C₆, alcynyle en C₂ à C₆, fluoralkyle en C₁ à C₄, cyano-(alkyle en C₁ à C₄), hydroxyalkyle en C₁ à C₄, (alkoxy en C₁ à C₄)-(alkyle en C₁ à C₄) ou (alcanoyle en C₂ à C₄)-oxy-(alkyle en C₁ à C₄) ;
ou d'un de ses sels pharmaceutiquement acceptables ; caractérisé par
(a) l'alkylation, en présence d'un réactif convenable, d'un composé de formule II 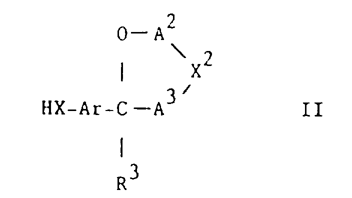 avec un composé de formule Q-A-Z dans laquelle Z représente un groupe déplaçable ; sous réserve que, lorsqu'il existe un groupe amino, imino, alkylamino, hydroxy ou carboxy dans Q, Ar, X ou R³, n'importe quel groupe amino, imino, alkylamino 5 ou carboxy soit protégé par un groupe protecteur classique et n'importe quel groupe hydroxy puisse être protégé par un groupe protecteur classique ou, en variante, un quelconque tel groupe hydroxy ne nécessite pas une protection ;
tout groupe protecteur non désiré dans Q, Ar, X ou R³ étant ensuite éliminé par un moyen classique ;
(b) la cyclisation, en présence d'une base convenable, d'un composé de formule VI 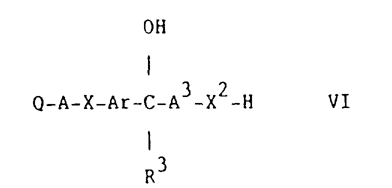 par réaction avec un composé de formule Z-A-Z, dans laquelle A et Z répondent aux définitions précitées, sous réserve que, lorsqu'il existe un groupe amino, imino, alkylamino, hydroxy ou carboxy dans Q, X, Ar ou R³, tout groupe amino, imino, alkylamino, hydroxy ou carboxy soit protégé par un groupe protecteur classique ;
tout groupe protecteur non désiré dans Q, X, Ar ou R³ étant ensuite éliminé par un moyen classique ;
(c) la cyclisation, en présence d'un acide convenable, d'un composé de formule VI 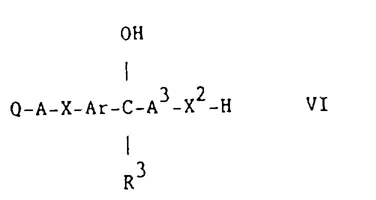 par réaction avec un aldéhyde approprié ou avec une cétone appropriée, ou avec un de leurs dérivés hémiacétaliques ou acétaliques correspondants, sous réserve que, lorsqu'il existe un groupe amino, imino, alkylamino, hydroxy ou carboxy dans Q, X, Ar ou R³, n'importe quel groupe imino, amino, alkylamino, hydroxy ou carboxy soit protégé par un groupe protecteur classique ; tout groupe protecteur non désiré dans Q, X, Ar ou R³ étant ensuite éliminé par un moyen classique ;
(d) pour la production des composés de formule I dans laquelle A représente un groupe alcynylène en C₃ à C₆, le couplage, en présence d'un catalyseur organométallique convenable, d'un composé hétérocyclique de formule Q-Z, dans laquelle Q répond à la définition précitée et Z représente un groupe halogéno, avec un composé à fonction éthynyle de formule VIII 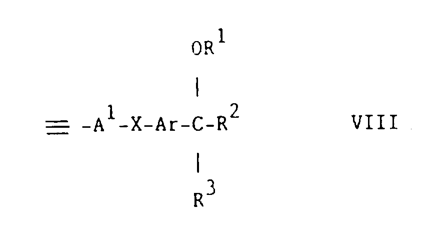 dans laquelle A¹ représente un groupe alkylène en C₁ à C₄ et X, Ar, R¹, R et R³ répondent aux définitions précitées ;
(e) pour la production des composés de formule I dans laquelle Ar porte un substituant alkylsulfinyle ou alkylsulfonyle, dans laquelle X représente un groupe sulfinyle ou sulfonyle, ou dans laquelle R¹ et R forment conjointement un groupe de formule -A-X-A³-, X représente un groupe sulfinyle ou sulfonyle, groupe qui peut porter un ou deux groupes alkylsulfinyle ou alkylsulfonyle, l'oxydation d'un composé de formule I dans laquelle Ar porte un substituant alkylthio, dans laquelle X représente un groupe thio ou dans laquelle R¹ et R forment conjointement un groupe de formule -A-X-A³-, X représente un groupe thio, groupe qui peut porter un ou deux groupes alkylthio ;
(f) pour la production des composés de formule I dans laquelle Ar porte un substituant alcanoylamino, l'acylation d'un composé de formule I dans laquelle Ar porte un substituant amino ;
(g) pour la production des composés de formule I dans laquelle A représente un groupe alcénylène, ou dans laquelle R¹ et R forment conjointement un groupe de formule -A-X-A³- et R³ représente un groupe alcényle, la réduction du composé correspondant dans lequel A représente un groupe alcynylène ou R³ représente un groupe alcynyle ;
(h) pour la production des composés de formule I dans laquelle Q porte un substituant alkyle ou alkyle substitué sur un atome d'azote disponible, ou dans laquelle Ar porte un substituant alkoxy ou alkoxy substitué, l'alkylation d'un composé de formule I dans laquelle Q porte un atome d'hydrogène sur ledit atome d'azote disponible, ou dans laquelle Ar porte un substituant hydroxy ; ou
(i) pour la production des composés de formule I dans laquelle Q ou Ar portent un substituant amino, la réduction d'un composé de formule I dans laquelle Q ou Ar portent un substituant nitro ;
et, lorsqu'un sel pharmaceutiquement acceptable d'un composé nouveau de formule I est requis, l'obtention de ce sel par réaction dudit composé avec un acide ou une base convenable au moyen d'un mode opératoire classique.

2. Procédé suivant la revendication 1 pour la production d'un éther cyclique hétérocyclique de formule I, dans lequel Q représente un groupe pyridyle, pyrimidinyle, pyrazinyle, quinolyle, isoquinolyle, quinazolinyle ou quinoxalinyle qui peut porter facultativement un, deux ou trois substituants choisis entre les substituants fluoro, chloro, hydroxy, oxo, méthyle, éthyle, propyle, trifluorométhyle, 2-fluoréthyle, 2-diméthylaminoéthyle et benzyle ;
A représente un groupe méthylène, 1-propénylène ou 1-propynylène ;
X représente un groupe oxy ;
Ar représente un groupe 1,3-phénylène ou 1,4-phénylène qui peut porter facultativement un ou deux substituants choisis entre les substituants fluoro, chloro, hydroxy, amino, méthoxy et trifluorométhyle, ou
Ar représente un groupe 3,5-pyridylène ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 ou 6 atomes dans le cycle, dans lequel A représente un groupe méthylène, A³ représente un groupe méthylène ou éthylène et X représente un groupe oxy, noyau qui peut porter un, deux ou trois substituants choisis entre les substituants méthyle, éthyle, propyle, isopropyle, butyle, isobutyle et sec.-butyle, et R³ représente un groupe méthyle ou éthyle ;
ou d'un de ses sels pharmaceutiquement acceptables.

3. Procédé suivant la revendication 1 pour la production d'un éther cyclique hétérocyclique de formule I dans laquelle Q représente un groupe 2-pyridyle, 3-pyridyle, 3-quinolyle, 5-quinolyle, 6-quinolyle, 7-quinolyle ou 6-quinoxalinyle qui peut porter facultativement un ou deux substituants choisis entre les substituants hydroxy, oxo, méthyle, éthyle, propyle, 2-fluoréthyle, 2-diméthylaminoéthyle et benzyle ;
A représente un groupe méthylène, 1-propénylène ou 1-propynylène ;
X représente un groupe oxy ;
Ar représente un groupe 1,3-phénylène ou 1,4-phénylène qui peut porter facultativement un ou deux substituants choisis entre les substituants fluoro, chloro, amino, méthoxy et trifluorométhyle, ou Ar représente un groupe 3,5-pyridylène ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 atomes dans le cycle, dans lequel A représente un groupe méthylène, A³ représente un groupe méthylène et X représente un groupe oxy, noyau qui peut porter un, deux ou trois substituants choisis entre les substituants méthyle, éthyle, propyle, isopropyle, butyle, isobutyle et sec.-butyle, et R³ représente un groupe méthyle ou éthyle ;
ou d'un de ses sels pharmaceutiquement acceptables.

4. Procédé suivant la revendication 1 pour la production d'un éther cyclique hétérocyclique de formule I dans laquelle Q représente un groupe 2-pyridyle, 1,2-dihydro-1-méthyl-2-oxoquinoline-3-yle, 2-quinolyle, 3-quinolyle, 1,2-dihydro-2-oxoquinoline-3-yle, 3-isoquinolyle ou 6-quinoxalinyle ;
A représente un groupe méthylène ou 1-propynylène ;
Ar représente un groupe 1,3-phénylène ou 5-fluoro-1,3-phénylène ; et
R¹ et R forment conjointement un groupe de formule -A-X-A³- qui, conjointement avec l'atome d'oxygène auquel A est fixé et avec l'atome de carbone auquel A³ est fixé, définit un noyau ayant 5 atomes dans le cycle, dans lequel chacun des groupes A et A³ représente un groupe méthylène, X représente un groupe oxy, noyau qui peut porter un ou deux substituants méthyle, et R³ représente un groupe éthyle ;
ou d'un de ses sels pharmaceutiquement acceptables.

5. Procédé suivant la revendication 1 pour la production d'un éther cyclique hétérocyclique de formule I dans laquelle Q représente un groupe 1,2-dihydro-2-oxoquinoline-5-yle, 1,2-dihydro-2-oxoquinoline-6-yle ou 1,2-dihydro-2-oxoquinoline-7-yle qui porte un substituant en position 1 choisi entre les substituants méthyle, éthyle et 2-fluoréthyle ;
A représente un groupe méthylène ;
X représente un groupe oxy ;
Ar représente un groupe 1,3-phénylène qui peut porter facultativement un substituant fluoro ;
R¹ et R forment conjointement un groupe de formule -A-X-A³- qui, conjointement avec l'atome d'oxygène auquel A est fixé et avec l'atome de carbone auquel A³ est fixé, définit un noyau ayant 5 atomes dans le cycle, dans lequel A représente un groupe méthylène, A³ représente un groupe méthylène et X représente un groupe oxy, noyau qui peut porter un ou deux substituants choisis entre les substituants méthyle, éthyle, propyle et isopropyle, et R³ représente un groupe méthyle ou éthyle ;
ou d'un de ses sels pharmaceutiquement acceptables.

6. Procédé pour la production d'une composition pharmaceutique, caractérisé par la mise en association d'un éther cyclique hétérocyclique de formule I, ou d'un de ses sels pharmaceutiquement acceptables, répondant à la définition suivant l'une quelconque des revendications 1 à 5, et d'un diluant ou support pharmaceutiquement acceptable.
